# EUROPEAN PATENT SPECIFICATION

(11) **EP 4 468 943 B1**
(45) Date of publication and mention of the grant of the patent: **25.11.2026**
(21) Application number: 23701727.2
(22) Date of filing: 24.01.2023
(51) Int. Cl.: A61B 5/00, G06T 7/00, G06V 20/69, G06V 10/26, G06V 10/44

(54) **METHODS USING TRAINED CLASSIFIER TO DISTINGUISH BETWEEN HEALTHY AND DISEASED MYOTUBES**
VERFAHREN MIT TRAINIERTEM KLASSIFIKATOR ZUR UNTERSCHEIDUNG ZWISCHEN GESUNDEN UND ERKRANKTEN MYOTUBES
MÉTHODES UTILISANT UN CLASSIFICATEUR ENTRAÎNÉ POUR FAIRE LA DISTINCTION ENTRE DES MYOTUBES SAINS ET MALADES

(30) Priority: 24.01.2022 WO PCT/EP2022/051514
(43) Date of publication of application: 04.12.2024
(73) Proprietor: Cytoo, 38000 Grenoble (FR)
(72) Inventor: CHAMPETIER, Tiphaine, 38100 GRENOBLE (FR); DARIMONT, Beatrice, SAN DIEGO, California 92111 (US); FLAENDER, Mélanie, 38000 GRENOBLE (FR); GASTON, Cécile, 92350 LE PLESSIS ROBINSON (FR); LORINTIU, Oana, 38320 EYBENS (FR); MARTIN TISSIER, Antoine, 38000 GRENOBLE (FR); POYDENOT, Pauline, 73470 NOVALAISE (FR); SELIG, Luc, 94220 CHARENTON LE PONT (FR); VENTRE, Erwann, 38700 LA TRONCHE (FR)
(74) Representative: Cabinet Becker et Associés
(86) International application number: PCT/EP2023/051702
(87) International publication number: WO 2023/139290

(56) References cited:
- WO-A1-2021/081484
- WO-A1-2021/142267
- AURORA SÁEZ ET AL: "Quantifiable diagnosis of muscular dystrophies and neurogenic atrophies through network analysis", BMC MEDICINE, BIOMED CENTRAL LTD., LONDON, GB, vol. 11, no. 1, 20 March 2013 (2013-03-20), pages 77, XP021147043, ISSN: 1741-7015, DOI: 10.1186/1741-7015-11-77
- DOMINIC SCAGLIONI ET AL: "A high-throughput digital script for multiplexed immunofluorescent analysis and quantification of sarcolemmal and sarcomeric proteins in muscular dystrophies", ACTA NEUROPATHOLOGICA COMMUNICATIONS, BIOMED CENTRAL LTD, LONDON, UK, vol. 8, no. 1, 17 April 2020 (2020-04-17), pages 1 - 16, XP021275454, DOI: 10.1186/S40478-020-00918-5
- KABEYA YOSHINORI ET AL: "Deep convolutional neural network-based algorithm for muscle biopsy diagnosis", LABORATORY INVESTIGATION, NATURE PUBLISHING GROUP, THE UNITED STATES AND CANADIAN ACADEMY OF PATHOLOGY, INC, vol. 102, no. 3, 2 October 2021 (2021-10-02), pages 220 - 226, XP037697146, ISSN: 0023-6837, [retrieved on 20211002], DOI: 10.1038/S41374-021-00647-W

## Description

### FIELD OF THE INVENTION

The present invention relates to methods for determining if a myotube is a healthy myotube or a myotube exhibiting features of a neuromuscular disorder of interest ("diseased myotube"), for assessing potency of a compound to revert the phenotype of a diseased myotube into a healthy phenotype, of predicting the ability of a compound to treat a neuromuscular disorder of interest, for monitoring the response to a therapeutic compound of a patient affected with a neuromuscular disorder, for selecting a patient affected with a neuromuscular disorder for a treatment with a therapeutic compound, for determining whether a patient affected with a neuromuscular disorder is susceptible to benefit from a treatment with a therapeutic compound and for diagnosing a neuromuscular disorder of interest in a subject.

### BACKGROUND OF THE INVENTION

During the last decades genetic analysis identified more than 500 genes involved in the cause of neuromuscular disorders. The identification of these genes greatly advanced our understanding of muscle function and disease and opened many opportunities for therapeutic intervention. Despite those advances, for many neuromuscular diseases the disease mechanism is still unknown and effective therapies remain to be identified.

The unique structural and functional properties of muscles provide substantial challenges for the functional characterization of muscle disorders and discovery of therapies. Particularly challenging are striated muscles, which are formed by the fusion of mononuclear progenitor cells, called myoblasts, to multinucleated myotubes. These myotubes then differentiate into myofibers that contain a contractile apparatus (sarcomere) allowing these fibers to contract upon stimulation from an attached motor neuron. The ability of muscle fibers to differentiate and contract is dependent on their interactions with connective tissues that surround these fibers (basal lamina), which allow these fibers to contract without getting damaged and to differentiate in response to mechanical forces. Each muscle fiber is innervated by a single motor neuron through a neuro-muscular junction (NJM). A process called excitation-contraction coupling converts the neuronal excitation mediated by the motor neuron into Ca²⁺ signaling that induces the muscle contractions. Skeletal muscle fibers are terminally differentiated. However, specific processes are in place to repair contraction-induced damages in the plasma membrane, or to replace damaged muscle fibers through activation of muscle stem cells.

Neuromuscular disorders are generally classified as dystrophies, myopathies and myasthenic syndromes dependent on the morphology of the diseased muscle fibers and on the function affected by the disease. The underlying defects in these diseases are diverse and can affect either directly or indirectly the myofiber-matrix interactions, the excitation-contraction coupling, the contractile apparatus, the muscle metabolic activity, or ability to regenerate (Dowling et al., Nat Rev Mol Cell Biol, 2021, 22, 713-732). In addition to the muscle cells itself, neuromuscular disorders can also be caused by mutations in motor neurons (e.g., Spinal muscular dystrophy, Kennedy's disease), or in the basal lamina (e.g., Ullrich myopathy). Furthermore, the structure and function of muscles can be impaired metabolically in response to malnutrition, immobility, advanced age, or acute and chronic diseases. In these cases, changes in the structure and function of muscles are often driven by changes in protein turnover mediated by the ubiquitin/proteasome system and autophagy-lysosomal pathways, by the inhibition of growth factor pathways, and/or activation of inflammatory pathways.

Among the best characterized dystrophies are Duchenne and Becker muscular dystrophies (DMD, BMD). In DMD patients, mutations in the DMD gene prevent the expression of a functional dystrophin and the assembly of the Dystrophin-associated protein complex (DAPC) at the plasma membrane (Gao & McNally, 2015, Compr Physiol 5(3): 1223-1239). As part of the DAPC, dystrophin acts as shock absorber and prevents muscle cells from being damaged during contractions. Boys who do not express dystrophin usually lose the ability to walk around age 10 and have a shortened live span due to pulmonary and cardiac complications. In contrast to DMD, BMD patients express a partially functional dystrophin usually associated with less severe symptoms. Another example of a well-studied neuromuscular disease is myotonic dystrophy (DM1). In DM1 the disease is caused by the expansion of a (CAG) repeat in the gene DMPK. Upon expression, those expanded CAG repeats form a stem loop that is recognized by an RNA splicing factor MBNL1. The MBLN1 bound mutated DMPK transcripts form RNA foci in the nucleus of DM1 cells, resulting in a depletion of MBLN1 and consequent miss-splicing of other MBLN1 target RNAs. The mechanism by which MBLN1 depletion affects muscle function remains to be identified.

The advances in understanding of the cellular processes involved in muscle function has opened the door for therapeutic discovery. For an increasing number of disorders, various therapies have been developed and are currently passing through clinical development (Dowling et al., 2021 *supra*)*.* The most promising are gene therapies and oligonucleotide therapies that attempt to substitute a mutated disease driver or altering the processing or stability of RNA. However, even for well-understood neuromuscular disorders, discovery and development of these therapies have been challenging. In case of DMD, the main challenge is the size of the DMD gene, the largest gene in the human genome which encodes a 420 kDa protein. Since current gene therapies rely on viral vectors that have limited load capacities, gene therapies targeting DMD can only deliver the information for a drastically truncated and functionally impaired dystrophin. Likewise, oligonucleotide therapies targeting DMD aim to convert DMD patients into BMD patients by inducing the expression of a partially functional dystrophin. It remains to be determined to which extent these truncated dystrophins maintain the various activities mediated by dystrophin. The clinical success of these therapies has been further hampered by the lack of appropriate animal models that represent the diversity of the genetic backgrounds found in DMD patients. Similar challenges exist for many neuromuscular disorders.

For many diseases that affect complex biological mechanisms it has been proven difficult to reliably assess the activity of a therapy in vitro by monitoring an individual disease driver, isolated downstream effects, or a particular biomarker. Consequently, most drug discovery programs rely heavily on in vivo experiments using animal disease models. The sensitivity of in vitro studies is further hampered by the use of population-level transcriptional and proteomic profiling techniques to quantify cellular responses. These methods often lack the sensitivity to reliably detect subtle responses within the naturally occurring variability in a cell population.

There is thus a strong need of new methods to assess the function of therapeutic agents in patient-derived muscle cells under physiological relevant conditions and to target these therapies to patient subpopulations that have the greatest chance to benefit. AURORA SÁEZ ET AL: "Quantifiable diagnosis of muscular dystrophies and neurogenic atrophies through network analysis", BMC MEDICINE, vol. 11, no. 1, March 2013, 77 discloses a method of diagnosing muscular dystrophies from images of biopsied tissue samples using a neural network.

### SUMMARY OF THE INVENTION

The present invention is defined by the appended claims. The dependent claims set out particular embodiments.

Although not part of the claimed invention, the disclosure relates to a computer-implemented method for determining if a myotube is a healthy myotube or a myotube exhibiting features of a neuromuscular disorder of interest ("diseased myotube") from at least one image, wherein the method comprises:
- providing at least one input image of a myotube, or preprocessed information obtained from said at least one input image, to a classifier trained to distinguish between healthy myotubes and diseased myotubes, wherein the myotube has been stained for an imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one region of interest (ROI) selected from the group consisting of individual myotubes, structures of myotubes, and any combination thereof, and
- using the classifier to identify the myotube on the image as a healthy myotube or as a diseased myotube as an output of the classifier.

Although not part of the claimed invention, the disclosure also relates to a computer-implemented method of training a classifier for accurately distinguishing between healthy myotubes and myotubes exhibiting features of a neuromuscular disorder of interest ("diseased myotube"), said method comprising
a) providing a training set of images of stained myotubes, or preprocessed information obtained from said training set of images, to a classifier, said training set of images comprising images of healthy and diseased myotubes stained for an imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one region of interest (ROI) selected from the group consisting of individual myotubes, structures of myotubes, and any combination thereof;
b) generating an output of the classifier for each input ROI, said output classifying the input ROI as associated to a healthy or diseased myotube;
c) comparing the generated output for each input ROI to a label associated with said input ROI, said label comprising an indication of the healthy or diseased status of the myotube corresponding to said input ROI;
d) evaluating the classifier's accuracy for distinguishing between healthy myotubes and diseased myotubes,
wherein the classifier is considered as an accurate classifier to distinguish between healthy myotubes and diseased myotubes if it exhibits an accuracy corresponding to a F-score equal to or greater than 0.9.

Although not part of the claimed invention, the disclosure further relates to a computer-implemented method of identifying an imaging marker of a neuromuscular disorder of interest comprising
a) providing a training set of images of stained myotubes, or preprocessed information obtained from said training set of images, to a classifier, the training set of images comprising images of healthy myotubes and of myotubes exhibiting features of a neuromuscular disorder of interest ("diseased myotube"), said myotubes being stained for a candidate imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one region of interest (ROI) selected from the group consisting of individual myotubes, structures of myotubes, and any combination thereof;
b) generating an output of the classifier for each input ROI, said output classifying the input ROI as associated to a healthy or diseased myotube;
c) comparing the generated output for each input ROI to a label associated with said input ROI said label comprising an indication of the healthy or diseased status of the myotube corresponding to said input ROI;
d) evaluating the classifier's accuracy for distinguishing between healthy myotubes and diseased myotubes,
wherein said candidate imaging marker is identified as an imaging marker of said neuromuscular disorder if the classifier exhibits an accuracy to distinguish between healthy myotubes and diseased myotubes corresponding to a F-score equal to or greater than 0.9.

Preferably, the images of stained myotubes are obtained by
i) culturing myoblasts derived from at least one healthy subject and myoblasts derived from at least one patient suffering from the neuromuscular disorder of interest on a substrate allowing the production of homogeneous population of myotubes;
ii) staining these myotubes for said imaging marker and with said at least one labelling agent; and
iii) capturing images of these stained myotubes.

Preferably, evaluation of the classifier's accuracy carried out in step d) is based on the classification of myotubes comprised in a test set of images which is distinct from the training set of images, the healthy or diseased status of each myotube being known, and the test set of images being obtained and processed using the same method as that used to obtain and process the training set of images.

The present invention relates to an *in vitro* method of assessing potency of a compound to revert the phenotype of a myotube exhibiting features of a neuromuscular disorder of interest ("diseased myotube") into a healthy phenotype comprising
(i) providing at least one image comprising a plurality of myotubes derived from at least one patient suffering from a neuromuscular disorder of interest, or preprocessed information obtained from said at least one image, to a classifier trained to distinguish between healthy myotubes and diseased myotubes, wherein the myotubes have been contacted with the compound to be tested and have been stained for an imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one region of interest (ROI) selected from the group consisting of individual myotubes, structures of myotubes, and any combination thereof; and
(ii) using the classifier to identify each myotube corresponding to an input ROI as a healthy myotube or as a diseased myotube as an output of the classifier,
a number of myotubes classified as healthy myotubes which is above a statistically significant threshold being indicative that the compound to be tested is able to revert the phenotype of a myotube exhibiting features of the neuromuscular disorder of interest.

The method further comprises before step (i)
- culturing myoblasts derived from said at least one patient on a substrate allowing the production of homogeneous population of myotubes and contacting said myoblasts and/or myotubes with the compound to be tested;
- staining these myotubes for said imaging marker and for said at least one labelling agent; and
- capturing said at least one image of these stained myotubes.

Preferably, the method further comprises calculating a health score which is defined as the percentage of myotubes out of the total that have been classified as healthy myotubes.

The present invention also relates to an *in vitro* method of predicting the ability of a compound to treat a neuromuscular disorder of interest comprising
assessing potency of a compound to be tested to revert the phenotype of a myotube exhibiting features of a neuromuscular disorder of interest into a healthy phenotype according to the method of the invention,
and optionally calculating a health score which is defined as the percentage of myotubes out of the total that have been classified as healthy myotubes,
wherein a number of myotubes classified as healthy myotubes, or a calculated health score, which is above a statistically significant threshold is indicative that said compound is useful in the treatment of said neuromuscular disorder.

The present invention also relates to an *in vitro* method for monitoring the response to a therapeutic compound of a patient affected with a neuromuscular disorder, wherein the method comprises
(i) providing at least one first image comprising a plurality of myotubes derived from a patient suffering from a neuromuscular disorder of interest before the administration of the therapeutic compound to the patient, or preprocessed information obtained from said at least one first image, and at least one second image comprising a plurality of myotubes derived from said patient after the administration of the therapeutic compound, or preprocessed information obtained from said at least one second image, to a classifier trained to distinguish between healthy myotubes and diseased myotubes, wherein the myotubes have been stained for an imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one regions of interest (ROI) selected from the group consisting of individual myotubes, structures of myotubes, and any combination thereof; and
(ii) using the classifier to identify each myotube of said at least one first image corresponding to an input ROI as a healthy myotube or as a diseased myotube as a first output of the classifier, and to identify each myotube of said at least second image corresponding to an input ROI as a healthy myotube or as a diseased myotube as a second output of the classifier, and
   optionally calculating health scores for the first and second outputs of the classifier which are each defined as the percentage of myotubes out of the total that have been classified as healthy myotubes,
   wherein a number of myotubes classified as healthy myotubes, or a calculated health score, in the second output of the classifier which is above a number of myotubes classified as healthy myotubes or above a calculated health score in the first output of the classifier is indicative that the patient is responsive to said therapeutic compound, and wherein a number of myotubes classified as healthy myotubes or a calculated health score in the second output of the classifier which is equal to or below a number of myotubes classified as healthy myotubes or equal to or below a calculated health score in the first output of the classifier is indicative that the patient does not respond to said therapeutic compound.

The method further comprises before step (i)
culturing myoblasts derived from said patient before and after the administration of the therapeutic compound on a substrate allowing the production of homogeneous population of myotubes;
staining these myotubes for said imaging marker and with said at least one labelling agent; and
capturing said at least one image of these stained myotubes.

Although not part of the claimed invention, the disclosure also relates to an *in vitro* method for selecting a patient affected with a neuromuscular disorder for a treatment with a therapeutic compound or for determining whether a patient affected with a neuromuscular disorder is susceptible to benefit from a treatment with a therapeutic compound, wherein the method comprises
(i) providing at least one image comprising a plurality of myotubes derived from a patient suffering from a neuromuscular disorder of interest, or preprocessed information obtained from said at least one image, to a classifier trained to distinguish between healthy myotubes and diseased myotubes, wherein the myotubes have been contacted with a therapeutic compound and have been stained for an imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one region of interest (ROI) selected from the group consisting of individual myotubes, structures of myotubes, and any combination thereof; and
(ii) using the classifier to identify each myotube corresponding to an input ROI as a healthy myotube or as a diseased myotube as an output of the classifier, and
   optionally calculating a health score which is defined as the percentage of myotubes out of the total that have been classified as healthy myotubes,
   wherein a number of myotubes classified as healthy myotubes, or a calculated health score, which is above a statistically significant threshold is indicative that a treatment with said therapeutic compound is suitable for said patient and wherein a number of myotubes classified as healthy myotubes or a health score which is equal to or below a statistically significant threshold is indicative that a treatment with said therapeutic compound is not suitable for said patient

The method may further comprise before step (i)
culturing myoblasts derived from said patient on a substrate allowing the production of homogeneous population of myotubes and contacting said myoblasts and/or myotubes with said therapeutic compound;
staining these myotubes for said imaging marker and with said at least one labelling agent; and
capturing said at least one image of these stained myotubes.

Although not part of the claimed invention, the disclosure also relates to an *in vitro* method for diagnosing a neuromuscular disorder of interest in a subject, wherein the method comprises
(i) providing at least one image comprising a plurality of myotubes derived from a subject, or preprocessed information obtained from said at least one image, to a classifier trained to distinguish between healthy myotubes and myotubes exhibiting features of a neuromuscular disorder of interest ("diseased myotubes"), wherein the myotubes have been stained for an imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one region of interest (ROI) selected from the group consisting of individual myotubes, structures of myotubes, and any combination thereof; and
(ii) using the classifier to identify each myotube corresponding to an input ROI as a healthy myotube or as a diseased myotube as an output of the classifier, and
   optionally calculating a health score which is defined as the percentage of myotubes out of the total that have been classified as healthy myotubes,
   wherein a number of myotubes classified as healthy myotubes, or a calculated health score, which is below a statistically significant threshold is indicative that said subject suffers from said neuromuscular disorder of interest, and a number of myotubes classified as healthy myotubes, or a calculated health score, which is equal to or above a statistically significant threshold is indicative that said subject does not suffer from said neuromuscular disorder of interest.

The method may further comprise before step (i)
culturing myoblasts derived from the subject on a substrate allowing the production of homogeneous population of myotubes and ;
staining these myotubes for said imaging marker and with said at least one labelling agent; and
capturing said at least one image of these stained myotubes.

Preferably, in said methods, said imaging marker and said at least one labelling agent have been used during the training of the classifier.

Preferably, in said methods, said classifier has been trained using the method of the invention.

Preferably, in said methods, said preprocessed information are obtained by performing an image segmentation with an algorithm on appropriate staining channel(s) in order to identify ROI. Alternatively, in said methods, said preprocessed information are obtained by performing an image segmentation with an algorithm on appropriate staining channel(s) in order to identify ROI, and extracting from each input ROI phenotypic features associated with said imaging marker.

Preferably, said extracted phenotypic features are selected from the group consisting of intensity features, granularity features, intensity distribution features, texture features, size and shape features, colocalization features, run-length grey level matrix-based features, wavelet transform based features and combinations thereof, preferably selected from the group consisting of intensity features, granularity features, intensity distribution features, texture features, and any combinations thereof.

Preferably, the classifier is selected from Support Vector Machine (SVM) classifier, random forest (RF) classifier, decision tree classifier, K-nearest neighbor classifier (KNN), logistic regression classifier, nearest neighbor classifier, Gaussian mixture model (GMM), nearest centroid classifier, linear regression classifier, and neural networks such as artificial, deep, convolutional, fully connected neural networks, more preferably selected from Support Vector Machine (SVM) classifier, random forest (RF) classifier and convolutional neural network (CNN), and even more preferably is SVM classifier.

Preferably, said identified disease drivers of the neuromuscular disorder of interest are one or several of those listed in Table 1 and corresponding to the neuromuscular disorder of interest. Preferably, said proteins associated with specific functional or structural properties of muscle cells are those listed in Table 2. Preferably, said cell morphological features affected in neuromuscular disorders are those listed in Table 3.

Preferably, the neuromuscular disorder of interest is selected from the group consisting of muscular dystrophies, myopathies, congenital myasthenic syndromes, motor neuron diseases and metabolic muscle disorders. More preferably, the neuromuscular disorder of interest is selected from the group consisting of muscular dystrophies, myopathies, congenital myasthenic syndromes and motor neuron diseases. In particular, the neuromuscular disorder of interest may be a muscular dystrophy selected from the group consisting of Duchenne Muscular Dystrophy (DMD), Becker Muscular Dystrophy (BMD), Myotonic Dystrophy 1 (DM1), Myotonic Dystrophy 2 (DM2), Facioscapulohumeral Muscular Dystrophy (FSHD), Emery-Dreifuss muscular dystrophy, Limb-girdle muscular dystrophies, Walker-Warburg syndrome, Muscle-eye-brain disease, Congenital muscular dystrophy, Scapuloperoneal muscular dystrophy, Tibial muscular dystrophy and Autosomal Recessive Muscular Dystrophy. Alternatively, the neuromuscular disorder of interest may be a myopathy selected from the group consisting of Bethlem & Ullrich myopathy, Myofibrillar myopathy, Distal myopathy, Rimmed vacuole myopathy, Centronuclear myopathy (CNM), X-linked myotubular myopathy (XLMTM), Tubular aggregate myopathy, Malignant hyperthermia syndrome, Inclusion body myopathy, Myofibrillar myopathy, Protein aggregate myopathy, Nemaline myopathy, Congenital myopathy (CM), Myoshi myopathy, Vici syndrome, X-linked myopathy with excessive autophagy, Danon disease, Pompe disease and Primary mitochondrial myopathies.

Alternatively, the neuromuscular disorder of interest may be a congenital myasthenic syndrome selected from the group consisting of Myasthenia gravis and other myasthenic syndromes driven by mutations in CHAT, COLQ, RAPSN, CHRNE, DOK7 and/or GFPT1 genes.

Alternatively, the neuromuscular disorder of interest may be a motor neuron disease selected from the group consisting of Spinal Muscular Atrophy (SMA), Amyotrophic Lateral Sclerosis (ALS) and Kennedy's disease.

Alternatively, the neuromuscular disorder of interest may be a metabolic muscle disorder selected from the group consisting of cachexia, sarcopenia and muscle atrophy.

In particular, the neuromuscular disorder may be Duchenne muscular dystrophy and the myotubes may be stained for an imaging marker which is selected from the group consisting of utrophin, alpha-sarcoglycan delta-sarcoglycan, the combinations of utrophin with alpha-sarcoglycan, delta-sarcoglycan, alpha-dystroglycan or beta-dystroglycan, preferably utrophin and/or alpha-sarcoglycan.

Alternatively, the neuromuscular disorder may be myotonic dystrophy type 1 and the myotubes may be stained for an imaging marker which is a combination of the protein MBNL1 and RNA foci, in particular DMPK mRNA foci.

Although not part of the claimed invention, the disclosure also relates to the use of a protein selected from the group consisting of utrophin, alpha-sarcoglycan, delta-sarcoglycan, and the combinations of utrophin with alpha-sarcoglycan, delta-sarcoglycan, alpha-dystroglycan or beta-dystroglycan, as an imaging marker for DMD, in particular as an imaging marker for assessing potency of a compound to revert the phenotype of a myotube exhibiting features of DMD into a healthy phenotype, for predicting the ability of a compound to treat DMD, for monitoring the response to a therapeutic compound of a patient affected with DMD, for selecting a patient affected with DMD for a treatment with a therapeutic compound or for determining whether a patient affected with DMD is susceptible to benefit from a treatment with a therapeutic compound, or for diagnosing DMD, preferably using a classifier trained to distinguish between healthy myotubes and myotubes exhibiting features of DMD, more preferably using a classifier trained to distinguish between healthy myotubes and myotubes exhibiting features of DMD according to the method of the invention.

Although not part of the claimed invention, the disclosure also relates to the use of a combination of the protein MBNL1 and RNA foci, in particular DMPK mRNA foci, as an imaging marker for DM1, in particular as an imaging marker for assessing potency of a compound to revert the phenotype of a myotube exhibiting features of DM1 into a healthy phenotype, for predicting the ability of a compound to treat DM1, for monitoring the response to a therapeutic compound of a patient affected with DM1, for selecting a patient affected with DM1 for a treatment with a therapeutic compound or for determining whether a patient affected with DM1 is susceptible to benefit from a treatment with a therapeutic compound, or for diagnosing DM1, preferably using a classifier trained to distinguish between healthy myotubes and myotubes exhibiting features of DM1, more preferably using a classifier trained to distinguish between healthy myotubes and myotubes exhibiting features of DM1 according to the method of the invention.

The present invention also relates to a computing system comprising:
- a memory storing at least one instruction of a classifier trained according to the method of the invention, and
- a processor accessing to the memory for reading the aforesaid instructions and executing a method of the invention.

### BRIEF DESCRIPTION OF THE FIGURES

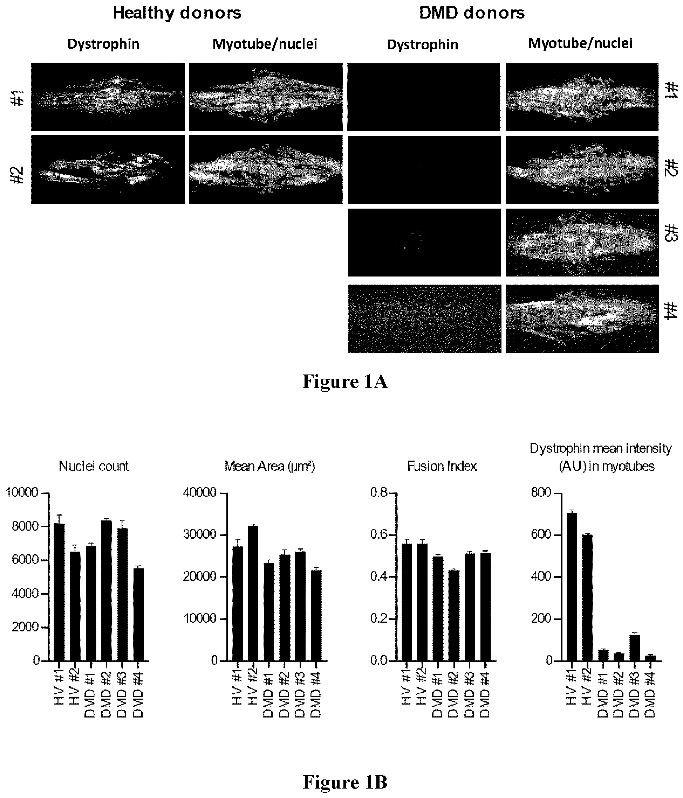

**Figure 1****:** Validation of myotubes from healthy and DMD patients for cell profiling analysis. Primary myoblasts from two non- DMD (HV #1, HV #2) and four DMD (DMD #1, DMD #2, DMD #3, DMD #4) donors were selected among other donors based on retaining their capacity to differentiate (% Desmin+ myoblasts and Fusion Index) into myotubes within the Myoscreen platform. Myoblasts from the selected donors were differentiated for 9 days in Myoscreen micropatterned plates. The differentiated myotubes were stained for Myosin Heavy Chain (MHC) and Dystrophin protein. **(A)** Sample fluorescent images from the 4 DMD donors and 2 healthy donors stained for dystrophin and myotube and nuclei staining. Dystrophin was not detected in differentiated myotubes from DMD donors. **(B)** High content analysis quantification of nuclei count, myotube area, fusion index and dystrophin mean intensity for myotubes generated from the 6 different donors.

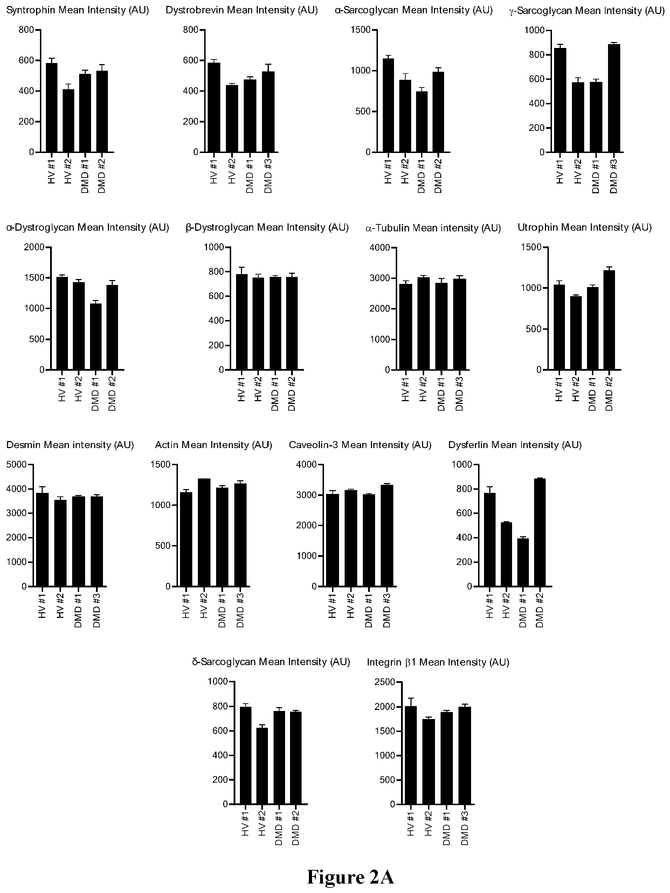

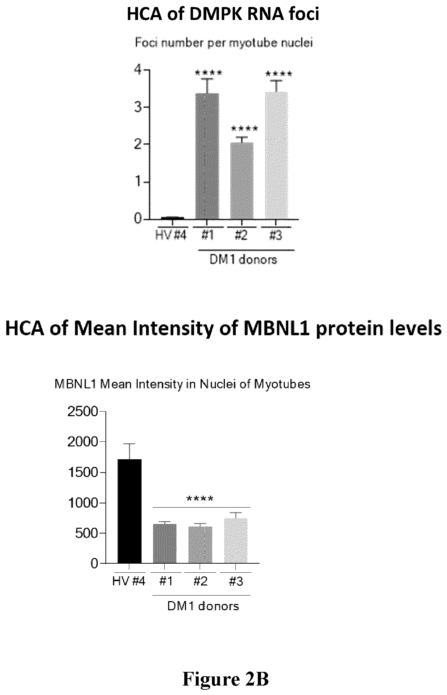

**Figure 2****:** HCA analysis of muscle markers **(A)** Mean intensity HCA of proteins in cells from healthy and DMD donors. Myoblasts from DMD and healthy donors were cultured and differentiated in myotubes on MyoScreen platform. Myotubes obtained after nine days of differentiation were fixed and expression of fourteen proteins involved in structural and functional properties of muscle cells were investigated by immunofluorescence using antibodies listed in the Table 12. Their expression was quantified using high content analysis through mean fluorescence intensity. **(B)** RNA foci and MBNL1 expression in cultured myotubes from healthy and DM1 donors. Myoblasts from one healthy (HV #4) and 3 DM1 donors were cultured and then differentiated on MyoScreen for 8 days. DMPK foci and MBNL1 protein expression in myotubes nuclei, two main characteristics of DM1 pathology, were detected by immunofluorescent staining combined with FISH and analyzed by high content analysis. DMPK foci were detected specifically in DM1 donors myotube nuclei and MBNL1 expression was decreased in those donors due to retention in aggregates. n=3 wells, One-way Anova DM1 donors vs Healthy donor #4, *p<0.05, **p<0.01, ***p<0.001, ****p<0.0001

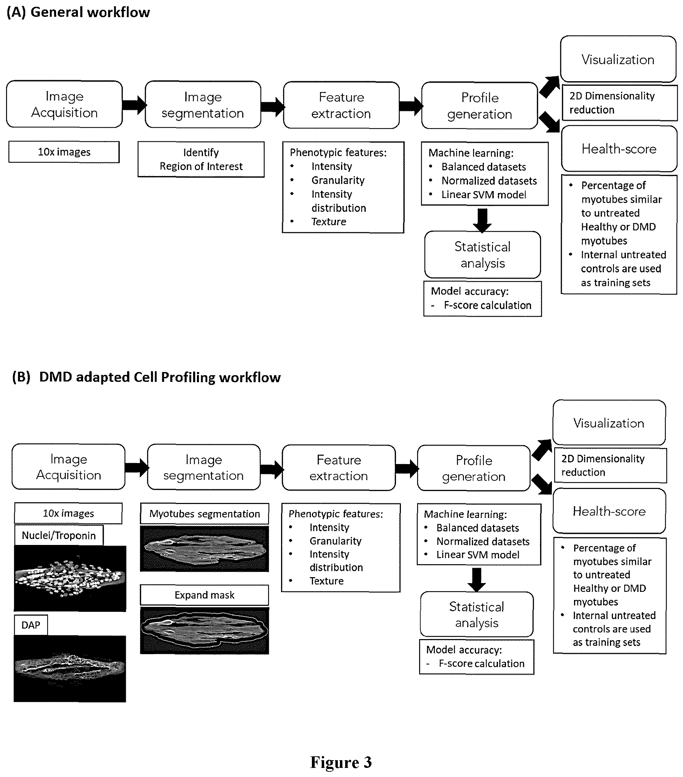

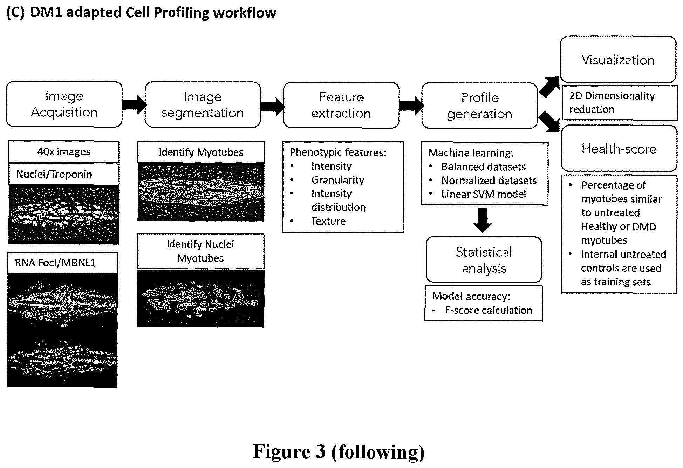

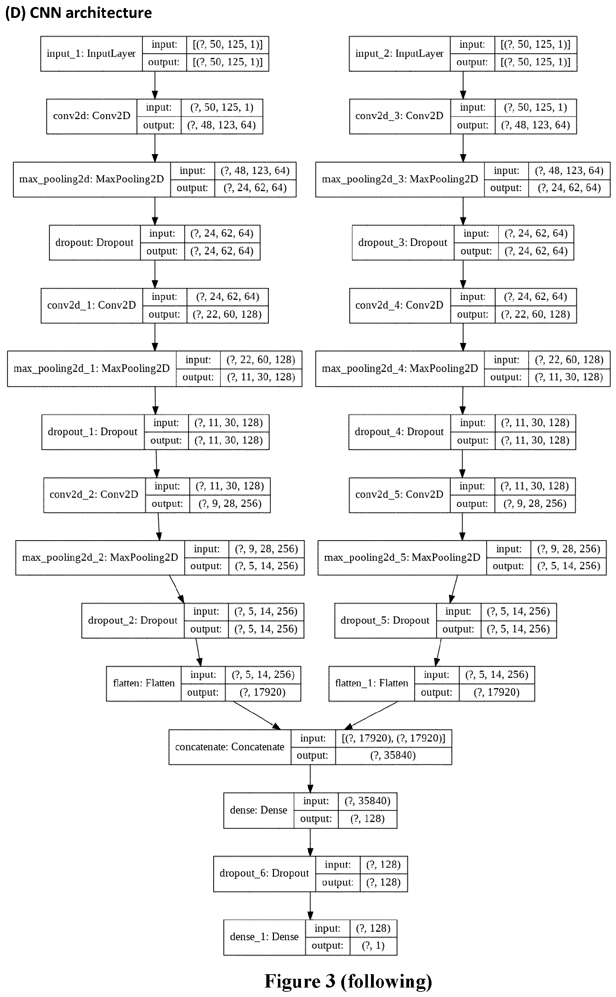

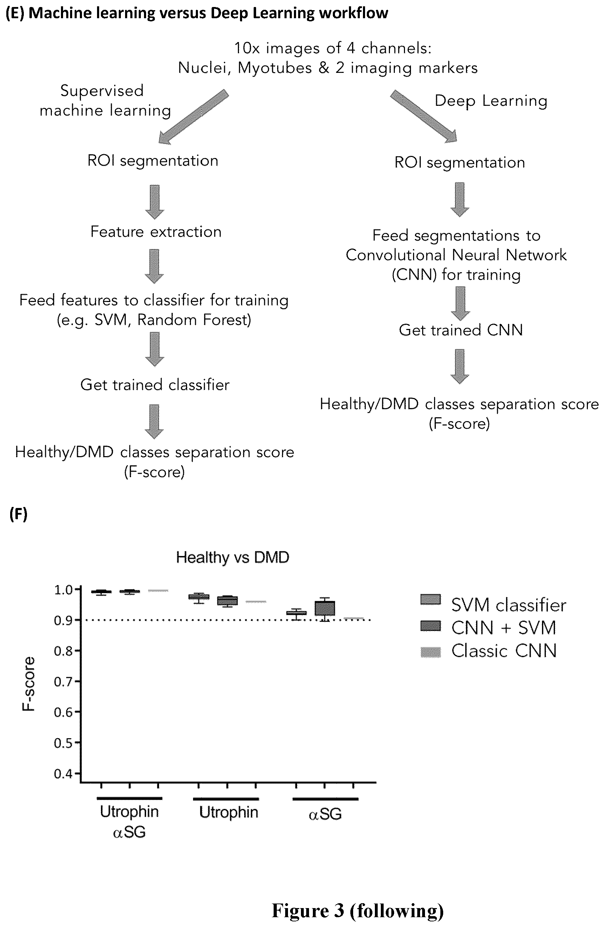

**Figure 3****:** Image processing and data analysis procedure for cell profiling assays monitoring labeled disease biomarkers. **(A)** The workflow presented in Figure 3A includes the main steps of a machine learning pipeline. We start by constructing an annotated database with immunostained plates acquired on a 10x Operetta HCS imaging system. The acquired images undergo the pre-processing, segmentation of region of interest (ROI) and feature extraction steps. Using machine learning or deep learning algorithms we next generate the profile of diseased and healthy cells. **(B)** This figure shows the adapted general workflow to perform cell profiling analysis on labeled DMD cells (monitoring the proteins characterized in Figure 2A). The region of interest is represented by the myotubes that are segmented using the myotube staining channel and dilated for further analysis. The features are extracted from the protein marker channel. **(C)** This figure shows the adapted general workflow to DM1 and foci RNA/MBNL1 based Cell Profiling. In this case, the nuclei situated inside the myotubes region are considered as the region of interest. The features are extracted from the RNA Foci/MBNL1 biomarkers channels. **(D)** Figure showing the chosen CNN architecture. **(E)** This figure shows the two different processing pipelines between the chosen supervised machine learning methods (RF, SVM) and the chosen deep learning method. **(F)** DMD vs healthy classification performance of the SVM, classic CNN and CNN with SVM classifier as last layer.

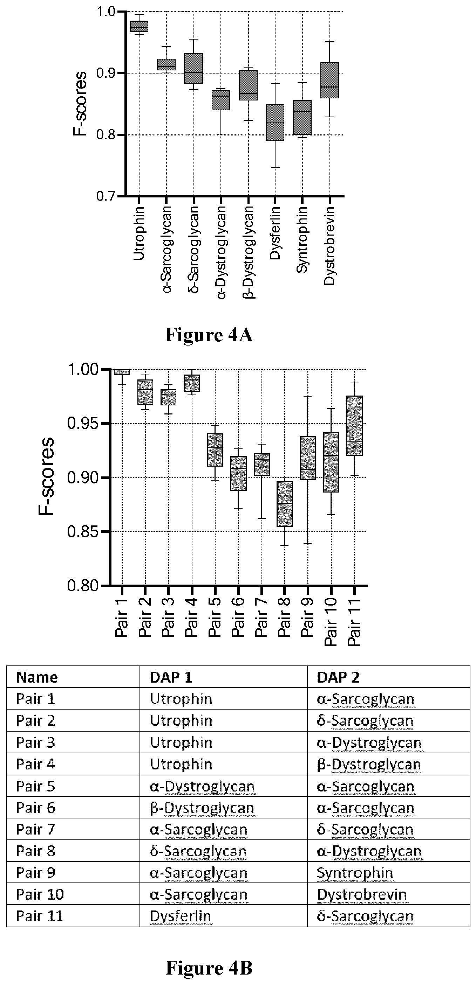

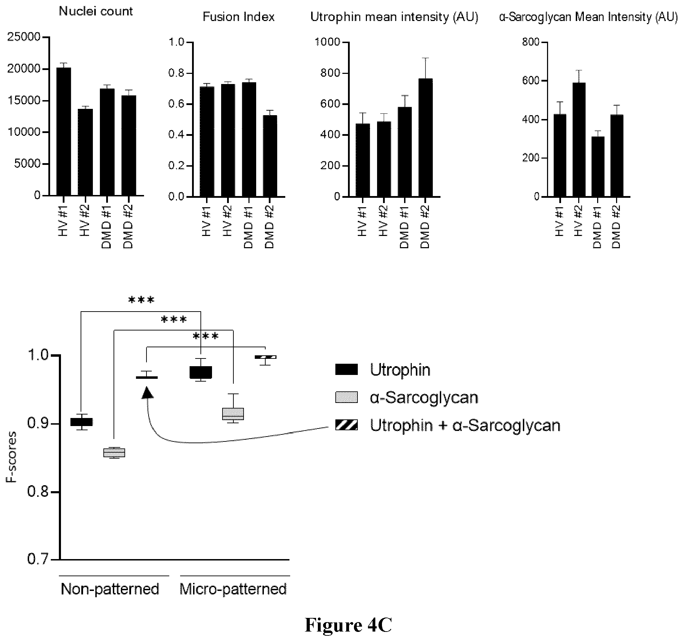

**Figure 4****:** Selection of imaging proteins for cell profiling analysis of healthy and DMD patient-derived myotubes. **(A)** Cell profile analysis distinguishes healthy and DMD donor cells labeled for utrophin and α-sarcoglycan but not cells labeled for δ-sarcoglycan, dysferlin, syntrophin, α-dystroglycan, β-dystroglycan, or dystrobrevin. F-scores obtained using the myotube features obtained by cell profiling monitoring individual imaging markers. Cells were cultured on MyoScreen platform. **(B)** F-scores obtained using the myotube features obtained by cell profiling monitoring combinations of imaging markers. Cells were cultured on MyoScreen platform. **(C)** Nuclei count and fusion index of myotubes cultured using standard 96 well plates. No significant differences between DMD and healthy donors were observed on the HCA readouts. F-scores using the subcellular features obtained from cell profiling from cells cultured in standard 96 well plates compared with cells cultured in the MyoScreen platform. A significantly higher cross validation F-score, sign of a better phenotype separation, can be observed when cells are cultured in the MyoScreen platform.

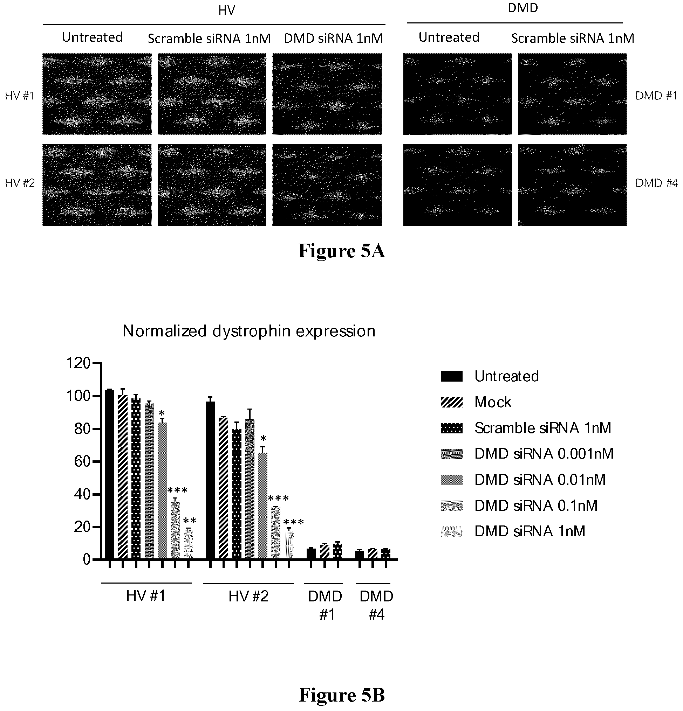

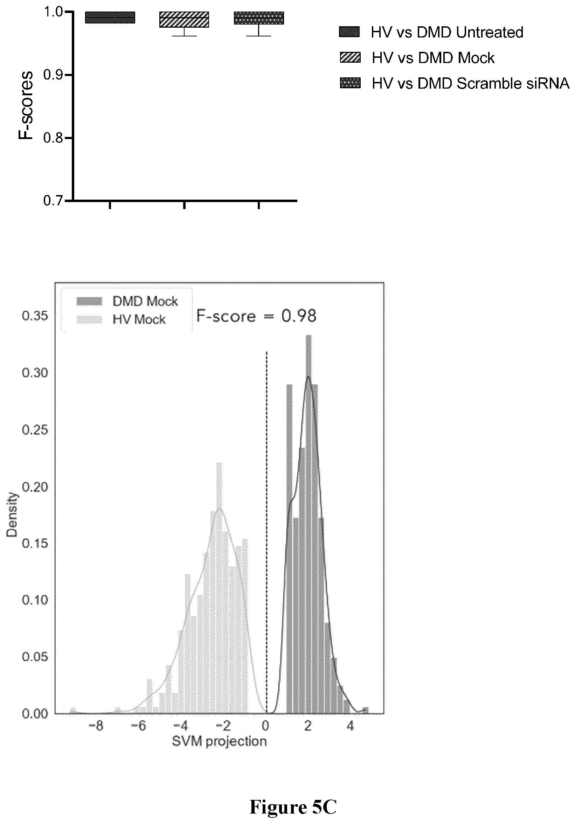

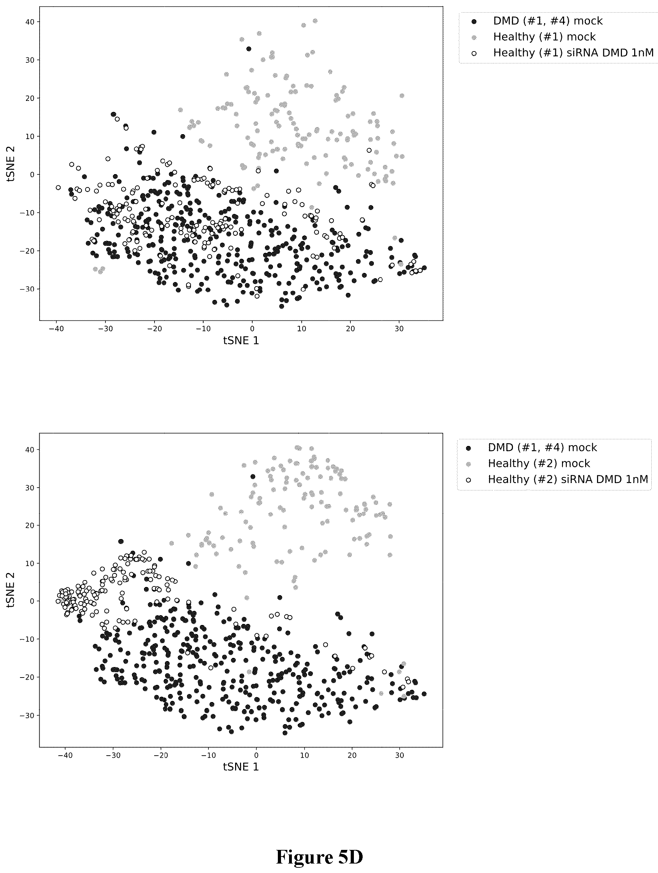

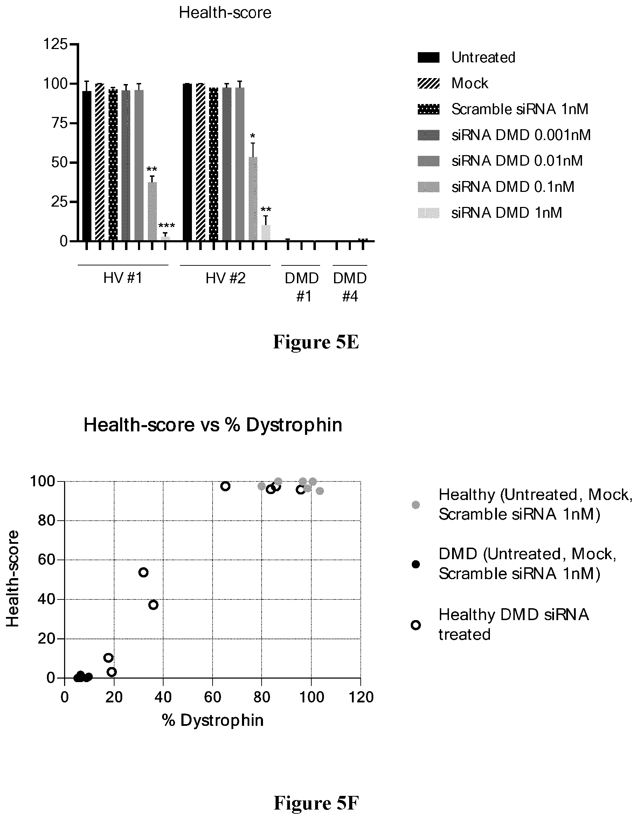

**Figure** 5: Phenotypic response of healthy myotubes to DMD downregulation using RNAi. **(A)** siRNA-based knock down of dystrophin in healthy differentiated myotube cells (HV #1 and HV #2). We screened one DMD siRNA at 4 concentrations: 1 nM, 0.1 nM, 0.01 nM and 0.001 nM, and one control Scramble siRNA at 1 nM. The gray intensity reflects dystrophin expression. For comparison, Dystrophin expression from the DMD-donors is included on the right. This is a baseline level of Dystrophin expression. In comparison, we saw that Dystrophin expression using the DMD siRNA at the highest concentration is comparable to Dystrophin expression detected from the DMD donors. **(B)** The graph shows the HCA quantification of dystrophin intensity normalized to the mean of healthy untreated donors. One-way Anova DMD siRNA treated conditions vs Scramble siRNA treated condition, *p<0.05, **p<0.01, ***p<0.001. **(C)** Healthy and DMD cells phenotype separability: F-score from cross validation analysis using SVM linear kernel by taking the 2 different categories at a time using Utrophin and α-Sarcoglycan features. As evident from the F-score, we can separate DMD and healthy cells in all control conditions: untreated, mock and scramble siRNA. The distribution of the SVM-projection of the myotubes features shows a good separation between healthy and DMD donors. **(D)** t-SNE plots of the pair Utrophin and α-Sarcoglycan features for healthy donor #1 (upper) and healthy donor #2 (bottom). Each dot is a myotube within a pattern. Light gray indicates mock treatment for healthy donors while black indicates mock treatment for the DMD donor cells. The circles indicate the DMD siRNA knock down of the healthy donor cells. **(E)** Using an SVM classifier trained on the scramble siRNA between the DMD and the healthy donors, we determined the percentage of cells with predicted healthy-like phenotypes (Heath-score) for myotubes that were either untreated, mock treated, treated with 1 nM scramble siRNA, or treated with various concentrations of the DMD siRNA. Reducing dystrophin expression in the healthy donor cells generated a phenotype that is similar to that of DMD donor cells. One-way Anova DMD siRNA treated conditions vs scramble siRNA treated, *p<0.05, **p<0.01, ***p<0.001. **(F)** Graph showing % dystrophin expression on the X-axis vs Health score on the Y-axis. Gray dots represent Untreated, Mock and Scramble siRNA (1nM) treated myotubes from Healthy donors. Black dots represent Untreated, Mock and Scramble siRNA (1nM) treated myotubes from DMD donors. Circles represent DMD siRNA (0.001, 0.01, 0.1 and 1nM) treated myotubes from Healthy donors. Myotubes that express >60% WT dystrophin levels display phenotypes comparable to healthy myotubes, myotubes that express <20% WT dystrophin levels display predominantly phenotypes comparable to DMD myotubes.

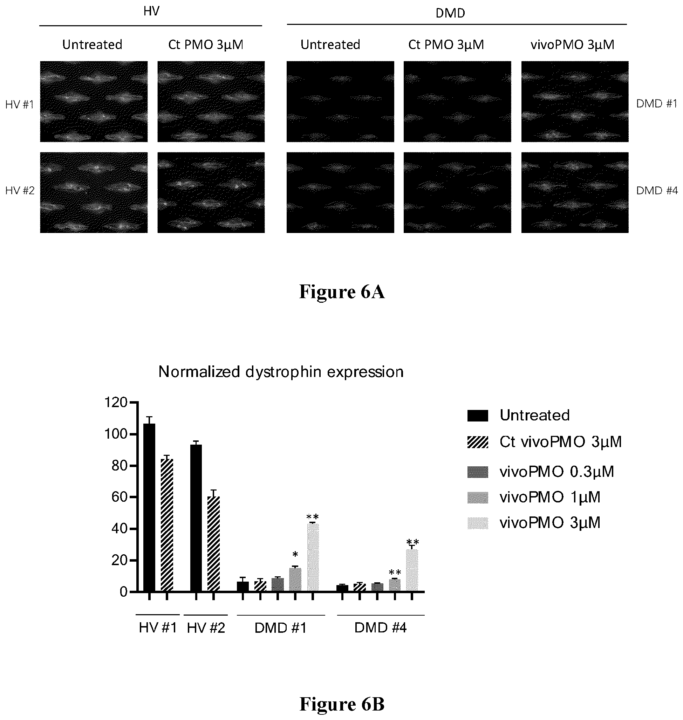

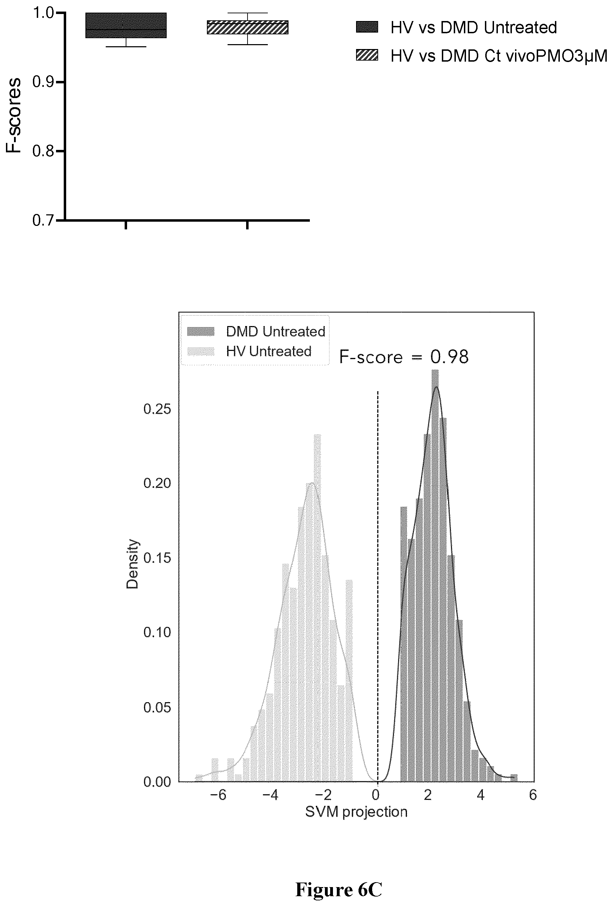

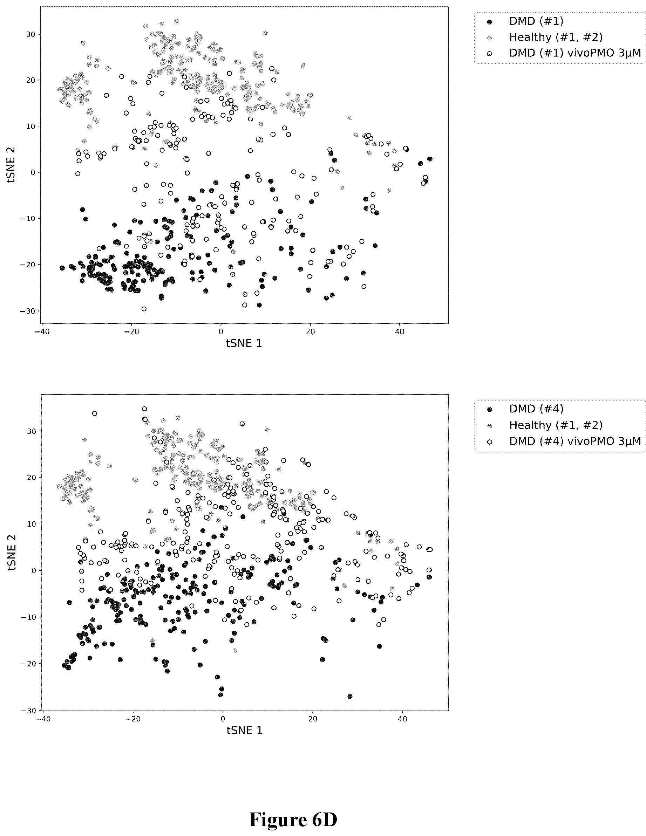

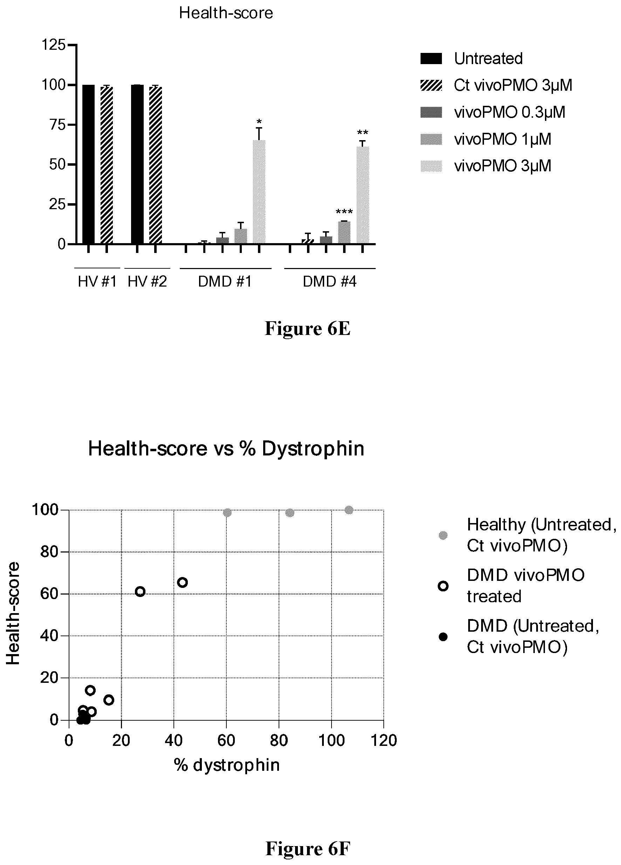

**Figure 6****:** Phenotypic response of DMD myotubes to a DMD exon skipping therapy. **(A)** Typical images of differentiated myotubes from healthy donors (HV#1, HV#2) and DMD donors (DMD #1, DMD #4) that are either untreated, treated with a control PMO (Ct vivoPMO, 3µM), or treated with a PMO that induces skipping of DMD exon 45 (vivo PMO, 3µM). The gray intensity reflects dystrophin expression. Treatment of myotubes from DMD patients with the exon 45 skipping vivoPMO partially restores DMD expression. **(B)** The graph shows the HCA quantification of dystrophin intensity normalized to the mean of healthy untreated donors. Myotubes from DMD donors were treated with 3 concentrations of the exon 45 skipping vivoPMO (0.3µM, 1µM, 3µM). One-way Anova vivoPMO treated conditions vs untreated condition, *p<0.05, **p<0.01, ***p<0.001. **(C)** Healthy and DMD cells phenotype separability: F-score from cross validation analysis using SVM linear kernel by taking the 2 different categories at a time using Utrophin and α-Sarcoglycan features. As evident from the F-score, we can separate DMD and healthy cells in all control conditions: untreated and vivoPMO control conditions. The distribution of the SVM-projection of the myotubes features shows a good separation between healthy and DMD donors. **(D)** t-SNE plots of the pair Utrophin and α-Sarcoglycan features for DMD donor #1 (upper) and DMD donor #4 (bottom). Each dot is a myotube within a pattern. Gray indicates untreated condition for healthy donors while black indicates untreated condition for the DMD donor cells. The circles indicate the vivoPMO treatment of DMD myotubes. **(E)** Using an SVM classifier trained on the untreated conditions between the DMD and the healthy donors, we predicted the exon-skipped myotubes. The y-axis shows the prediction of the classifier as healthy-like myotubes. As evident, by restoring dystrophin in the DMD donor cells, we can generate a phenotype that is similar to the healthy phenotype. One-way Anova vivoPMO treated conditions vs untreated condition, *p<0.05, **p<0.01, ***p<0.001. **(F)** Graph showing % dystrophin expression on the X-axis vs Health score on the Y-axis. Gray dots represent Untreated and control vivoPMO (Ct vivoPMO 3µM) treated myotubes from Healthy donors. Black dots represent Untreated and control vivoPMO (Ct vivoPMO 3µM) treated myotubes from DMD donors. Circles represent myotubes from DMD donors treated with the exon 45 skipping vivoPMO (0.3, 1, 3µM). The vivoPMO induced expression of dystrophin enables myotubes from DMD donors to adopt a healthy phenotype.

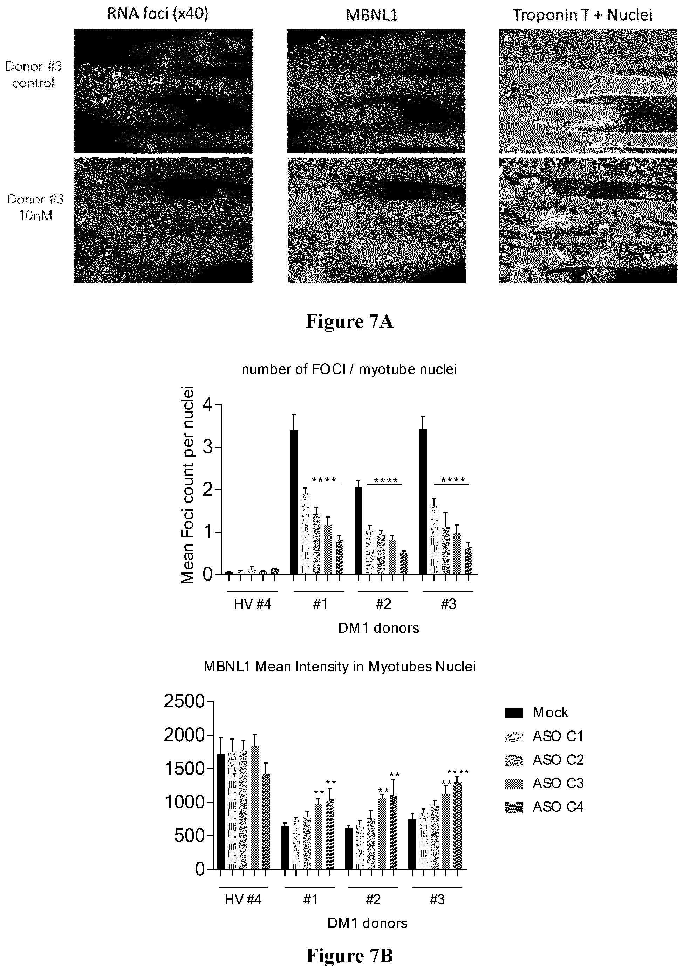

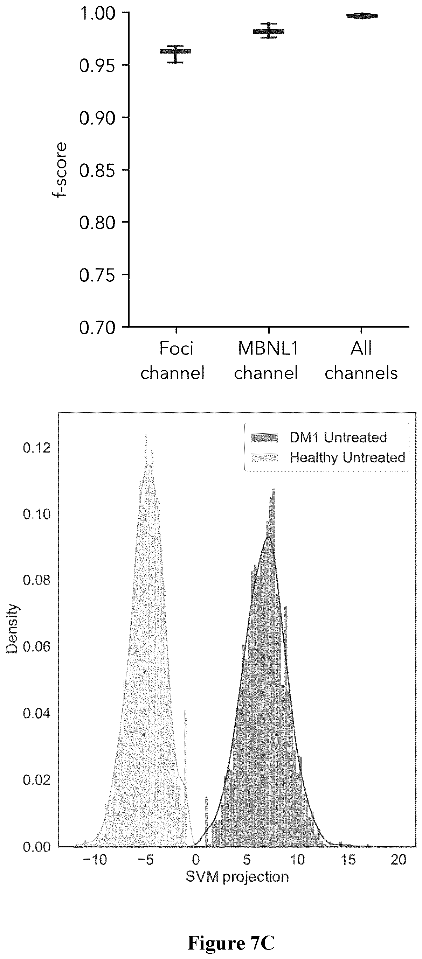

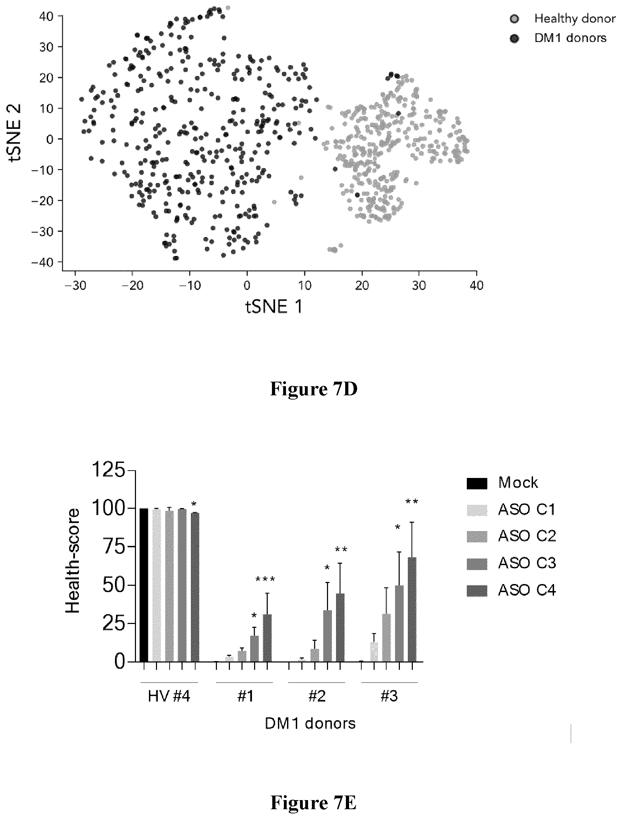

**Figure 7****:** Phenotypical response of various DM1 donors to treatment with ASOs targeting DMPK CUG extensions. **(A)** Typical images of ASO effect on DM1: DMPK RNA, MBNL1, Troponin-T and nuclei staining. **(B)** We screened an ASO at 4 concentrations (2.5nM, 5nM, 10, 20nM). ASO treatment dose-dependently decreased number of DMPK foci and restored MBNL1 expression in the three donors. **(C)** F-score from cross validation analysis using SVM linear kernel by taking the 2 different categories at a time using DMPK foci and MBNL1 based features. As evident from the F-score, we can separate the phenotypes of DM1 and healthy myotubes. The distribution of the SVM-projection of the myotubes features shows a good separation between healthy and DM1 donors. **(D)** t-SNE plot of DMPK foci and MBNL1 based features. Each dot is a nucleus within a myotube. Gray indicates untreated condition for healthy donors while shades of red indicate untreated treatment for the DM1 donor cells. **(E)** Using an SVM classifier trained on the untreated conditions between the DM1 and the healthy donors, we predicted the effect of the ASO at 4 concentrations on the 3 DM1 donors. The y-axis shows the prediction of the classifier as healthy-like myotubes. As evident, by treating with the ASO we can generate a phenotype that is similar to the healthy phenotype. One-way Anova ASO treated conditions vs mock condition, *p<0.05, **p<0.01, ***p<0.001.

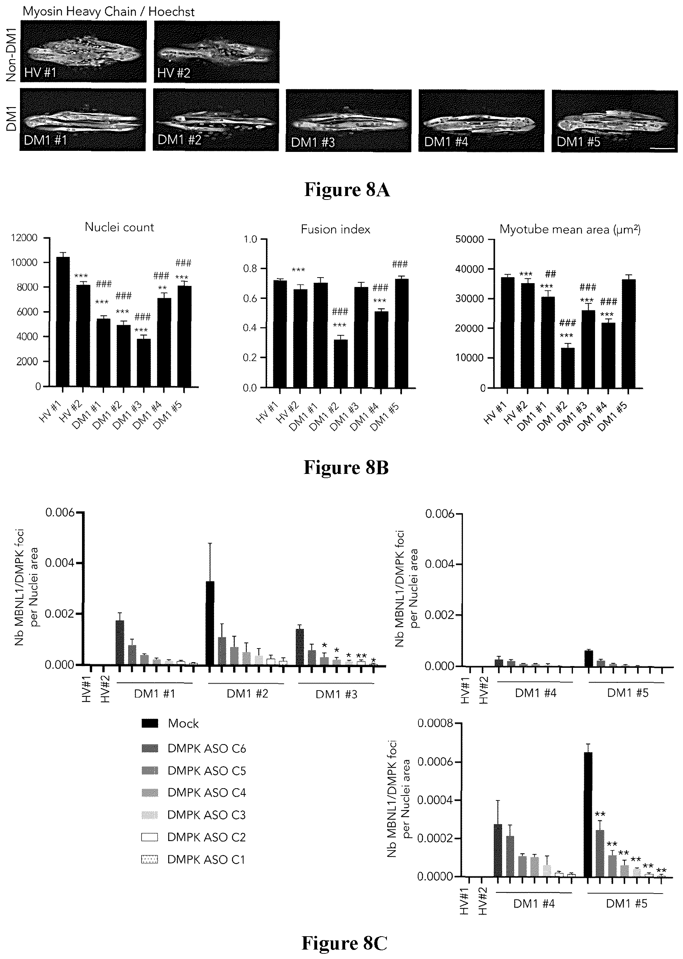

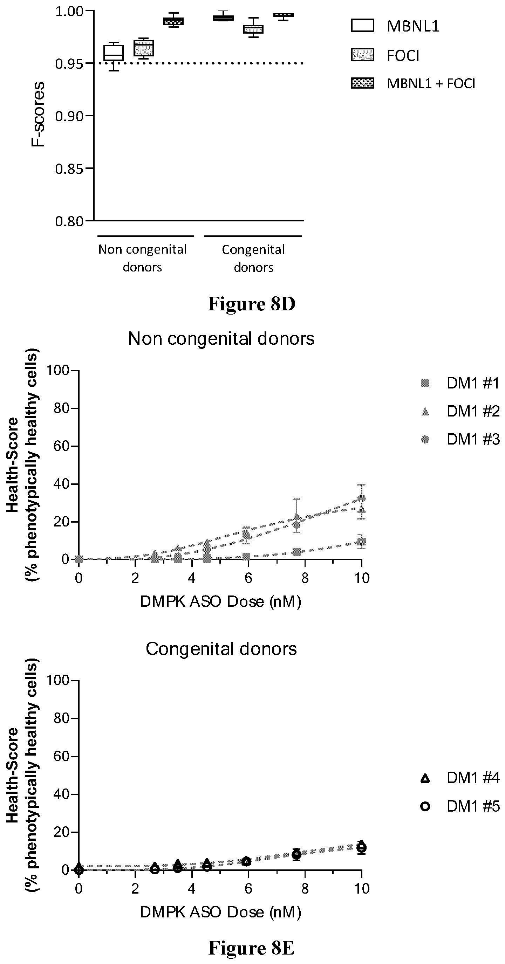

**Figure 8****: Characterization of myotubes from non-congenital and congenital DM1 donors on MyoScreen micropatterned plates.** Myotube differentiation and morphology of healthy and DM1 donor cells under MyoScreen conditions was assessed using Hoechst as a nuclei dye and myosin heavy chain (MHC) as a marker of differentiation to separate myoblasts and myotubes. **(A)** Morphology of healthy (HV #1 and #2) and non-congenital DM1 (DM1 #1, #2, #3) and congenital DM1 (DM1 #4 and #5) donors used in this study. Scale bar, 100µm. **(B)** Quantification of nuclei count, fusion index (ratio of the number of nuclei in myotubes over the total number of nuclei detected) and myotube mean area for HV and DM1 donors. Statistical significance was assessed by ANOVA and Dunnett's test for multiple comparison between HV and DM1 donors. * - ** - ***, p<0.05 - 0.01 - 0.001 for HV#1 and # - ## - ###, p<0.05 - 0.01 - 0.001 for HV#2 (n≥9 wells / condition). **(C)** The presence of DMPK mRNA foci was assessed using a (CAG)5-Cy3 FISH probe and the immunofluorescent staining of MBNL1 was used to determine the presence of MBNL1 protein trapped in the DMPK mRNA foci. DMPK ASO is used to remove the aberrant DMPK mRNA in the DM1 patients. Quantification of the number of spots co-labelled with MBNL1 and DMPK mRNA foci signal relative to the nuclei area in healthy (HV #1 and HV#2) and DM1 donors (DM1 #1-5). For the congenital donors (DM1 #4 and #5) the results are also presented at a different scale for visibility of the dose-response effect. ANOVA with Dunnett's multiple comparisons test for pairwise comparison. * - **, p<0.05 - 0.01 compared to the Mock condition for each donor. **(D)** F-score from cross validation analysis using a SVM linear kernel by taking the 2 different categories at a time using DMPK foci and MBNL1 based features in non-congenital and congenital donors. As evident from the F-score, we can separate the phenotypes of myotubes from DM1 (congenital and non-congenital donors) and healthy donors. **(E)** Using a SVM classifier trained on the untreated conditions between the DM1 and the healthy donors, we predicted the effect of ASO treatment (at 2.6, 3.5, 4.5, 5.9, 7.7, 10 nM) on the phenotypes of DM1 donors. The graph shows the health-score as a function of the DMPK ASO dose.

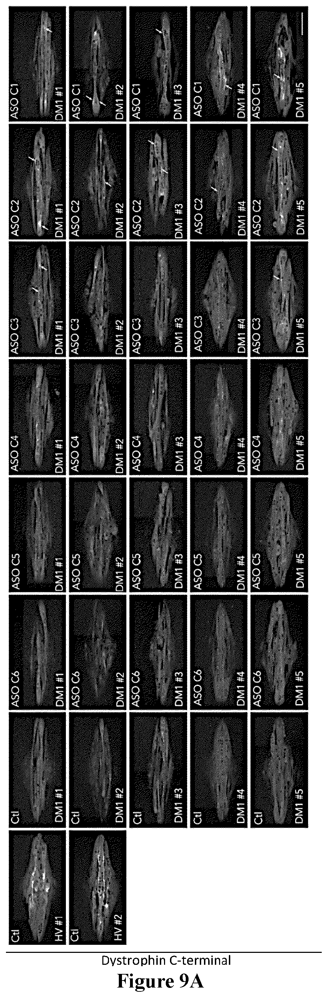

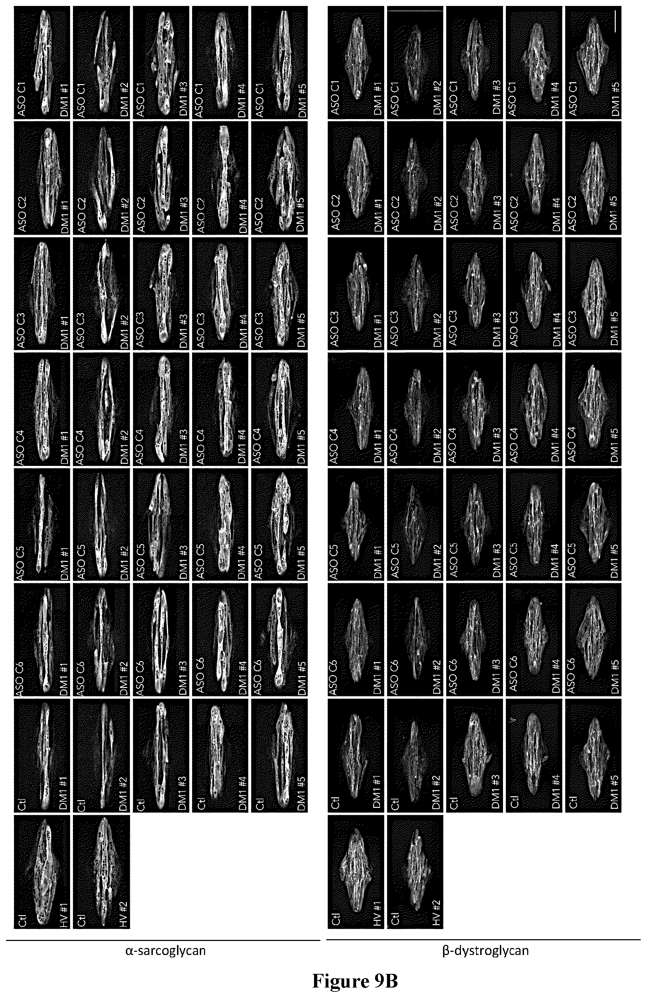

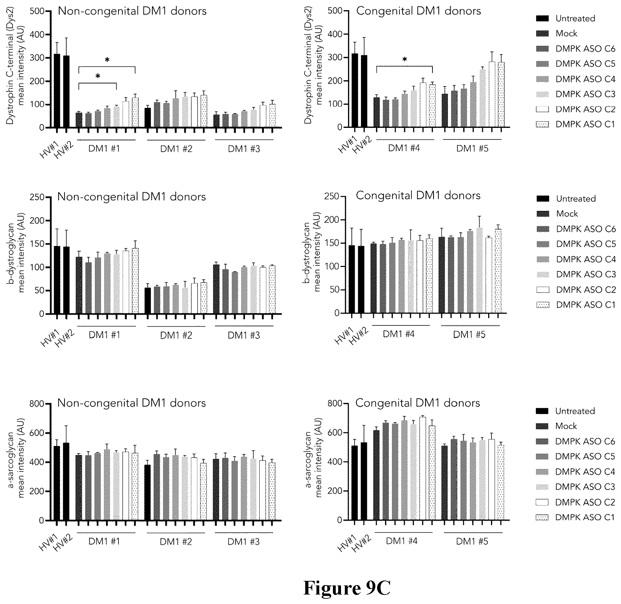

**Figure 9****: Expression of dystrophin (C-terminal domain), β-dystroglycan and α-sarcoglycan in DM1 donors treated with DMPK ASO. (A)** In DM1 patients the C-terminal domain of dystrophin is abnormally spliced, which inhibits the interaction of antibodies targeting the C-terminal domain of dystrophin. Treatment of DM1 donors with DMPK ASO partially restores the normal splicing of the dystrophin C-terminal domain. The antibody targeting the C-terminal domain of dystrophin does not show any specific sarcolemma staining in DM1 donors. Increasing DMPK ASO doses result in the restoration of dystrophin C-terminal signal in the DM1 donors (white arrows show sarcolemma positive for dystrophin signal). Scale bar, 100µm. **(B)** Expression of α-sarcoglycan (upper panel) and β-dystroglycan (lower panel) in healthy (HV#1 and #2) and DM1 donors (DM1 #1-5). Scale bar, 100 µm. **(C)** Quantification of Dystrophin, β-dystroglycan and α-sarcoglycan expression in healthy (HV#1 and #2) and DM1 donors (DM1 #1-5) treated with the DMPK ASO at 6 concentrations (2.7nM, 3.5nM, 4.6nM, 5.9nM, 7.7nM, 10nM). ANOVA with Dunnett's multiple comparisons test for pairwise comparison. *, p<0.05 compared to the Mock condition for each donor.

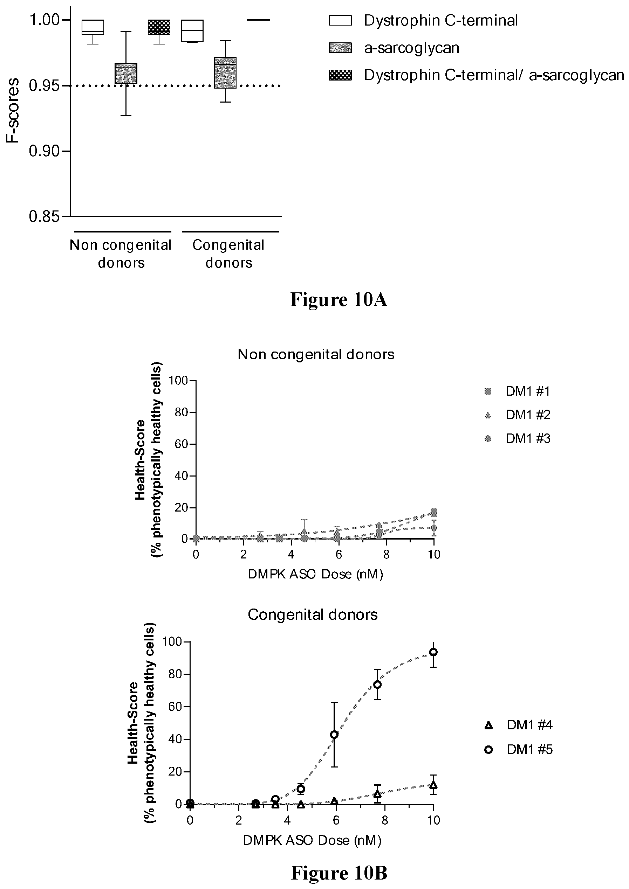

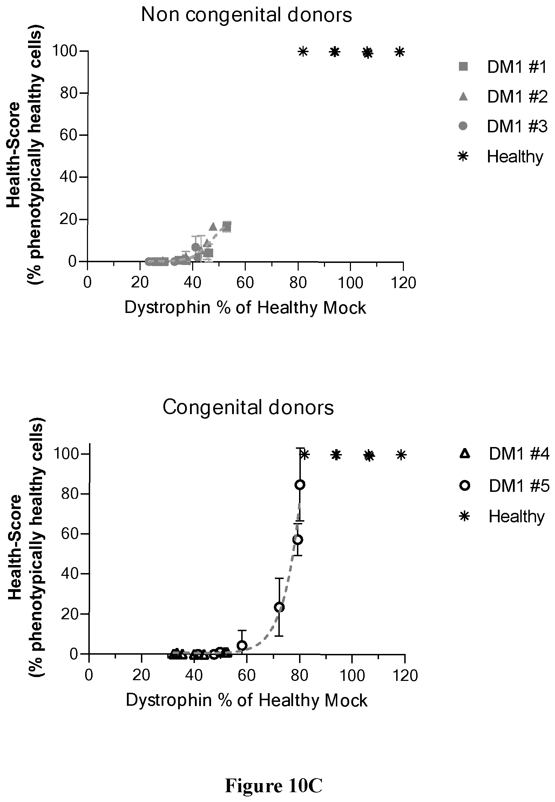

**Figure 10****: Cell profiling analysis of DMPK ASO-treated DM1 donors monitoring dystrophin (C-terminal domain) and α-sarcoglycan. (A)** Separation of myotube phenotypes of untreated healthy and DM1 donors labeled for dystrophin (C-terminal domain), α-sarcoglycan, or a combination of both as a result of machine learning (F-score). Labeling cells for both dystrophin (C-terminal domain) and α-sarcoglycan resulted in the highest F-score. **(B)** Using a SVM classifier trained on the combination of dystrophin (C-terminal domain) and α-sarcoglycan markers on untreated conditions between the DM1 and the healthy donors, we predicted the effect of DMPK ASO treatment at 6 concentrations on the DM1 donors. **(C)** Health score predictions shown in Figure 10 B presented as a function of correctly-spliced dystrophin levels (% untreated healthy donors).

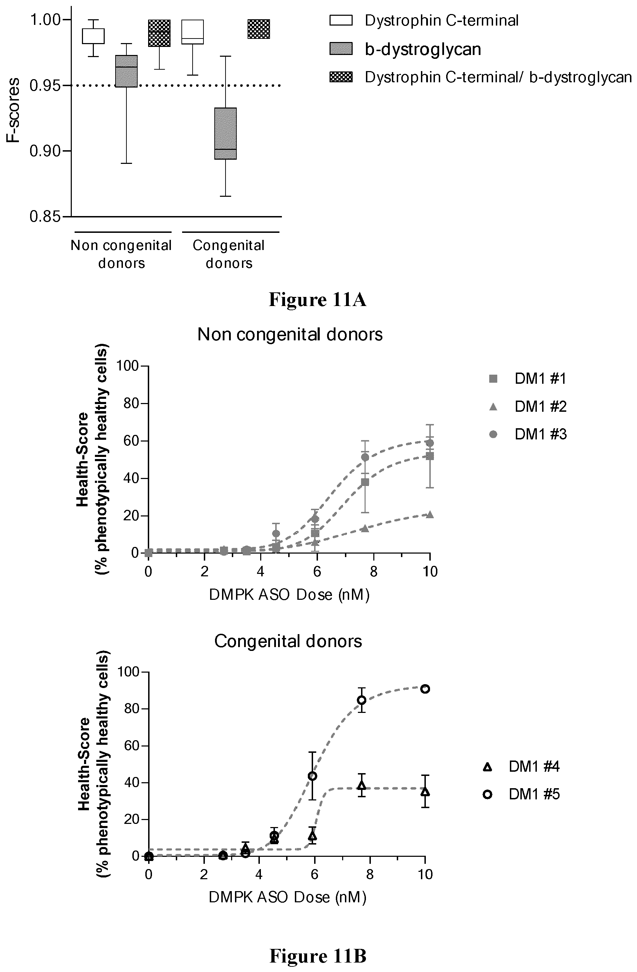

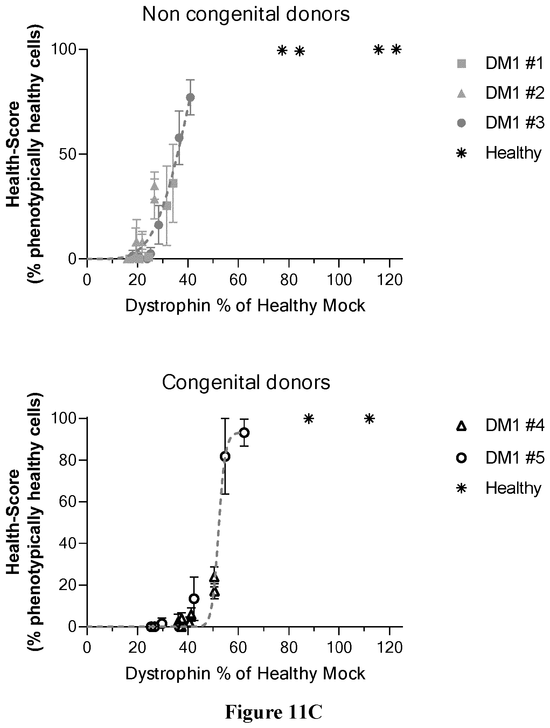

**Figure 11****: Cell profiling analysis of DMPK ASO-treated DM1 donors monitoring dystrophin (C-terminal domain) and β-dystroglycan. (A)** Separation of myotube phenotypes of untreated healthy and DM1 donors labeled for dystrophin (C-terminal domain), b-dystroglycan, or a combination of both as a result of machine learning (F-score). Labeling cells for both dystrophin (C-terminal domain) and b-dystroglycan resulted in the highest F-score. **(B)** Using a SVM classifier trained on the combination of dystrophin (C-terminal domain) and β-dystroglycan markers on untreated conditions between the DM1 and the healthy donors, we predicted the effect of DMPK ASO treatment at 6 concentrations on the DM1 donors. Health-score as a function of the of the ASO concentration. **(C)** Health score predictions shown in Figure 11 B presented as a function of correctly-spliced dystrophin levels (% untreated healthy donors).

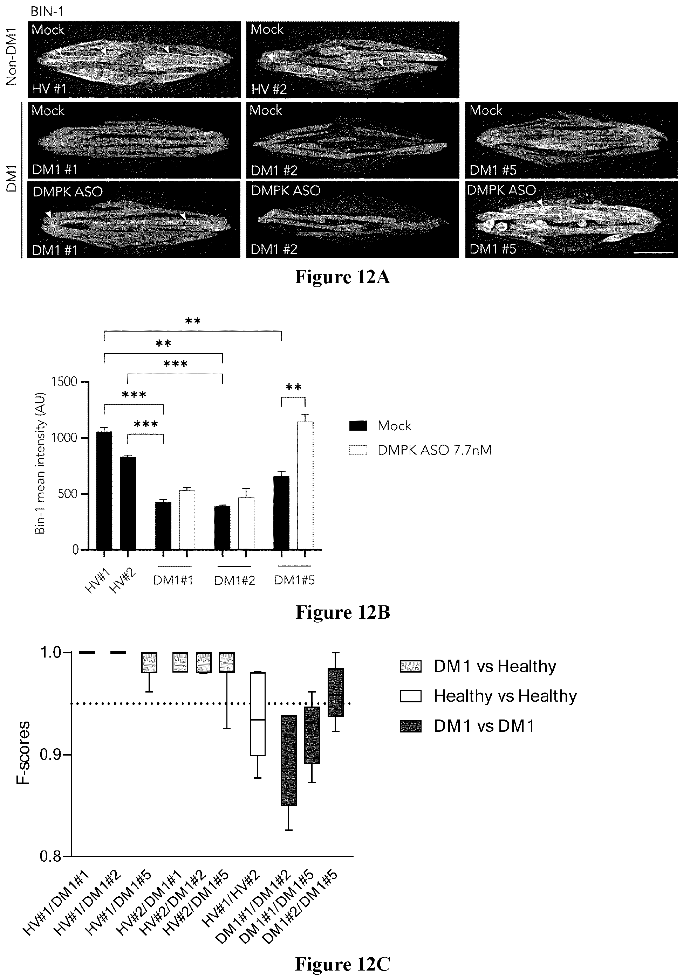

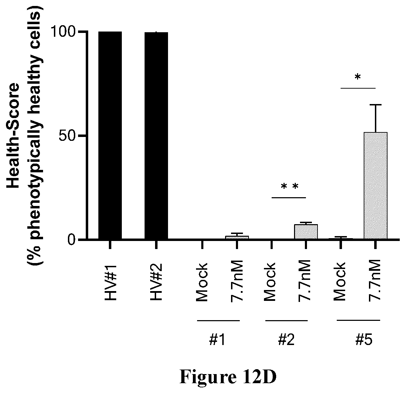

**Figure 12****: Cell profiling analysis of DMPK ASO-treated DM1 donors monitoring BIN-1. The loss of MBNL1 activity in DM1 patients results in a mis-splicing of** BIN1 exon11, which results in the expression of an inactive form of BIN1. The miss-spliced form of BIN1 localization is different from the correctly spliced form and can be distinguished by immunofluorescent staining using specific BIN1 antibodies. **(A)** BIN-1 expression in mock-treated healthy (HV#1, #2) and DM1 (DM1#1, #2 and #5) donors. Arrowheads show the presence of BIN1 at the sarcolemma. BIN-1 expression is reduced and sarcolemmal localization is lost in DM1 donors. Treatment with DMPK ASO (7.7nM) rescues BIN-1 localization at the sarcolemma. Scale bar, 100µm. **(B)** Quantification of BIN-1 expression in healthy (HV#1, #2) and DM1 (DM1#1, #2 and #5) donors treated with DMPK ASO (7.7nM). ANOVA with Dunnett's multiple comparisons test for pairwise comparison. ** - ***, p<0.01 - 0.001 compared to the Mock condition for each donor. DM1 donor #5 showed a marked restoration of BIN-1 expression upon treatment with the DMPK ASO. **(C)** Separation of phenotypes of myotubes from healthy and DM1 donors by monitoring BIN-1: F-score from cross validation analysis using SVM kernel by comparing BIN-1 based features between individual healthy and DM1 donors, healthy donors, and DM1 donors. As evident from the F-score, monitoring BIN-1 distinguishes between DM1 and healthy donors, but not between DM1 donors or between healthy donors. **(D)** Predicting the effect of DMPK ASO treatment on the phenotypes of myotubes from DM1 donors using a SVM classifier trained on BIN-1 on mock conditions between the DM1 and the healthy donors. The figure shows the health-score observed in mock and DMPK ASO (7.7 nM)-treated myotubes from healthy and DM1 donors. The DMPK ASO treatment partially restored a healthy phenotype in DM1 donors. The degree of restoration was donor-dependent. ANOVA with Dunnett's multiple comparisons test for pairwise comparison. ** - ***, p<0.01 - 0.001 compared to the Mock condition for each donor.

### DETAILED DESCRIPTION OF THE INVENTION

High-throughput cell profiling monitors a wide range of phenotypic responses in cells that can be quantified and analyzed, enabling the assessment of a cellular response to external perturbations in individual cells (Perlman et al., 2004, Science 306, 1194-1198). To monitor these phenotypic responses, cells are usually labeled with fluorescent antibodies to specific proteins and the nucleus, cytoplasm and/or other regions are identified by image processing. An investigator then defines a set of descriptors to track changes which are processed through data analyses programs capable of learning to extract a cellular phenotype.

The inventors herein demonstrated that by labeling myotubes for selective, disease-dependent markers, cell profiling algorithms can be derived that phenotypically distinguish myotubes derived from healthy subjects and myotubes derived from patients suffering from neuromuscular disorders. These cell profiling assays can be used to quantitatively assess the ability of a therapeutic agent to phenotypically convert diseased cells into healthy cells without requiring any prior knowledge of the mechanism of action of said therapeutic agent. They can also be used to monitor the response to a therapeutic compound, to select a patient for a treatment with a therapeutic compound, to determine whether a patient is susceptible to benefit from a treatment with a therapeutic compound or to diagnose a neuromuscular disorder. These methods are applicable to a wide variety of neuromuscular diseases and open novel opportunities for the functional assessment of therapies in primary, patient-derived myotubes.

In a first aspect, the present invention relates to a computer-implemented method of training a classifier for accurately distinguishing between healthy myotubes and myotubes exhibiting features of a neuromuscular disorder of interest.

Said method comprises
a) providing a training set of images of stained myotubes, or preprocessed information obtained from said training set of images, to a classifier, the training set of images comprising images of healthy myotubes and of myotubes exhibiting features of a neuromuscular disorder of interest ("diseased myotube"), said myotubes being stained for an imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one region of interest (ROI) selected from the group consisting of individual myotubes, structures of myotubes, and any combination thereof;
b) generating an output of the classifier for each input ROI, said output classifying the input ROI as associated to a healthy or diseased myotube;
c) comparing the generated output for each input ROI to a label associated with said input ROI said label comprising an indication of the healthy or diseased status of the myotube corresponding to said input ROI;
d) evaluating the classifier's accuracy for distinguishing between healthy myotubes and diseased myotubes,
wherein the classifier is considered as an accurate classifier to distinguish between healthy myotubes and diseased myotubes if it exhibits an accuracy corresponding to a F-score equal to or greater than 0.9.

As used herein, the term "computer-implemented method" refers to a method which involves a programmable apparatus, specifically a computer, a computer network, or a readable medium carrying a computer program, whereby at least one step of the method is performed by using at least one computer program. A computer-implemented method may further comprise at least one step that is not performed by using a computer program, e.g. a cell culture step.

In step a) of the method, a training set of images of stained myotubes, or preprocessed information obtained from said training set of images, is provided to a classifier.

As used herein, the term "classifier" refers to an algorithm that implements classification, i.e. that can determine a likelihood score or a probability that an object classifies with a group of objects (e.g., a group of healthy myotubes) as opposed to one or several other groups of objects (e.g., a group of diseased myotubes) and that maps said input object (e.g. input ROI) to a category (e.g. healthy or diseased myotubes). This term may refer to one or multiple classifiers. For example, multiple classifiers may be trained, which may process data in parallel and/or as a pipeline. For example, output of one type of classifier (e.g., from intermediate layers of a neural network) may be fed as input into another type of classifier.

Examples of classifiers that can be used in the present invention include, but are not limited to, neural networks of various architectures (e.g., artificial, deep, convolutional, fully connected) and supervised machine learning classifiers such as Support Vector Machine (SVM) classifier, random forest classifier, decision tree classifier, K-nearest neighbor classifier (KNN), logistic regression classifier, nearest neighbor classifier, Gaussian mixture model (GMM), nearest centroid classifier and linear regression classifier. It is not an exhaustive list and the skilled person in the art will also notice similar algorithms that can be equally used, although they are not specifically mentioned here. Details and rules of functioning of the mentioned algorithms have already been widely described in the literature. The most important contribution is the set of input data provided to the classifier (i.e. a set of images or preprocessed information obtained from a set of images). Based on this input data, it is possible to create a suitable model using any appropriate supervised machine learning techniques. The selection of appropriate algorithms is therefore of secondary nature and can be carried out in many different ways and in various combinations obvious to those skilled in the art.

Preferably, the classifier is selected from Support Vector Machine (SVM) classifier, random forest (RF) classifier, decision tree classifier, K-nearest neighbor classifier (KNN), logistic regression classifier, nearest neighbor classifier, Gaussian mixture model (GMM), nearest centroid classifier, linear regression classifier, and neural networks such as artificial, deep, convolutional, fully connected neural networks. More preferably, the classifier is selected from Support Vector Machine (SVM) classifier, random forest (RF) classifier and neural networks, in particular convolutional neural network (CNN). Even more preferably, the classifier is SVM classifier.

A classifier utilizes some training data to understand how given input objects relate to a category or another. The classifier may be provided with a training set of images of stained myotubes, said training set comprising images of healthy myotubes and of myotubes exhibiting features of a neuromuscular disorder of interest ("diseased myotube"). Alternatively, the classifier may be provided with preprocessed information obtained from such a training set of images.

Stained and imaged myotubes may be obtained by culturing myoblasts on a suitable substrate and under suitable conditions known by the skilled person. A myoblast is a mononucleate cell type that, by fusion with other myoblasts, gives rise to myotubes that maturate and later eventually develop into muscle fibers. Myotubes used in the present invention are thus *in vitro* cultured myotubes. Preferably, they are not organized in tissue.

The myoblasts or myotubes used in the present invention are preferably mammalian cells, more preferably human cells.

Methods for producing myotubes by culturing myoblasts are well known by the skilled person and include cultures on patterned or unpatterned substrates, on substrates with or without topological constraints, on soft substrates (e.g. synthetic hydrogels materials such as poly(hydroxyethyl methacrylate), polyacrylamide, polyethylene glycol, polyacrylic acid, poly(vinyl alcohol), polyvinylpyrrolidone, polyimide and polyurethane, natural hydrogel materials such as agarose, dextran, gelatin and matrigel, and silicone materials), or hard substrates (e.g. glass, silicone or plastics such as polystyrene, polypropylene, polyethylene), on plates or in wells (see e.g. WO 2016/202850, WO 2016/139312, WO 2015/091593, EP 1 882 736 , EP 2 180 042, EP 1 664 266 and US 2008/299086 patent applications).

Myoblasts are cultured on a substrate allowing the production of homogeneous population of myotubes, in particular cultured in constrained conditions. Myotubes of a homogeneous population of myotubes exhibits similar morphological parameters such as the fusion index (ratio of nuclei within myotubes of the total number on nuclei), the maturation index (number of nuclei per myotubes), the orientation angle, the width/length ratio, the width and the length of the myotubes.

In preferred embodiments, myotubes of a homogeneous population of myotubes exhibit similar fusion index, maturation index, and myotube area (or width/length ratio, width and/or length). Preferably, they also exhibit similar orientation angle. In particular, myotubes used in the present invention may exhibit a variation of fusion index, maturation index and myotube area (or width/length ratio, width and/or length) of less than 30 %, preferably of less than 20 %.

Typically, a culture in constrained conditions may be a culture on a substrate with topological constraints or adhesive patterns. Such substrates allowing the production of homogeneous population of myotubes are well known by the skilled person. In particular, homogeneous population of myotubes may be obtained by culturing myoblasts on patterned substrates that promote self-assembly of myoblasts into myotubes and enhance a specific orientation of myotubes. Examples of said patterns include, but are not limited to, linear grooves formed in the surface of a substrate by an etching technique (Yamamoto et al., 2008, J. Histochem. Cytochem 56, 881-892), and adhesive patterns forming lines, geometrical shapes such as circular, square and Y-shaped (Junkin et al. 2011, Journal of Cell Science 124, 4213-4220), adhesive "hybrid" patterns that consist of the combination of a linear element and an arcuate element centered on the linear element (Bajaj et al., 2011, Integrative Biology 3, 897-909) or adhesive patterns disclosed in WO 2015/091593, WO 2016/202850 and WO 2016/139312, Young et al. 2018 (Young et al. SLAS Discov. 2018 Sep;23(8):790-806). Preferably, the myoblasts are cultured on a substrate containing adhesive patterns, preferably adhesive patterns as disclosed in WO 2015/091593, WO 2016/202850 and WO 2016/139312, and in particular as disclosed in Figure 2A of International patent application WO 2015/091593 and in Young et al. 2018 (Young et al. SLAS Discov. 2018 Sep;23(8):790-806). Adhesive properties of patterns may be obtained by coating said patterns with one or several extracellular matrix proteins, preferably with fibronectin.

Preferably, the method of culturing myotubes is adaptable to high-throughput platforms and to perform high-throughput assays.

Healthy myotubes may be obtained by culturing myoblasts derived from at least one healthy subject, i.e. a subject who does not suffer from any muscular disease and in particular from any neuromuscular disorder as defined below. "Diseased myotubes" may be obtained by culturing myoblasts derived from at least one patient suffering from a neuromuscular disorder of interest. Alternatively, diseased myotubes may be obtained by culturing myoblasts which have been chemically or genetically modified to not express a molecule known as a disease driver or to express a mutated form thereof. For example, for DMD, diseased myotubes may be obtained by culturing myoblasts modified to not express dystrophin or to express a truncated form thereof. Such modifications may be obtained by any method known by the skilled person such as RNA interference using siRNAs specifically targeting the gene of interest, or genetic alteration of the gene of interest.

As used herein, the terms "subject", "individual" and "patient" are interchangeable and preferably refer to an animal, more preferably a mammal and even more preferably a human, including adult, child, newborns and human at the prenatal stage.

As used herein, the term "neuromuscular disorder", "neuromuscular disease" and "muscular disease" are used interchangeably and cover disorders that impair the functioning of the muscles, either directly, being pathologies of the voluntary muscle, or indirectly, being pathologies of nerves, neuromuscular junctions, or of the extracellular matrix. This term encompasses a wide range of disorders including, but not limited to, muscular dystrophies such as selected from the group consisting of Duchenne Muscular Dystrophy (DMD), Becker Muscular Dystrophy (BMD), Myotonic Dystrophy 1 (DM1), Myotonic Dystrophy 2 (DM2), Facioscapulohumeral Muscular Dystrophy (FSHD), Emery-Dreifuss muscular dystrophy, Limb-girdle muscular dystrophies, Walker-Warburg syndrome, Muscle-eye-brain disease, Congenital muscular dystrophy, Scapuloperoneal muscular dystrophy, Tibial muscular dystrophy and Autosomal Recessive Muscular Dystrophy; myopathies such as Bethlem & Ullrich myopathy, Myofibrillar myopathy, Distal myopathy, Rimmed vacuole myopathy, Centronuclear myopathy (CNM), X-linked myotubular myopathy (XLMTM), Tubular aggregate myopathy, Malignant hyperthermia syndrome, Inclusion body myopathy, Myofibrillar myopathy, Protein aggregate myopathy, Nemaline myopathy, Congenital myopathy (CM), Myoshi myopathy, Vici syndrome, X-linked myopathy with excessive autophagy, Danon disease, Pompe disease and Primary mitochondrial myopathies; congenital myasthenic syndromes such as myasthenia gravis and other myasthenic syndromes driven by mutations in CHAT, COLQ, RAPSN, CHRNE, DOK7 and/or GFPT1 genes; or motor neuron diseases such as Spinal Muscular Atrophy (SMA), Amyotrophic Lateral Sclerosis (ALS), Kennedy's disease, cachexia, sarcopenia and muscle atrophy.

In an embodiment, the neuromuscular disorder of interest is selected from the group consisting of muscular dystrophies, myopathies, congenital myasthenic syndromes, motor neuron diseases and metabolic muscle disorders. Preferably, the neuromuscular disorder of interest is selected from the group consisting of muscular dystrophies, myopathies, congenital myasthenic syndromes and motor neuron diseases.

In a particular embodiment, the neuromuscular disorder of interest is a muscular dystrophy, in particular a muscular dystrophy selected from the group consisting of Duchenne Muscular Dystrophy (DMD), Becker Muscular Dystrophy (BMD), Myotonic Dystrophy 1 (DM1), Myotonic Dystrophy 2 (DM2), Facioscapulohumeral Muscular Dystrophy (FSHD), Emery-Dreifuss muscular dystrophy, Limb-girdle muscular dystrophies, Walker-Warburg syndrome, Muscle-eye-brain disease, Congenital muscular dystrophy, Scapuloperoneal muscular dystrophy, Tibial muscular dystrophy and Autosomal Recessive Muscular Dystrophy. Preferably, the neuromuscular disorder of interest is a muscular dystrophy selected from the group consisting of Duchenne Muscular Dystrophy (DMD) and Myotonic Dystrophy 1 (DM1).

In another particular embodiment, the neuromuscular disorder of interest is a myopathy, in particular a myopathy selected from the group consisting of Bethlem & Ullrich myopathy, Myofibrillar myopathy, Distal myopathy, Rimmed vacuole myopathy, Centronuclear myopathy (CNM), X-linked myotubular myopathy (XLMTM), Tubular aggregate myopathy, Malignant hyperthermia syndrome, Inclusion body myopathy, Myofibrillar myopathy, Protein aggregate myopathy, Nemaline myopathy, Congenital myopathy (CM), Myoshi myopathy, Vici syndrome, X-linked myopathy with excessive autophagy, Danon disease, Pompe disease and Primary mitochondrial myopathies.

In another particular embodiment, the neuromuscular disorder of interest is a congenital myasthenic syndrome, in particular a congenital myasthenic syndrome selected from the group consisting of Myasthenia gravis and other myasthenic syndromes driven by mutations in CHAT, COLQ, RAPSN, CHRNE, DOK7 and/or GFPT1 genes.

In a further particular embodiment, the neuromuscular disorder of interest is a motor neuron disease, in particular a motor neuron disease selected from the group consisting of Spinal Muscular Atrophy (SMA), Amyotrophic Lateral Sclerosis (ALS) and Kennedy's disease.

In a further embodiment, the neuromuscular disorder of interest is a metabolic muscle disorder, in particular a metabolic muscle disorder selected from the group consisting of cachexia, sarcopenia and muscle atrophy.

Before being imaged, the myotubes are stained for an imaging marker and with at least one labelling agent revealing at least one region of interest (ROI). This staining may be performed during or after the culture of myotubes.

As used herein, the term "labelling agent" refers to any agent that is used to detect and label an imaging marker or to reveal a ROI. Said agent emits a signal that is visible on the captured images of stained myotubes. A labelling agent is able to specifically recognize the target (i.e. an imaging marker or a ROI) and to emit a detectable signal, e.g. a fluorescent, luminescent, chemiluminescent or radioactive signal, preferably a fluorescent signal. A labelling agent may comprise a moiety which is able to specifically recognize the target, i.e. an antibody or nucleic acid moiety, and a moiety emitting a detectable signal, i.e. a fluorochrome. In some embodiments, these moieties are covalently linked, e.g. an antibody or nucleic acid specifically recognizing the target and bearing a fluorochrome. In some other preferred embodiments, these moieties are born by two or more distinct molecules, e.g. a primary antibody or nucleic acid specifically recognizing the target and a secondary antibody or nucleic acid specifically recognizing the primary antibody or nucleic acid and emitting a detectable signal, e.g. bearing a fluorochrome. Such labelling agent may be for example an antibody or nucleic acid probe conjugated to a fluorochrome or the combination of a primary antibody or nucleic acid specifically recognizing the target and a secondary antibody or nucleic acid specifically recognizing the primary antibody or nucleic acid and conjugated to a fluorochrome (i.e. immunostaining). Alternatively, a labelling agent may be a molecule emitting a detectable signal, e.g. a fluorescent dye, that naturally bind to the target, e.g. DAPI that naturally binds to DNA. Typically, each labelling agent emits a detectable and distinctive signal, i.e. a signal which can be distinguished from the signal of the other labelling agents used to stain the cells. Preferably, each labelling agent comprises a different fluorescence label having a different emission and/or excitation wavelength.

As used herein, the term "imaging marker" refers to one or several molecules (e.g. nucleic acids, proteins, lipids, carbohydrates) and/or morphological features of the myotubes that can be revealed by using one or several labelling agents and that are then used to extract features from captured images of myotubes stained with said labelling agents. An imaging marker is a marker associated with a neuromuscular disorder of interest, i.e. a marker which allows, using the method of the present invention, distinguishing between healthy myotubes and diseased myotubes. The imaging marker for a neuromuscular disorder of interest may be identified using the method of invention of identifying an imaging marker of a neuromuscular disorder of interest as detailed below.

In particular, the imaging marker may be selected from the group consisting of identified disease driver(s) of the neuromuscular disorder of interest, proteins associated with specific functional or structural properties of muscle cells, cell morphological features affected in neuromuscular disorders, and any combination thereof. Disease drivers and proteins may vary depending on the species from which the myotubes originate. In embodiments wherein the myotubes are human myotubes, identified disease driver(s) of the neuromuscular disorder of interest and proteins associated with specific functional or structural properties of muscle cells are human disease driver(s) and human proteins, in particular as listed in Tables 1 and 2. For other species, the identified disease driver(s) of the neuromuscular disorder of interest and proteins associated with specific functional or structural properties of muscle cells may be ortholog disease driver(s) or orthologous proteins. The term "ortholog" or "orthologous protein" as used herein refers to a functional counterpart of a protein or disease driver in another species. Orthologous proteins or genes are similar to each other because they originated from a common ancestor. Sequence differences between the orthologs are thus the result of speciation. Methods for identification of orthologous proteins are well known in the art.

As used herein, the term "identified disease driver(s) of the neuromuscular disorder of interest" relates to a known genetic or physiological cause of a neuromuscular disorder.

Examples of such drivers include, but are not limited to, those listed in Table 1 below.

**Table 1: identified disease driver(s) of neuromuscular disorders**

| **Disease Class** | **Disease** | **disease driver(s)** |
|---|---|---|
| Muscular Dystrophies | Duchenne Muscular Dystrophy (DMD) | DMD gene |
| | Becker Muscular Dystrophy (BMD) | DMD gene |
| | Myotonic Dystrophy 1 (DM1) | DMPK gene |
| | Myotonic Dystrophy 2 (DM2) | ZnF9 gene |
| | Facioscapulohumeral Muscular Dystrophy (FSHD) | Dux4 gene |
| | Emery-Dreifuss muscular dystrophy | LMNA, EMD and/or SYNE1/2 genes |
| | Limb-girdle muscular dystrophies | SGCA, SGCB, SGCG, SGCD, DAG1, LAMA2, DES, MYOT, TTN, LMNA, CAV3, TNPO3, CAPN3, DYSF, ANOS, TRIM32 and/or DNAJB6 genes |
| | Walker-Warburg syndrome | DAG1 gene |
| | Muscle-eye-brain disease | DAG1 gene |
| | Congenital muscular dystrophy | LAMA2 and/or LMNA genes |
| | Scapuloperoneal muscular dystrophy | FHL1 and/or TRIM32 genes |
| | Tibial muscular dystrophy | TTN gene |
| | Autosomal Recessive Muscular Dystrophy | DAG1 gene |
| | Bethlem & Ullrich myopathy | COL6A1, COL6A2 and/or COL6A3 genes |
| | Myofibrillar myopathy | FLMNC, HSPBS and/or BAG3 genes |
| | Distal myopathy | MYH7, DES, MYOT and/or TTN genes |
| | Rimmed vacuole myopathy | MYH7 and/or SQSTM1 genes |
| | Centronuclear myopathy (CNM) | BIN-1, DNM2 and/or SPEG genes |
| | X-linked myotubular myopathy (XLMTM) | MTM1 gene |
| | Tubular aggregate myopathy | STIM1 gene |
| | Malignant hyperthermia syndrome | STAC3, RYR1 and/or CACNA1S genes |
| Myopathies | Inclusion body myopathy | MYH2 gene |
| | Myofibrillar myopathy | DES, MYOT and/or SQSTM1 genes |
| | Protein aggregate myopathy | MYOT, TTN and/or MuRF1 (TRIM63) genes |
| | Nemaline myopathy | ACTA1, NEB, TPM2, TPM3, TNNT1, TNNT3, TNNI2, LMOD3, CFL2, KBTBD13, KLHL40 and/or KLHL41 genes |
| | Congenital myopathy (CM) | MYH2, DES, ACTA1, NEB, BIN1, DNM2, MTM1, RYR1, CACNA1S and/or SPEG genes |
| | Myoshi myopathy | DYSF gene |
| | Vici syndrome | EPG5 gene |
| | X-linked myopathy with excessive autophagy | VMA21 gene |
| | Danon disease | LAMP2 gene |
| | Pompe disease | GAA gene |
| | Primary mitochondrial myopathies | TP53, NRAS, SNX31, NF1, KIT and/or APC genes |
| Congenital Myasthenic Syndromes | Myasthenia gravis | Presence of antibodies directed against MuSK or AchR |
| | Other Myasthenic syndromes | CHAT, COLQ, RAPSN, CHRNE, DOK7 and/or GFPT1 genes |
| Motor neuron diseases | Spinal Muscular Atrophy (SMA) | SMN1 gene |
| | ALS | SOD1, C9ORF72, TARDBP and/or FUS genes |
| | Kennedy's disease | AR gene |

In some embodiments, identified disease drivers of the neuromuscular disorder of interest are one or several of those listed in Table 1 and corresponding to the neuromuscular disorder.

In some particular embodiments, the imaging marker comprises, or consists of, one or several identified disease driver(s) of the neuromuscular disorder of interest.

Labelling agents used to stain myotubes for these markers depend on the nature of the markers and may be easily determined by the skilled person. In particular, in embodiments wherein the marker is a gene, the labelling agent may be or comprise an antibody directed against the wild-type or mutated protein encoded by said gene or may be or comprise a nucleic acid probe which specifically hybridizes on the wild-type or mutated gene. In embodiments wherein the marker is the presence of a specific antibody (e.g. for myasthenia gravis), the labelling agent may be or comprise an antibody directed against said antibody (e.g. anti-MuSK or anti-AchR antibody).

As used herein, the term "proteins associated with specific functional or structural properties of muscle cells" relates to proteins known to be involved in muscle structural integrity/contractibility such as proteins belonging to ECM-Sarcolemma connection (dystrophin-associated protein complex or DAPC), sarcomere and sarcolemma-nuclear envelope connection; proteins known to be involved neuro-muscular junctions or excitation-contraction coupling such as proteins involved in T-tubule biogenesis/ endosomal trafficking, Ca2+ homeostasis, or belonging to triad structure or channels; proteins known to be involved in muscle membrane repair and proteins known to be involved in protein turnover in muscle cells.

Examples of such proteins include, but are not limited to, those listed in Table 2 below.

**Table 2: list of proteins associated with specific functional or structural properties of muscle cells**

| **Muscle Function** | **Structure** | **Associated Proteins (markers)** |
|---|---|---|
| | | Dystrophin |
| | | α, β DAG |
| | | Sarcospan |
| | | α, β Syntrophin |
| | | α, β, γ, δ Sarcoglycan |
| | | Dystrobrevin |
| | | nNOS |
| | ECM-Sarcolemma connection (DAPC) | Ankyrin |
| | | Collagen I, III, IV, VI |
| | | Laminin |
| | | Filamin C |
| | | Perlecan |
| | | Integrin β1 |
| | | Actin |
| | | α Tubulin |
| | | Utrophin |
| Muscle structural integrity/ Contractibility | Sarcomere | Nebulin |
| | | Tropomyosin |
| | | Actin |
| | | Tropomodulin |
| | | Leiomodulin |
| | | Troponin (TNNC, TNNT, TNNI) |
| | | Myosin (MyHC, MyELC, MyRLC) |
| | | Titin |
| | | Dystrophin |
| | | F-actin |
| | | Nesprin |
| | Sarcolemma-Nuclear envelope connection | Desmin |
| | | SUN1/2 |
| | | Lamin A/C |
| | | Emerin |
| | | BAF1 |
| | | LAP1 |
| | | Plectin |
| | T-tubule biogenesis/ endosomal trafficking | DNM2 |
| | | BIN1 |
| | | CAV3 |
| | | SPEG |
| Neuro-muscular junctions/ Excitation-contraction coupling | | MTM1 |
| | | Rab |
| | Triad | DHPR |
| | | STAC3 |
| | | RYR1 |
| | | Triadin |
| | | Junctin |
| | | CAPN3 |
| | Ca2+ homeostasis | ORAI1 |
| | | STIM1 |
| | Channels | SCN4A |
| | | CLCN1 |
| Muscle membrane repair | | BIN1 |
| | | TRIM72 |
| | | EHD2 |
| | | EHD1 |
| | | DYSF |
| | | Annexins A1, A2, A5, A6 |
| | Chaperone surveillance | SIL1 |
| | | DNAJB6 |
| | | BAG3 |
| | | HSPB5 |
| | Ubiquitin/Proteasome system | TRIM32 |
| Protein turnover | | MuRF1 |
| | | KLHL40/KLHL41 |
| | | KBTBD13 |
| | Autophagy-lysosomal pathway | LAMP1,2 |
| | | LC3 |
| | | VMA21 |
| | | EPG5 |
| | | SQSTM 1 |

In some embodiments, proteins associated with specific functional or structural properties of muscle cells are those listed in Table 2.

In some particular embodiments, the imaging marker comprises, or consists of, one or several proteins associated with specific functional or structural properties of muscle cells listed in Table 2.

Labelling agents used to stain myotubes for these markers may be easily chosen by the skilled person. Typically, labelling agents used to stain myotubes for such proteins are or comprise antibodies directed against the wild-type form of the protein or a mutated form of the protein, preferably antibodies directed against the wild-type form of the protein.

As used herein, the term "cell morphological features affected in neuromuscular disorders" relates to morphological features of muscle cells that can be altered in myotubes derived from a patient affected with a neuromuscular disorder by comparison to healthy myotubes.

Examples of such features include, but are not limited to, those listed in Table 3 below.

**Table 3: list of cell morphological features affected in neuromuscular disorders**

| **Morphological features altered in neuromuscular diseases** | |
|---|---|
| | Myotube thickness |
| | Number of nuclear foci |
| | Number and size of vacuoles |
| | Presence of inclusion bodies |
| | Presence of ubiquitylated proteins and aggregates |
| | Number and size of lysosomes |
| | Number and location of nuclei |
| | Number, shape and location of mitochondria |
| | Lipid accumulation |

In some embodiments, cell morphological features affected in neuromuscular disorders are those listed in Table 3.

In some particular embodiments, the imaging marker comprises, or consists of, one or several cell morphological features affected in neuromuscular disorders listed in Table 3.

Labelling agents used to stain myotubes for these markers may be easily chosen by the skilled person. Examples of such labelling agents are disclosed in Table 4 below.

**Table 4: Examples of labelling agents that can be used to reveal cell morphological features of Table 3**

| **Markers** | **Examples of labelling agents** |
|---|---|
| Myotube thickness | Antibodies directed against Troponin T or myosin heavy chain |
| Number of nuclear foci | oligonucleotide probes hybridizing to CAG repeats |
| Number and size of vacuoles | Antibodies directed against p62, LC3 or LAMP2 |
| Presence of inclusion bodies | Antibodies directed against DNAJB6, HSPA8, MLF1, desmin, myotilin, filamin or KRT18 |
| Presence of ubiquitylated proteins and aggregates | Antibodies directed against ubiquitin, PROTEOSTAT^{®} detection reagent |
| Number and size of lysosomes | Antibodies directed against LAMP1, LAMP2 or EPG5 |
| Number and location of nuclei | DAPI, Hoechst 33342 |
| Number, shape and location of mitochondria | MitoTracker (Hoechst 33342), JC-1 dye, antibodies directed against F1F0 ATPase or TOMM20 |
| Lipid accumulation | HCS LipidTOX^{™} |

The myotubes are also stained with at least one labelling agent revealing at least one region of interest (ROI) which is selected from the group consisting of individual myotubes, structures of myotubes, and any combination thereof. Examples of myotube structures include, but are not limited to, nucleus, vacuole, mitochondrion, lysosome, cell membrane and cytoskeleton.

Labelling agents capable of revealing said ROI are well-known by the skilled person and are commercially available. Examples of labelling agents that can be used to reveal individual myotubes include, but are not limited to, antibodies directed against troponin-T or myosin heavy chain. Examples of labelling agents that can be used to reveal nuclei include, but are not limited to, Hoechst and DAPI dyes. Examples of labelling agents that can be used to reveal mitochondria include, but are not limited to, Mitotracker^{™} dyes.

The choice of ROI depends on the neuromuscular disorder of interest and may be easily chosen by the skilled person based on his general knowledge. For disorders driven by a mutation in a gene related to the function of an organelle or inducing a change in the structure of said organelle, the skilled person may chose said organelle as ROI. As illustration, when the disorder is DM1, the skilled person may easily choose to stain myotubes for two ROI, individuals myotubes and nuclei because DM1 is known to induce RNA foci.

In preferred embodiments, said at least one ROI comprises individual myotubes.

In some particular embodiments, said at least one ROI comprises individual myotubes and nuclei, in particular their nuclei.

After myotube staining, images of these stained myotubes are captured.

This step may be performed using any device suitable to capture microscopic images. The microscopic images may for example be taken by bright-field imaging, dark-field imaging, cross-polarized light imaging, phase-contrast imaging, fluorescence imaging, confocal imaging and/or super-resolution imaging. The choice of the imaging technique depends on the nature of the signals emitting by the labelling agents. Preferably, the labelling agents emit fluorescence signals and the microscopic images are taken by fluorescence imaging and by acquiring each channels corresponding a labelling agent used to reveal ROI and to a labelling agent used to reveal imaging markers. Optionally, an illumination function may be applied on the acquired images to correct uneven illumination.

An image may comprise only healthy myotubes, only diseased myotubes or a mix thereof. However, for the training set of images, the healthy or diseased status of each myotubes is known. Preferably, each captured image comprises only healthy myotubes or only diseased myotubes.

The training set of images comprises healthy myotubes, i.e. derived from at least one healthy subject, and diseased myotubes, i.e. myotubes derived from at least one patient suffering from a neuromuscular disorder of interest.

Preferably, the training set of images comprises healthy myotubes derived from at least two healthy subjects, more preferably from at least 3 or 4 healthy subjects. Preferably, these subjects are of different genders, of different ages and/or of different genetic backgrounds, e.g. Asian, Eurasian, African and/or Caucasian genetic backgrounds.

Preferably, the training set of images comprises diseased myotubes derived from at least two patients suffering from a neuromuscular disorder of interest, more preferably from at least 3 or 4 patients. Preferably, these subjects are of different genders, of different ages and/or of different genetic backgrounds, e.g. Asian, Eurasian, African and/or Caucasian genetic backgrounds. If the neuromuscular disorder of interest may be induced by different genetic alterations, the patients are preferably chosen in order to illustrate this variety and thus to comprise different genetic alterations inducing said disorder.

Typically, the training set of images comprises approximately the same number of healthy myotubes and diseased myotubes. Each image may comprise the same number of myotubes or a different number of myotubes. The total number of myotubes of each status has to be sufficient to allow training of the classifier and can be easily determined by the skilled person. Typically, the training set comprises the images of 300 to 3000 healthy myotubes and the images of 300 to 3000 diseased myotubes.

In step a) of the method, the classifier may be provided directly with the training set of images or may be provided with preprocessed information obtained from said training set of images.

In an embodiment, preprocessed information may be obtained by performing an image segmentation with an algorithm on appropriate staining channel(s) in order to identify ROI as defined above. Each image is thus segmented into a plurality of ROI.

Appropriate staining channel(s) is(are) the channel(s) corresponding the labelling agent(s) used to reveal ROI. For example, segmentation of individual myotubes and nuclei may be done using the channel of the labelling agent revealing Troponin T or Myosin heavy chain and the channel of Hoechst dye, respectively.

Preferably, the threshold of segmentation is set-up in order to avoid detecting the background noise and eliminate aberrant small myotube structures.

According to the type of classifier used, segmentations may be directly provided to the classifier or may be used to extract phenotypic features. In particular, in embodiments wherein the classifier is a neural network, segmentations may be directly provided to the classifier. Alternatively, in embodiments wherein the classifier is a supervised machine learning, segmentations may be used to extract phenotypic features and said features may then be provided to the classifier.

In another embodiment, preprocessed information may be obtained by
- performing an image segmentation with an algorithm on appropriate staining channel(s) in order to identify ROI as detailed above, and
- extracting from each input ROI (i.e. ROI provided to the feature extractor software), phenotypic features associated with the imaging marker.

Extraction of the phenotypic features is performed with an algorithm/software on appropriate staining channel(s), i.e. using the channel(s) of the labelling agent(s) revealing the imaging marker.

Examples of phenotypic features include, but are not limited to, intensity features, granularity features, intensity distribution features, texture features, size and shape features, colocalization features, run-length gray level matrix-based features and wavelet transform based features. Indeed, depending on the feature extractor software/algorithm, other suitable features may be used to create a feature dataset capable to distinguish healthy from diseased myotubes. The skilled person may easily adapt this feature dataset in order to obtain a trained classifier with a good accuracy.

In an embodiment, extracted phenotypic features are selected from the group consisting of intensity features, granularity features, intensity distribution features, texture features, size and shape features, colocalization features, run-length gray level matrix-based features, wavelet transform based features and combinations thereof, preferably selected from the group consisting of intensity features, granularity features, intensity distribution features, texture features, and any combinations thereof.

Intensity features are the first-order statistics, calculated from the image histograms. They do not consider relationships in pixel neighborhood. Examples of intensity features include, but are not limited to, intensity features listed in Table 5 below.

**Table 5: Examples of intensity features**

| **Feature** | **Definition** |
|---|---|
| Integrated Intensity | The sum of the pixel intensities within an object |
| Mean Intensity | The average pixel intensity within an object |
| Std Intensity | The standard deviation of the pixel intensities within an object |
| Max Intensity | The maximal pixel intensity within an object |
| Min Intensity | The minimal pixel intensity within an object |
| Integrated Intensity Edge | The sum of the edge pixel intensities of an object |
| Mean Intensity Edge | The average edge pixel intensity of an object |
| Std Intensity Edge | The standard deviation of the edge pixel intensities of an object |
| Max Intensity Edge | The maximal edge pixel intensity of an object |
| Min Intensity Edge | The minimal edge pixel intensity of an object |
| Mass Displacement | The distance between the centers of gravity in the gray-level representation of the object and the binary representation of the object |
| Lower Quartile Intensity | The intensity value of the pixel for which 25% of the pixels in the object have lower values |
| Median Intensity | The median intensity value within the object |
| MAD Intensity | The median absolute deviation (MAD) value of the intensities within the object. The MAD is defined as the median(\|xi - median(x)\|) |
| Upper Quartile Intensity | The intensity value of the pixel for which 75% of the pixels in the object have lower values |

In an embodiment, intensity features are selected from the group consisting of intensity features listed in Table 5, and any combinations thereof.

In a preferred embodiment, intensity features include all intensity features listed in Table 6.

Granularity features relates to image granularity which is a texture measurement that measures the quantity of grains at different sizes. This set of features was produced by a series of openings of the original image with structuring elements of increasing size. At each step, the volume of the open image was calculated as the sum of all pixels in the ROI. The difference in volume between the successive steps of opening was the granular spectrum. The distribution was normalized to the total volume (integrated intensity) of the ROI. The module returns one measurement for each instance of the granularity spectrum set in Range of the granular spectrum.

Texture features measure the degree and nature of textures within ROI to quantify their roughness and smoothness. This set of features measured information regarding the spatial distribution of the various channel intensity levels. A region of interest without much texture has a smooth appearance; a region of interest with a lot of texture will appear rough and show a wide variety of pixel intensities. Texture features may be defined as Haralick texture features (Haralick et al., 1973, IEEE Transactions on Systems, Man and Cybernetics, SMC-3(6), 610-621). This texture definition uses a covariance matrix between each pixel and its neighbors. The matrix contains information about the correlation of intensity between one pixel and the one placed n-pixel further.

Examples of texture features include, but are not limited to, texture features listed in Table 6 below.

**Table 6: Examples of texture features**

| **Feature** | **Definition** |
|---|---|
| Angular Second Moment | Measure of image homogeneity. A higher value of this feature indicates that the intensity varies less in an image. Has a value of 1 for a uniform image |
| Contrast | Measure of local variation in an image, with 0 for a uniform image and a high value indicating a high degree of local variation |
| Correlation | Measure of linear dependency of intensity values in an image. For an image with large areas of similar intensities, correlation is much higher than for an image with noisier, uncorrelated intensities. Has a value of 1 or -1 for a perfectly positively or negatively correlated image, respectively. |
| Variance | Measure of the variation of image intensity values. For an image with uniform intensity, the texture variance would be zero |
| Inverse Difference Moment | Another feature to represent image contrast. Has a low value for inhomogeneous images, and a relatively higher value for homogeneous images |
| Sum Average | The average of the normalized grayscale image in the spatial domain |
| Sum Variance | The variance of the normalized grayscale image in the spatial domain |
| Sum Entropy | A measure of randomness within an image |
| Entropy | An indication of the complexity within an image. A complex image produces a high entropy value |
| Difference Variance | The image variation in a normalized co-occurrence matrix, |
| Difference Entropy | Another indication of the amount of randomness in an image |
| InfoMeas1 | A measure of the total amount of information contained within a region of pixels derived from the recurring spatial relationship between specific intensity values |
| InfoMeas2 | An additional measure of the total amount of information contained within a region of pixels derived from the recurring spatial relationship between specific intensity values. It is a complementary value to InfoMeas1 and is on a different scale |

In an embodiment, texture features are selected from the group consisting of texture features listed in Table 6, and any combinations thereof.

In a preferred embodiment, texture features include all texture features listed in Table 6.

Intensity distribution features measure the spatial distribution of intensities within each object. Given an image with identified ROI, this set of features measures the intensity distribution from each object's center to its boundary within a set of rings. The distribution is measured from the center of the object, where the center is defined as the point farthest from any edge.

Examples of intensity distribution features include, but are not limited to, intensity distribution features listed in Table 7 below.

**Table 7: Examples of intensity distribution features**

| **Feature** | **Definition** |
|---|---|
| FracAtD | Fraction of total stain in an object at a given radius |
| MeanFrac | Mean fractional intensity at a given radius; calculated as fraction of total intensity normalized by fraction of pixels at a given radius. |
| RadialCV | Coefficient of variation of intensity within a ring, calculated across 8 slices. |
| Zernike | The Zernike features characterize the distribution of intensity across the object. For instance, Zernike 1,1 has a high value if the intensity is low on one side of the object and high on the other. The Zernike Magnitude feature records the rotationally invariant degree magnitude of the moment and the Zernike Phase feature gives the moment's orientation |

In an embodiment, intensity distribution features are selected from the group consisting of intensity distribution features listed in Table 7, and any combinations thereof.

In a preferred embodiment, intensity distribution features include all intensity distribution features listed in Table 7.

Size and shape features measure several area and shape features of identified objects. Given an image with identified ROI, this set of features measures area and shape-based features of each one.

Examples of size and shape features include, but are not limited to, size and shape features listed in Table 8 below.

**Table 8: Examples of size and shape features**

| **Feature** | **Definition** |
|---|---|
| Area | The number of pixels in the region. |
| Perimeter | The total number of pixels around the boundary of each region in the image. |
| FormFactor | Calculated as 4*π*Area/Perimeter2. |
| Convex Area | The area of a convex polygon containing the whole object. |
| Solidity | The proportion of the pixels in the convex hull that are also in the object. |
| Extent | The proportion of the pixels in the bounding box that are also in the region. |
| EulerNumber | The number of objects in the region minus the number of holes in those objects, assuming 8-connectivity. |
| BoundingBoxArea | The area of a box containing the object. |
| Eccentricity | The eccentricity of the ellipse that has the same second moments as the region. |
| MajorAxisLength | The length of the major axis of the ellipse that has the same normalized second central moments as the region. |
| MinorAxisLength | The length of the minor axis of the ellipse that has the same normalized second central moments as the region. |
| EquivalentDiameter | The diameter of a circle or sphere with the same area as the object. |
| Orientation | The angle (in degrees ranging from -90 to 90 degrees) between the x-axis and the major axis of the ellipse that has the same second-moments as the region. |
| Compactness | The mean squared distance of the object's pixels from the centroid divided by the area. |
| MaximumRadius | The maximum distance of any pixel in the object to the closest pixel outside of the object. |
| MedianRadius | The median distance of any pixel in the object to the closest pixel outside of the object |
| MeanRadius | The mean distance of any pixel in the object to the closest pixel outside of the object. |
| MinFeretDiameter, MaxFeretDiameter | The Feret diameter is the distance between two parallel lines tangent on either side of the object. The minimum and maximum Feret diameters are the smallest and largest possible diameters, rotating the calipers along all possible angles. |
| Spatial Moment features | A series of weighted averages representing the shape, size, rotation and location of the object. |
| Central Moment features | Similar to spatial moments but normalized to the object's centroid. |
| Normalized Moment features | Similar to central moments, but further normalized to be scale invariant. |
| Hu Moment features | Hu's set of image moment features. |
| Inertia Tensor features | A representation of rotational inertia of the object relative to its center. |
| Inertia Tensor Eigenvalues features | Values describing the movement of the Inertia Tensor array. |

In an embodiment, size and shape features are selected from the group consisting of size and shape features listed in Table 8, and any combinations thereof.

In a preferred embodiment, size and shape features include all size and shape features listed in Table 8.

Colocalization features measure the colocalization and correlation between intensities in different images on a pixel-by-pixel basis, within identified ROI.

Examples of colocalization features include, but are not limited to, colocalization features listed in Table 9 below.

**Table 9: Examples of colocalization features**

| | |
|---|---|
| **Feature** | **Definition** |
| Correlation | The correlation between a pair of images, calculated as Pearson's correlation coefficient. |
| Slope | The slope of the least-squares regression between a pair of images. |
| Overlap coefficient | The overlap coefficient is a modification of Pearson's correlation where average intensity values of the pixels are not subtracted from the original intensity values. |
| Manders coefficient | The Manders coefficient for a pair of images. |
| Manders coefficient (Costes Automated Threshold) | Costes' automated threshold estimates maximum threshold of intensity for each image based on correlation. |
| Rank Weighted Colocalization coefficient | The RWC coefficient for a pair of images R and G is measured as RWC1 = sum(Ri_coloc*Wi)/sum(Ri) and RWC2 = sum(Gi_coloc*Wi)/sum(Gi), where Wi is Weight defined as Wi = (Rmax - Di)/Rmax where Rmax is the maximum of Ranks among R and G based on the max intensity, and Di = abs(Rank(Ri) - Rank(Gi)) and Ri_coloc = Ri when Gi > 0, 0 otherwise and Gi_coloc = Gi when Ri >0, 0 otherwise. |

In an embodiment, colocalization features are selected from the group consisting of colocalization features listed in Table 9, and any combinations thereof.

In a preferred embodiment, colocalization features include all colocalization features listed in Table 9.

Run-length gray level matrix-based features are features derived from the run-length gray level matrices (RLGLM) using the run-length metric proposed by Galloway (Galloway, 1975, Computer Graphics and Image Processing, 4(2), 172-179). A gray-level run is a set of consecutive, collinear image points that have the same gray-level value. The length of a gray level run is defined as the number of elements in the run and can be used as a feature for texture analysis. A major reason for the use of gray-level run length features has been that the lengths in a certain direction and orientation give information on the texture elements. In addition, the RLGLM features are able to characterize the 2D orientation, 3D orientation and scale of a texture. For a given direction (usually 0, 45, 90 or 135) a matrix is constructed from the run lengths of gray levels starting at each position in the image.

Examples of features that can be extracted from the RLGLM include, but are not limited to, features listed in Table 10 below.

**Table 10: Examples of run-length gray level matrix-based features features**

| **Feature** | **Definition** |
|---|---|
| Short Run Emphasis | $Average over 13 directions \left(\frac{1}{H}{\sum }_{i} {\sum }_{j} \frac{GLRLM \left(i, j\right)}{{j}^{2}}\right)$ |
| Long Run Emphasis | $Average over 13 directions \left(\frac{1}{H}{\sum }_{i} {{\sum }_{j} GLRLM \left(i, j\right) ∗ j}^{2}\right)$ |
| Gray Level non-uniformity | $Average over 13 directions \left(\frac{1}{H}{\sum }_{i} {\left({\sum }_{j} GLRLM \left(i, j\right)\right)}^{2}\right)$ |
| Run-length non-uniformity | $Average over 13 directions \left(\frac{1}{H}{\sum }_{j} {\left({\sum }_{i} GLRLM \left(i, j\right)\right)}^{2}\right)$ |
| Run Percentage | $Average over 13 directions \left(\frac{H}{{\sum }_{i} {\sum }_{j} j ∗ GLRLM \left(i, j\right)}\right)$ |
| Low Gray Level Run Emphasis | $Average over 13 directions \left(\frac{1}{H}{\sum }_{i} {\sum }_{j} \frac{GLRLM \left(i, j\right)}{{i}^{2}}\right)$ |
| High Gray Level Run Emphasis | $Average over 13 directions \left(\frac{1}{H}{\sum }_{i} {\sum }_{j} GLRLM \left(i, j\right) ∗ {i}^{2}\right)$ |
| Short Run Low Gray Level Emphasis | $Average over 13 directions \left(\frac{1}{H}{\sum }_{i} {\sum }_{j} \frac{GLRLM \left(i, j\right)}{{i}^{2} ∗ {j}^{2}}\right)$ |
| Short Run High Gray Level Emphasis | $Average over 13 directions \left(\frac{1}{H}{\sum }_{i} {\sum }_{j} \frac{GLRLM \left(i, j\right) ∗ {i}^{2}}{{j}^{2}}\right)$ |
| Long Run Low Gray Level Emphasis | $Average over 13 directions \left(\frac{1}{H}{\sum }_{i} {\sum }_{j} \frac{GLRLM \left(i, j\right) ∗ {j}^{2}}{{i}^{2}}\right)$ |
| Long Run High Gray Level Emphasis | $Average over 13 directions \left(\begin{matrix}\frac{1}{H} {\sum }_{i} {\sum }_{j} GLRLM \left(i, j\right) ∗ {i}^{2} \\ ∗ {j}^{2}\end{matrix}\right)$ |

In an embodiment, run-length gray level matrix-based features are selected from the group consisting of run-length gray level matrix-based features listed in Table 10, and any combinations thereof.

In a preferred embodiment, run-length gray level matrix-based features include all run-length gray level matrix-based features listed in Table 10.

Wavelet transform based features are features based on the wavelet transform which is a solid mathematical framework for decomposing an image into different frequency components: three detail images and one low frequency approximation image. By recursively applying the wavelet transformation to the low frequency approximation a multi-resolution decomposition can be achieved. If used for image analysis the wavelet transform is extended to both vertical and horizontal directions.

In a particular embodiment, extracted phenotypic features are selected from the group consisting of intensity features as listed in Table 5, granularity features, intensity distribution features as listed in Table 7 and texture features as listed in Table 6, and any combinations thereof. In a more particular embodiment, extracted phenotypic features are intensity features as listed in Table 5, granularity features, intensity distribution features as listed in Table 7 and texture features as listed in Table 6.

Segmentation of region of interest and feature extraction may be done by a feature extractor such as the open-source software Cell Profiler (Carpenter et al., 2006, Genome Biology, 7(10)) or using appropriate software applications such as Matlab or Fiji or programming languages such as Python, Java, C++. Extracted phenotypic features can be then provided to the classifier.

Steps b) and c) of the method comprise
b) generating an output of the classifier for each input ROI, said output classifying the input ROI as associated to a healthy or diseased myotube; and
c) comparing the generated output for each input ROI to a label associated with said input ROI said label comprising an indication of the healthy or diseased status of the myotube corresponding to said input ROI.

As detailed above, the classifier is provided with a training set of images of stained myotubes, or with preprocessed information obtained from said training set of images. Thus, depending on the nature of the classifier, each input ROI may be identified by the classifier, may belong to preprocessed information provided to the classifier (for it to extract phenotypic features) or may be already preprocessed and decomposed in phenotypic features.

By generating an output classifying each input ROI as associated to a healthy or diseased myotube and comparing each generated output to a label associated with each input ROI indicating the healthy or diseased status of the myotube corresponding to said input ROI, the classifier is getting trained to distinguish between healthy myotubes and diseased myotubes.

Step d) of the training method is the evaluation of the classifier's accuracy for distinguishing between healthy myotubes and diseased myotubes.

The accuracy of the classifier may be assessed using any method known by the skilled person. In particular, the classifier's accuracy may be assessed by calculating the F-score. The F-score is a measure that evaluates the model's accuracy and is defined as: $F - score = \frac{TP}{TP + 0.5 \left(FP+FN\right)}$ where TP = True Positive, FN = False Negative and FP = False Positive values.

A F-score of 1 depicts a perfect classification: the two categories, healthy myotubes and diseased myotubes, are completely distinguishable. A F-score equal to or greater than 0.9 is considered to allow a good separation between healthy myotubes and diseased myotubes.

Preferably, the evaluation of the classifier's accuracy carried out in step d) is based on the classification of myotubes comprised in a test set of images which is distinct from the training set of images, the healthy or diseased status of each myotube being known, and the test set of images being obtained and processed using the same method as that used to obtain and process the training set of images. Optionally, the evaluation of the classifier's accuracy may be carried out several times on different test sets of images.

The classifier is considered as an accurate classifier to distinguish between healthy myotubes and diseased myotubes if it exhibits an accuracy corresponding to a F-score equal to or greater than 0.9. An accuracy corresponding to a F-score equal to or greater than 0.9 may be assessed in step d) using another method than the F-score.

If the classifier exhibits an accuracy below 0.9, the training method, i.e. steps a) to d), may be reiterated with some modifications such as increasing the number of images or of imaged myotubes in the training set of images, using a distinct training set of images, modifying the imaging marker and/or the ROI, modifying and/or increasing the number of extracted phenotypic features, and/or modifying some parameters of the classifier, until achieving an accuracy corresponding to a F-score equal to or greater than 0.9. Preferably, the steps a) to d) of the method are repeated with a different imaging marker as defined above.

Thanks to the possibility of using high-throughput platforms to culture, stain and capture images of myotubes, as disclosed for example in International patent application WO 2015/091593, the method may be easily reiterated with such modifications without inducing an undue burden on the skilled person.

In another aspect, the present invention also relates to a computer-implemented method of identifying an imaging marker of a neuromuscular disorder of interest comprising
a) providing a training set of images of stained myotubes, or preprocessed information obtained from said training set of images, to a classifier, the training set of images comprising images of healthy myotubes and of myotubes exhibiting features of a neuromuscular disorder of interest ("diseased myotube"), said myotubes being stained for a candidate imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one region of interest (ROI) selected from the group consisting of individual myotubes, structures of myotubes, and any combination thereof;
b) generating an output of the classifier for each input ROI, said output classifying the input ROI as associated to a healthy or diseased myotube;
c) comparing the generated output for each input ROI to a label associated with said input ROI said label comprising an indication of the healthy or diseased status of the myotube corresponding to said input ROI;
d) evaluating the classifier's accuracy for distinguishing between healthy myotubes and diseased myotubes,
wherein said candidate imaging marker is identified as an imaging marker of said neuromuscular disorder if the classifier exhibits an accuracy to distinguish between healthy myotubes and diseased myotubes corresponding to a F-score equal to or greater than 0.9.

All embodiments disclosed above for the method of the invention of training a classifier are also contemplated in this aspect.

In particular, the candidate imaging marker may be selected from the group consisting of identified disease driver(s) of the neuromuscular disorder of interest, proteins associated with specific functional or structural properties of muscle cells, cell morphological features affected in neuromuscular disorders, and any combination thereof.

Preferably, the candidate imaging marker may be selected from the group consisting of identified disease driver(s) of the neuromuscular disorder of interest listed in Table 1, proteins associated with specific functional or structural properties of muscle cells listed in Table 2, cell morphological features affected in neuromuscular disorders listed in Table 3, and any combination thereof.

If the classifier exhibits an accuracy below 0.9, the candidate imaging marker is not selected and steps a) to d) of the method may be repeated with a different candidate imaging marker as defined above.

As mentioned above, thanks to the possibility of using high-throughput platforms to culture, stain and capture images of myotubes, as disclosed for example in International patent application WO 2015/091593, the method may be easily reiterated with such modifications without inducing an undue burden on the skilled person.

An imaging marker of a neuromuscular disorder of interest selected with this method can be used for all applications using the trained classifier to distinguish between healthy myotubes and diseased myotubes.

An aspect not part of the claimed invention relates to a computer-implemented method for determining if a myotube is a healthy myotube or a myotube exhibiting features of a neuromuscular disorder of interest ("diseased myotube") from at least one image, wherein the method comprises:
- providing at least one input image of a myotube, or preprocessed information obtained from said at least one input image, to a classifier trained to distinguish between healthy myotubes and diseased myotubes, wherein the myotube has been stained for an imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one region of interest (ROI) selected from the group consisting of individual myotubes, structures of myotubes, and any combination thereof, and
- using the classifier to identify the myotube on the image as a healthy myotube or as a diseased myotube as an output of the classifier.

All embodiments disclosed above for the method of the invention of training a classifier and for the method of identifying an imaging marker are also contemplated in this aspect.

Preferably, all parameters of the method are parameters used during the training of the classifier, preferably using the training method of the invention. In particular, said imaging marker and said at least one labelling agent have been used during the training of the classifier.

Said imaging marker of the neuromuscular disorder of interest may have been identified using the method of the invention of identifying an imaging marker.

Preferably, said at least one input image has been obtained by culturing and staining a myotube as described above.

Preferably, said preprocessed information is obtained as described above.

Preferably, the classifier has been trained in order to accurately distinguish between healthy myotubes and diseased myotubes, i.e. the classifier exhibits an accuracy corresponding to a F-score equal to or greater than 0.9.

The method may comprise providing one input image of several myotubes or several input images of several myotubes and the classifier may thus be used to identify the myotubes on the image(s) as healthy myotubes or as diseased myotubes as an output.

The present invention also relates to an *in vitro* method of assessing potency of a compound to revert the phenotype of a myotube exhibiting features of a neuromuscular disorder of interest ("diseased myotube") into a healthy phenotype comprising
(i) providing at least one image comprising a plurality of diseased myotubes, preferably myotubes derived from at least one patient suffering from a neuromuscular disorder of interest, or preprocessed information obtained from said at least one image, to a classifier trained to distinguish between healthy myotubes and diseased myotubes, wherein the myotubes have been contacted with the compound to be tested and have been stained for an imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one region of interest (ROI) selected from the group consisting of individual myotubes, structures of myotubes, and any combination thereof; and
(ii) using the classifier to identify each myotube corresponding to an input ROI as a healthy myotube or as a diseased myotube as an output of the classifier,
a number of myotubes classified as healthy myotubes which is above a statistically significant threshold being indicative that the compound to be tested is able to revert the phenotype of a myotube exhibiting features of the neuromuscular disorder of interest.

All embodiments disclosed above for the method of the invention of training a classifier, for the method of identifying an imaging marker and for the method for determining if a myotube is a healthy myotube or a myotube exhibiting features of a neuromuscular disorder of interest, are also contemplated in this aspect.

Preferably, all parameters of the method are parameters used during the training of the classifier, preferably using the training method of the invention. In particular, said imaging marker and said at least one labelling agent have been used during the training of the classifier.

Said imaging marker of the neuromuscular disorder of interest may have been identified using the method of the invention of identifying an imaging marker.

Preferably, said at least one image has been obtained by culturing and staining myotubes as described above.

Preferably, said preprocessed information may also be obtained as described above.

Preferably, the classifier has been trained in order to accurately distinguish between healthy myotubes and diseased myotubes, i.e. the classifier exhibits an accuracy corresponding to a F-score equal to or greater than 0.9.

The method may comprise providing one image comprising a plurality of myotubes or several images comprising a plurality of myotubes.

The method further comprises before step (i)
- culturing myoblasts, preferably myoblasts derived from said at least one patient, on a substrate allowing the production of homogeneous population of myotubes and contacting said myoblasts and/or myotubes with the compound to be tested;
- staining these myotubes for said imaging marker and for said at least one labelling agent; and
- capturing said at least one image of these stained myotubes.

The myoblasts/myotubes may be contacted with the compound to be tested before, during or after the culture.

The method may further comprise providing at least one second image comprising a plurality of diseased myotubes, preferably myotubes derived from at least one patient suffering from the neuromuscular disorder of interest, or preprocessed information obtained from said at least one second image, to the trained classifier, wherein the myotubes have been stained for the imaging marker and with said at least one labelling agent; and using the classifier to identify each myotube corresponding to an input ROI as a healthy myotube or as a diseased myotube as an output of the classifier. These myotubes are not contacted with the compound to be tested. Preferably, the myotubes are derived from the same patient(s) than the myotubes contacted with the compound to be tested. The number of myotubes classified as healthy myotubes or as diseased myotubes may be used as a control or in order to determine the statistically significant threshold.

The statistically significant threshold is preferably determined by carrying out a statistical test in order to determine a p-value between the number of myotubes classified as healthy myotubes in the control condition disclosed above (with myotubes not contacted with the compound to be tested) and in the test condition (with myotubes contacted with the compound to be tested) or a p-value between the number of myotubes classified as diseased myotubes in the control condition and in the test condition. A p-value below 0.05 is indicative that the difference is significant.

The compound to be tested may be of any nature, e.g. a nucleic acid, a protein, a small molecule (i.e. an organic or inorganic compound, usually less than 1000 daltons), a lipid, a carbohydrate or a combination thereof. In particular, this compound may be a drug authorized by a regulatory authority such as FDA or EMA.

The method may also further comprise calculating a health score which is defined as the percentage of myotubes out of the total that have been classified as healthy myotubes.

Optionally, the method of assessing potency of a compound of the invention may be performed several times with different concentrations of the compound to be tested, in particular to evaluate the dose-response effect of the compound to be tested.

In another aspect, the present invention also relates to an *in vitro* method of predicting the ability of a compound to treat a neuromuscular disorder of interest comprising
assessing potency of a compound to be tested to revert the phenotype of a myotube exhibiting features of a neuromuscular disorder of interest into a healthy phenotype according to method of the invention,
and optionally calculating a health score which is defined as the percentage of myotubes out of the total that have been classified as healthy myotubes,
wherein a number of myotubes classified as healthy myotubes, or a calculated health score, which is above a statistically significant threshold is indicative that said compound is useful in the treatment of said neuromuscular disorder.

Optionally, the method may be performed several times with different concentrations of the compound to be tested, in particular to evaluate the dose-response effect of the compound to be tested.

All embodiments disclosed above for the method of the invention of training a classifier, for the method of identifying an imaging marker, for the method for determining if a myotube is a healthy myotube or a myotube exhibiting features of a neuromuscular disorder of interest and for the method of assessing potency of a compound to revert the phenotype of a diseased myotube, are also contemplated in this aspect.

The statistically significant threshold is preferably determined by carrying out a statistical test in order to determine a p-value between the number of myotubes classified as healthy myotubes in the control condition disclosed above (with myotubes not contacted with the compound to be tested) and in the test condition (with myotubes contacted with the compound to be tested) or a p-value between the number of myotubes classified as diseased myotubes in the control condition and in the test condition. A p-value below 0.05 is indicative that the difference is significant.

Preferably, all parameters of the method are parameters used during the training of the classifier, preferably using the training method of the invention. In particular, said imaging marker and said at least one labelling agent have been used during the training of the classifier.

Preferably, the classifier has been trained in order to accurately distinguish between healthy myotubes and diseased myotubes, i.e. the classifier exhibits an accuracy corresponding to a F-score equal to or greater than 0.9.

The present invention also relates to an *in vitro* method for monitoring the response to a therapeutic compound of a patient affected with a neuromuscular disorder, wherein the method comprises
(i) providing at least one first image comprising a plurality of myotubes derived from a patient suffering from a neuromuscular disorder of interest before the administration of the therapeutic compound to the patient, or preprocessed information obtained from said at least one first image, and least one second image comprising a plurality of myotubes derived from said patient after the administration of the therapeutic compound, or preprocessed information obtained from said at least one second image, to a classifier trained to distinguish between healthy myotubes and diseased myotubes, wherein the myotubes have been stained for an imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one regions of interest (ROI) selected from the group consisting of individual myotubes, structures of myotubes, and any combination thereof; and
(ii) using the classifier to identify each myotube of said at least one first image corresponding to an input ROI as a healthy myotube or as a diseased myotube as a first output of the classifier, and to identify each myotube of said at least second image corresponding to an input ROI as a healthy myotube or as a diseased myotube as a second output of the classifier, and
   optionally calculating health scores for the first and second outputs of the classifier which are each defined as the percentage of myotubes out of the total that have been classified as healthy myotubes,
   wherein a number of myotubes classified as healthy myotubes, or a calculated health score, in the second output of the classifier which is above a number of myotubes classified as healthy myotubes or above a calculated health score in the first output of the classifier is indicative that the patient is responsive to said therapeutic compound, and wherein a number of myotubes classified as healthy myotubes or a calculated health score in the second output of the classifier which is equal to or below a number of myotubes classified as healthy myotubes or equal to or below a calculated health score in the first output of the classifier is indicative that the patient does not respond to said therapeutic compound.

All embodiments disclosed above for the method of the invention of training a classifier, for the method of identifying an imaging marker, for the method for determining if a myotube is a healthy myotube or a myotube exhibiting features of a neuromuscular disorder of interest, for the method of assessing potency of a compound to revert the phenotype of a diseased myotube and for the method of predicting the ability of a compound to treat a neuromuscular disorder of interest, are also contemplated in this aspect.

Preferably, all parameters of the method are parameters used during the training of the classifier, preferably using the training method of the invention. In particular, said imaging marker and said at least one labelling agent have been used during the training of the classifier.

Said imaging marker of the neuromuscular disorder of interest may have been identified using the method of the invention of identifying an imaging marker.

Preferably, at least one first image and said at least one second image have been obtained by culturing and staining myotubes as described above.

Preferably, said preprocessed information is obtained as described above.

The method may comprise providing a plurality of first images and/or a plurality of second images.

Preferably, the classifier has been trained in order to accurately distinguish between healthy myotubes and diseased myotubes, i.e. the classifier exhibits an accuracy corresponding to a F-score equal to or greater than 0.9.

The method further comprises before step (i)
culturing myoblasts derived from said patient before and after the administration of the therapeutic compound on a substrate allowing the production of homogeneous population of myotubes;
staining these myotubes for said imaging marker and with said at least one labelling agent; and
capturing said at least one image of these stained myotubes.

The method may further comprise determining if the response of the first output and the response of the second output is statistically significant. This can be determined by carrying out a statistical test in order to determine a p-value between the response of the first output and the response of the second output. A p-value below 0.05 is indicative that the difference is significant.

The therapeutic compound may be of any nature, e.g. a nucleic acid, a protein, a small molecule (i.e. an organic or inorganic compound, usually less than 1000 daltons), a lipid, a carbohydrate or a combination thereof. Preferably, the therapeutic compound is a drug authorized by a regulatory authority such as FDA or EMA.

An aspect not part of the claimed invention relates to an *in vitro* method for selecting a patient affected with a neuromuscular disorder for a treatment with a therapeutic compound or for determining whether a patient affected with a neuromuscular disorder is susceptible to benefit from a treatment with a therapeutic compound, wherein the method comprises
(i) providing at least one image comprising a plurality of myotubes derived from a patient suffering from a neuromuscular disorder of interest, or preprocessed information obtained from said at least one image, to a classifier trained to distinguish between healthy myotubes and diseased myotubes, wherein the myotubes have been contacted with a therapeutic compound and have been stained for an imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one region of interest (ROI) selected from the group consisting of individual myotubes, structures of myotubes, and any combination thereof; and
(ii) using the classifier to identify each myotube corresponding to an input ROI as a healthy myotube or as a diseased myotube as an output of the classifier, and

optionally calculating a health score which is defined as the percentage of myotubes out of the total that have been classified as healthy myotubes,
wherein a number of myotubes classified as healthy myotubes, or a calculated health score, which is above a statistically significant threshold is indicative that a treatment with said therapeutic compound is suitable for said patient and wherein a number of myotubes classified as healthy myotubes or a health score which is equal to or below a statistically significant threshold is indicative that a treatment with said therapeutic compound is not suitable for said patient.

All embodiments disclosed above for the method of the invention of training a classifier, for the method of identifying an imaging marker, for the method for determining if a myotube is a healthy myotube or a myotube exhibiting features of a neuromuscular disorder of interest, for the method of assessing potency of a compound to revert the phenotype of a diseased myotube, for the method of predicting the ability of a compound to treat a neuromuscular disorder of interest and for the method for monitoring the response to a therapeutic compound of a patient affected with a neuromuscular disorder are also contemplated in this aspect.

Preferably, all parameters of the method are parameters used during the training of the classifier, preferably using the training method of the invention. In particular, said imaging marker and said at least one labelling agent have been used during the training of the classifier.

Said imaging marker of the neuromuscular disorder of interest may have been identified using the method of the invention of identifying an imaging marker.

Preferably, said at least one image has been obtained by culturing and staining myotubes as described above.

Preferably, said preprocessed information is obtained as described above.

The method may comprise providing one image comprising a plurality of myotubes or several images comprising a plurality of myotubes.

Preferably, the classifier has been trained in order to accurately distinguish between healthy myotubes and diseased myotubes, i.e. the classifier exhibits an accuracy corresponding to a F-score equal to or greater than 0.9.

The method may further comprise before step (i)
culturing myoblasts derived from said patient on a substrate allowing the production of homogeneous population of myotubes and contacting said myoblasts and/or myotubes with said therapeutic compound;
staining these myotubes for said imaging marker and with said at least one labelling agent; and
capturing said at least one image of these stained myotubes.

The method may further comprise providing at least one second image comprising a plurality of myotubes derived from at least one patient suffering from the neuromuscular disorder of interest, or preprocessed information obtained from said at least one second image, to the trained classifier, wherein the myotubes have been stained for the imaging marker and with said at least one labelling agent; and using the classifier to identify each myotube corresponding to an input ROI as a healthy myotube or as a diseased myotube as an output of the classifier. These myotubes are not contacted with the therapeutic compound to be tested. Preferably, the myotubes are derived from the same patient(s) than the myotubes contacted with the therapeutic compound to be tested. The number of myotubes classified as healthy myotubes or as diseased myotubes may be used as a control or in order to determine the statistically significant threshold.

The statistically significant threshold is preferably determined by carrying out a statistical test in order to determine a p-value between the number of myotubes classified as healthy myotubes in the control condition disclosed above (with myotubes not contacted with the therapeutic compound to be tested) and in the test condition (with myotubes contacted with the therapeutic compound to be tested) or a p-value between the number of myotubes classified as diseased myotubes in the control condition and in the test condition. A p-value below 0.05 is indicative that the difference is significant.

An aspect not part of the claimed invention relates an *in vitro* method for diagnosing a neuromuscular disorder of interest in a subject, wherein the method comprises
(i) providing at least one image comprising a plurality of myotubes derived from a subject, or preprocessed information obtained from said at least one image, to a classifier trained to distinguish between healthy myotubes and myotubes exhibiting features of a neuromuscular disorder of interest ("diseased myotubes"), wherein the myotubes have been stained for an imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one region of interest (ROI) selected from the group consisting of individual myotubes, structures of myotubes, and any combination thereof; and
(ii) using the classifier to identify each myotube corresponding to an input ROI as a healthy myotube or as a diseased myotube as an output of the classifier, and
   optionally calculating a health score which is defined as the percentage of myotubes out of the total that have been classified as healthy myotubes,
   wherein a number of myotubes classified as healthy myotubes, or a calculated health score, which is below a statistically significant threshold is indicative that said subject suffers from said neuromuscular disorder of interest, and a number of myotubes classified as healthy myotubes, or a calculated health score, which is equal to or above a statistically significant threshold is indicative that said subject does not suffer from said neuromuscular disorder of interest.

All embodiments disclosed above for the method of the invention of training a classifier, for the method of identifying an imaging marker, for the method for determining if a myotube is a healthy myotube or a myotube exhibiting features of a neuromuscular disorder of interest, for the method of assessing potency of a compound to revert the phenotype of a diseased myotube, for the method of predicting the ability of a compound to treat a neuromuscular disorder of interest, for the method for monitoring the response to a therapeutic compound of a patient affected with a neuromuscular disorder, and for the method for selecting a patient affected with a neuromuscular disorder for a treatment with a therapeutic compound or for determining whether a patient affected with a neuromuscular disorder is susceptible to benefit from a treatment with a therapeutic compound are also contemplated in this aspect.

Preferably, all parameters of the method are parameters used during the training of the classifier, preferably using the training method of the invention. In particular, said imaging marker and said at least one labelling agent have been used during the training of the classifier.

Said imaging marker of the neuromuscular disorder of interest may have been identified using the method of the invention of identifying an imaging marker.

Preferably, said at least one image has been obtained by culturing and staining myotubes as described above.

Preferably, said preprocessed information is obtained as described above.

The method may comprise providing one image comprising a plurality of myotubes or several images comprising a plurality of myotubes.

Preferably, the classifier has been trained in order to accurately distinguish between healthy myotubes and diseased myotubes, i.e. the classifier exhibits an accuracy corresponding to a F-score equal to or greater than 0.9.

The method may further comprise before step (i)
culturing myoblasts derived from the subject on a substrate allowing the production of homogeneous population of myotubes and ;
staining these myotubes for said imaging marker and with said at least one labelling agent; and
capturing said at least one image of these stained myotubes.

The method may further comprise providing at least one second image comprising a plurality of myotubes derived from at least one healthy subject, or preprocessed information obtained from said at least one second image, to the trained classifier, wherein the myotubes have been stained for the imaging marker and with said at least one labelling agent; and using the classifier to identify each myotube corresponding to an input ROI as a healthy myotube or as a diseased myotube as an output of the classifier. The number of myotubes classified as healthy myotubes or as diseased myotubes may be used as a control or in order to determine the statistically significant threshold.

The statistically significant threshold is preferably determined by carrying out a statistical test in order to determine a p-value between the number of myotubes provided from the healthy subject and classified as healthy myotubes and the number of myotubes provided from the subject to be diagnosed and classified as healthy myotubes or a p-value between the number of myotubes provided from the healthy subject and classified as diseased myotubes and the number of myotubes provided from the subject to be diagnosed and classified as diseased myotubes. A p-value below 0.05 is indicative that the difference is significant.

In particular embodiments, and for all methods of the invention, the neuromuscular disorder is Duchenne muscular dystrophy and the myotubes are stained for an imaging marker which is selected from the group consisting of utrophin, alpha-sarcoglycan, delta-sarcoglycan, and the combinations of utrophin with alpha-sarcoglycan, delta-sarcoglycan, alpha-dystroglycan or beta-dystroglycan, preferably utrophin and/or alpha-sarcoglycan, more preferably utrophin and optionally alpha-sarcoglycan, even more preferably utrophin and alpha-sarcoglycan.

In some other particular embodiments, and for all methods of the invention, the neuromuscular disorder is myotonic dystrophy type 1 and the myotubes are stained for an imaging marker which is selected from the group consisting of Amphiphysin 2 protein (also known as BIN-1), alpha-sarcoglycan, beta-dystroglycan, dystrophin, preferably C-terminal dystrophin, a combination of the protein MBNL1 and RNA foci, in particular DMPK mRNA foci, and any combinations thereof. Preferably, the myotubes are stained for an imaging marker which is selected from the group consisting of (i) Amphiphysin 2 protein (also known as BIN-1), (ii) alpha-sarcoglycan, optionally in combination with dystrophin, preferably C-terminal dystrophin, (iii) beta-dystroglycan, optionally in combination with dystrophin, preferably C-terminal dystrophin, (iv) dystrophin, preferably C-terminal dystrophin, optionally in combination with alpha-sarcoglycan and/or beta-dystroglycan, and (v) a combination of the protein MBNL1 and RNA foci, in particular DMPK mRNA foci, and any combination thereof. More preferably, the myotubes are stained for an imaging marker which is a combination of the protein MBNL1 and RNA foci, in particular DMPK mRNA foci.

As used herein the term C-terminal dystrophin refers to amino acids 3361 to 3685 of human dystrophin isoform Dp427m (NCBI Reference Sequence: NP_003997.2; Bhattacharya et al., Gene Technol 2015, 4:3).

In another aspect, the present invention also relates to the use of a protein selected from the group consisting of utrophin, alpha-sarcoglycan, delta-sarcoglycan, and the combinations of utrophin with alpha-sarcoglycan, delta-sarcoglycan, alpha-dystroglycan or beta-dystroglycan, preferably utrophin and/or alpha-sarcoglycan, more preferably utrophin and optionally alpha-sarcoglycan, even more preferably utrophin and alpha-sarcoglycan, as an imaging marker for DMD, in particular as an imaging marker for assessing potency of a compound to revert the phenotype of a myotube exhibiting features of DMD into a healthy phenotype, for predicting the ability of a compound to treat DMD, for monitoring the response to a therapeutic compound of a patient affected with DMD, for selecting a patient affected with DMD for a treatment with a therapeutic compound or for determining whether a patient affected with DMD is susceptible to benefit from a treatment with a therapeutic compound, or for diagnosing DMD, preferably using a classifier trained to distinguish between healthy myotubes and myotubes exhibiting features of DMD, more preferably using a classifier trained to distinguish between healthy myotubes and myotubes exhibiting features of DMD according to the method of the invention.

Preferably, said imaging marker is used in a method of the invention for assessing potency of a compound to revert the phenotype of a myotube exhibiting features of DMD into a healthy phenotype, for predicting the ability of a compound to treat DMD, for monitoring the response to a therapeutic compound of a patient affected with DMD, for selecting a patient affected with DMD for a treatment with a therapeutic compound or for determining whether a patient affected with DMD is susceptible to benefit from a treatment with a therapeutic compound, or for diagnosing DMD.

In another aspect, the present invention also relates to
- the use of Amphiphysin 2 protein (also known as BIN-1), alpha-sarcoglycan, beta-dystroglycan, dystrophin, preferably C-terminal dystrophin, a combination of the protein MBNL1 and RNA foci, in particular DMPK mRNA foci, and any combinations thereof,
- preferably the use of (i) Amphiphysin 2 protein (also known as BIN-1), (ii) alpha-sarcoglycan, optionally in combination with dystrophin, preferably C-terminal dystrophin, (iii) beta-dystroglycan, optionally in combination with dystrophin, preferably C-terminal dystrophin, (iv) dystrophin, preferably C-terminal dystrophin, optionally in combination with alpha-sarcoglycan and/or beta-dystroglycan, and (v) a combination of the protein MBNL1 and RNA foci, in particular DMPK mRNA foci, and any combination thereof,
- more preferably, the use of the combination of the protein MBNL1 and RNA foci, in particular DMPK mRNA foci,
as an imaging marker for DM1, in particular as an imaging marker for assessing potency of a compound to revert the phenotype of a myotube exhibiting features of DM1 into a healthy phenotype, for predicting the ability of a compound to treat DM1, for monitoring the response to a therapeutic compound of a patient affected with DM1, for selecting a patient affected with DM1 for a treatment with a therapeutic compound or for determining whether a patient affected with DM1 is susceptible to benefit from a treatment with a therapeutic compound, or for diagnosing DM1, preferably using a classifier trained to distinguish between healthy myotubes and myotubes exhibiting features of DM1, more preferably using a classifier trained to distinguish between healthy myotubes and myotubes exhibiting features of DM1 according to the method of the invention.

Preferably, said imaging marker is used in a method of the invention for assessing potency of a compound to revert the phenotype of a myotube exhibiting features of DM1 into a healthy phenotype, for predicting the ability of a compound to treat DM1, for monitoring the response to a therapeutic compound of a patient affected with DM1, for selecting a patient affected with DM1 for a treatment with a therapeutic compound or for determining whether a patient affected with DM1 is susceptible to benefit from a treatment with a therapeutic compound, or for diagnosing DM1.

In another aspect, the present invention also relates to a computing system comprising:
- a memory storing at least one instruction of a classifier trained according to the method of the invention, and
- a processor accessing to the memory for reading the aforesaid instructions and executing a method of the invention, in particular a method for assessing potency of a compound to revert the phenotype of a myotube exhibiting features of a neuromuscular disorder of interest into a healthy phenotype, for predicting the ability of a compound to treat a neuromuscular disorder of interest, for monitoring the response to a therapeutic compound of a patient affected with a neuromuscular disorder of interest, for selecting a patient affected with a neuromuscular disorder of interest for a treatment with a therapeutic compound or for determining whether a patient affected with a neuromuscular disorder of interest is susceptible to benefit from a treatment with a therapeutic compound, or for diagnosing a neuromuscular disorder of interest.

Others features and advantages of the invention will become clearer in the following examples which are given for purposes of illustration and not by way of limitation.

### EXAMPLES

### MATERIALS & METHODS

### Cell Source and Culture Maintenance

Primary human skeletal muscle myoblasts from Healthy, DMD and DM1 donors were sourced from different donors (see Table 11). Muscle cells were amplified to create master and working cell banks following suppliers' recommendations.

**Table 11: Donor Characteristics**

| | **Donor Age** | **Donor Sex** | **Status** | **Desmin positive cells** |
|---|---|---|---|---|
| **HV #1** | 2 y 8 mo | Male | Healthy | > 90% |
| **HV #2** | 8 y 9 mo | Male | Healthy | > 90% |
| **HV #3** | 4 y | Female | Healthy | > 80% |
| **HV #4** | 20 y | Female | Healthy | > 90% |
| **DMD #1** | 1 y 4mo | Male | DMD del Ex18-44 | >70% |
| **DMD #2** | 1 y | Male | DMD del Ex46-52 | > 80% |
| **DMD #3** | 3y 1mo | Male | DMD mutation c.5602_5605del, p.Asp1866Glufs*5 | > 80% |
| **DMD #4** | 2 y 1 mo | Male | DMD del Ex46-55 | >70% |
| **DM1 #1** | 27 y | Male | DM1 (infantile form, 1300 blood CTG repeats) | > 90% |
| **DM1 #2** | 38 y | Male | DM1 (Juvenile form, 350 blood CTG repeats) | > 90% |
| **DM1 #3** | 34 y | Male | DM1 (Late-onset form, 300 blood CTG repeats) | > 90% |
| **DM1 #4** | 17y | Male | DM1 congenital form 2000 blood CTG repeats | > 90% |
| **DM1 #5** | 7y | Female | DM1 congenital form 3000 blood CTG repeats | > 90% |

Cells expanded following patient biopsy collection were subsequently enriched for myoblasts using CD56+ cell sorting. Primary vials were sourced, thawed, and the proportion of Desmin+ cells was determined. Cells were expanded and cryopreserved into master banks (MB) at which point they were characterized using immunostaining (Desmin+ cells) and the Myoscreen platform (fusion index). Finally, master bank vials were thawed, expanded, and finally cryopreserved into working cell banks (WB) at which point they were characterized using immunostaining (Desmin+ cells) and the Myoscreen platform (fusion index). Cells were selected based on consistency in their doubling time, proportion of Desmin+ cells and fusion index.

### High-Throughput Myotube Formation

All steps were accomplished automatically using a Freedom EVO150 workstation (Tecan).

For experiments for DMD, myoblasts was Skeletal Muscle Cell Growth Medium provided by ZENBIO, while for DM1 experiments, cells were cultured in DMEM/F10 (Thermo Fisher Scientific) supplemented with 20% Fetal Bovine Serum, 5µg/ml Bovine Insulin (Sigma), 0.4µg/ml Dexamethasone (Sigma) and 10ng/ml FGF2 (Miltenyi Biotec).

For all experiments (unless explicitly indicated otherwise), at Day 0, MyoScreen^{™} plates (CYTOO, France, as described in International patent application WO 2015/091593 and in particular in Figure 2A of this application, Young et al. SLAS Discov. 2018 Sep;23(8):790-806) containing micropatterns coated with 10 µg/ml fibronectin (Invitrogen) were pre-filled with 100µl/well of growth medium and stored in the incubator at 37°C. Human primary myoblasts were detached from the flasks, counted, and seeded into the plates with 15 000 cells per well in 100µl of growth medium. At Day 1, the growth medium was changed for a differentiation medium well (DMEM/F12 (Invitrogen), 2% horse serum (GE Healthcare), 0.5% penicillin-streptomycin (Invitrogen)), in which myoblasts started differentiating and forming myotubes. Myotubes formation process was then continued for 8 or 9 days in differentiation medium without medium replacement.

For experiment performed using standard culture plates, plates were coated with 10 µg/ml fibronectin (Invitrogen) for 2h at room temperature then washed with PBS. Plates were then pre-filled with 100µl/well of growth medium and stored in the incubator at 37°C. Human primary myoblasts were detached from the flasks, counted, and seeded into the plates with 15 000 cells per well in 100µl of growth medium. The day after, growth medium was replaced for a differentiation medium well (DMEM/F12 (Invitrogen), 2% horse serum (GE Healthcare), 0.5% penicillin-streptomycin (Invitrogen), in which myoblasts started differentiating and forming myotubes. Myotubes formation process was continued for 9 days.

### siRNA and vivoPMO treatment

After four days of culture, differentiated healthy and DMD myotubes were transfected with either dystrophin and scramble siRNAs (Thermo Fisher Scientific) at 0.001, 0.01, 0.1 and 1 nM final concentration using Lipofectamine RNAiMAX (Thermo Fisher Scientific) or with vivo-phosphorodiamidate morpholino oligonucleomers ("vivoPMOs"; GeneTools) according to respective manufacturers' instructions.

Vivo-phosphorodiamidate morpholino oligonucleomers ("vivoPMOs") were used to perform exon-skipping of Exon 45 of the DMD transcript. To induce DMD exon 45 skipping, a 26-mer vivoPMO version (Gene Tools) of exon 45 oligonucleotide was used at a final concentration of 0.3, 1, and 3 µM.

### Immunofluorescence staining

At Day 9, myotubes were fixed for 30 min in 3.3% formalin (Sigma-Aldrich). Subsequent immunofluorescence staining was according to (Young et al., 2018, Advancing Life Sciences R&D, 23(8), 790-806). Myotubes were washed three times in Dulbecco's Phosphate-Buffered Saline (DPBS, Invitrogen) and permeabilized in 0.5% Triton X-100 (Sigma-Aldrich) or 1% saponin (Sigma-Aldrich). After blocking in 1% bovine serum albumin for 20 min (BSA, Sigma-Aldrich), cells were incubated with primary antibodies listed in Table 12 prepared in BSA 1% for 2h at room temperature (or overnight for NCL-Dys2 antibody) and then washed three times in DPBS. Secondary antibodies (Thermo Fisher Scientific and Jackson ImmunoResearch) were added for 2 h with Hoechst 33342 (Invitrogen). Cells were washed three times in DPBS before acquisition.

**Table 12: Primary antibodies**

| **Dystrophin associated protein (DAP)** | **Antibody Name** | **Supplier** |
|---|---|---|
| α-dystroglycan | Anti-α-Dystroglycan Antibody, clone IIH6C4 | Merck |
| β-dystroglycan | anti β-dystroglycan antibody- sc-33702 | Santa Cruz |
| | anti β-dystroglycan antibody- NCL-bDG | Novocastra Leica |
| α-sarcoglycan | Recombinant Anti-α-sarcoglycan antibody - ab189254 | Abcam |
| β-sarcoglycan | Anti β-sarcoglycan antibody | Leica |
| δ-sarcoglycan | Anti δ-sarcoglycan antibody | Leica |
| Syntrophin | SNTB2 Monoclonal Antibody (1351) | Thermo |
| Dystrobrevin | Purified Mouse Anti-Dystrobrevin | Becton Dickinson |
| Caveolin-3 | Anti-Caveolin-3 antibody | Abcam |
| Dysferlin | Recombinant Anti-Dysferlin antibody [JAI-1-49-3] | Abcam |
| Utrophin | Anti Utrophin antibody - NCL-DRP2CE | Novocastra Leica |
| Integrin β1 | Integrin, beta-1 subunit | DSHB |
| F-Actin | Alexa Fluor^{™} 488 Phalloidin | Thermo |
| α-Tubulin | Rat anti Tubulin Alpha | Abd Serotec |
| Desmin | Recombinant Anti-Desmin antibody [Y66] | Abcam |
| MHC | Myosin 4 Monoclonal Antibody (MF20) - 14-6503-82 | Thermo Fisher Scientific |
| Troponin T | Polyclonal cardiac troponin T | Abcam |
| Dystrophin (N-terminal) | NCL-DysB | Novocastra Leica |
| Dystrophin (C-terminal) | NCL-Dys2 | Novocastra Leica |
| MBNL1 | MB1a (4A8) | DSHB |
| BIN-1 | sc-13575 | Santa Cruz |

### ASO treatment, nuclear DMPK Foci and immunofluorescence staining

After four days of culture, differentiated healthy and DM1 myotubes were transfected with a (CAG)7 ASO (antisense oligonucleotide) (Mulders et al., 2009, Cell, 106(33), 13915-13920) with doses ranging from 2.5 to 20nM final concentration using Lipofectamine RNAiMAX (Thermo Fisher Scientific) according to respective manufacturers' instructions.

Myotube cultures were maintained until 5 days post treatment and then fixed with 10% formalin or 4% paraformaldehyde ) for 10 min the cells are washed three times in Dulbecco's Phosphate-Buffered Saline (DPBS, Invitrogen) and permeabilized with 0.5% Triton X-100 (Sigma-Aldrich). Fluorescence in situ hybridization was performed using a Cy3-labeled (CAG)5 oligonucleotide probe (PNABio) as described in (Maury et al., 2019, iScience 11, 258-271) with the following modifications. The cells are incubated with pre-hybridization buffer (phosphate buffer 0.5M + 40% formamide) for 20 min at room temperature. The RNA probe (CAG)5-Cy3 (PNA Bio) is added to the hybridization buffer (7% dextran, 0.2% BSA in pre-hybridization buffer) and incubated overnight at 37°C. The next day, cells are washed with wash buffer (0.2% BSA in pre-hybridization buffer) warmed at 37°C followed by three washes in DPBS. Subsequent immunofluorescence staining was according to (Young et al., 2018, supra) except that incubations with primary antibodies were performed sequentially, rabbit anti-troponinT antibody (Abcam) for 2 hours and after 3 washes in DPBS with monoclonal anti-MBNL1 (Santa Cruz) overnight at 4°C in 1% BSA. Secondary antibodies were added for 2 h with Hoechst 33342. Cells were washed three times in DPBS before acquisition.

### High content analysis

### Myotubes morphology and muscle marker high content analysis

After fixation and immunostaining, quantitative microscopy was performed using the Operetta HCS imaging system with a 10×/0.3 NA objective (PerkinElmer). Images were analyzed using scripts developed in Acapella software (PerkinElmer) by the inventors. Eleven fields of view per well were acquired. First, segmentation of myotubes and nuclei were done using respectively the Troponin T or Myosin heavy chain staining and the Hoechst staining. One to two myotubes per micropattern were usually identified. The threshold of segmentation was set-up in order to avoid detecting the background noise and eliminate aberrant small myotube structures. At the end of this first step, specific readouts were calculated in the whole well, like the nuclei count and the fusion index (percentage of nuclei included in Troponin T or MHC staining) and optionally mean myotube area, to control the quality of the myotube viability and differentiation. Usually around 50 to 60 myotubes were detected per well in a control condition. Then, an image clean-up step was performed on the myotubes images to remove myotubes that touch the border of the image. The resulted myotubes were used to extract myotube morphology parameters including the myotube mean area as well as specific marker expression (e.g. dystrophin, utrophin staining intensity, α-sarcoglycan staining intensity ...).

### HCA analysis of DM1 associated markers:

Images of cells were acquired with the Operetta HCS platform (Perkin Elmer) using a x40 objective. 81 fields were acquired per well. Image processing and analysis were performed using dedicated algorithms developed on the Acapella High Content Imaging Software (Perkin Elmer) by the inventors. First, myotubes and nuclei segmentation was performed using respectively the TroponinT or MHC staining intensity and the Hoechst staining. The segmentation threshold was selected to avoid the detection of background noise and eliminate aberrant structures. Second, RNA foci stained in cy3 were detected specifically in the myotube nuclei and quantified using a threshold applied to the Cy3 channel. MBNL1 mean expression intensity was extracted in myotube nuclei.

### Cell profiling

The workflow presented in Figure 3 includes the main steps of a machine learning pipeline. The pipeline starts by constructing an annotated database with immunostained plates. Fluorescence images of the plates were acquired on a 10x Operetta HCS imaging system. The acquired images underwent a pre-processing step in which we apply to images an illumination function to correct uneven illumination. Then the regions of interest (ROI) were segmented. From these ROI were extracted features. The pre-processing, segmentation of region of interest and feature extraction was done in the open-source software Cell Profiler (Carpenter et al., 2006, Genome Biology, 7(10)). Using machine learning or deep learning algorithms, we next generated the profile of diseased and healthy cells. For profile generation, dimensionality reduction, and data visualization, we used the programming language Python.

### Image Acquisition:

The immunostained MyoScreen plates were acquired with a 10x Operetta HCS imaging system. Four channels were acquired: HOECHST 33342 for the nuclei staining, DRAQ5 for the myotube staining and Cy3 and Alexa 488 channels that were detected to identified disease biomarkers.

### 1. Image segmentation:

Region of interest (ROI) identification was performed through segmentation algorithms on the appropriate staining channels. In DMD, the myotubes were segmented using the myotube channel and dilated for further analysis. In DM1, nuclei in myotubes were also considered as ROI. In the rest of the material and method section, "myotube" reference can be understood as ROI.

### 2. Feature extraction:

For each identified ROI, features were extracted. The 4 categories that were used are: intensity, granularity, texture, and intensity distribution features. The extracted features chosen here are only an example of suitable set of features that may be employed to create the feature dataset capable to distinguish healthy from diseased phenotype.

**Intensity features:** Intensity features are the first-order statistics, calculated from the image histograms. They do not consider relationships in pixel neighborhood. Among others, the features are: mean, variance, range, intensity on the edge of the ROI, quantile values. These features are extracted from both Cy3 and Alexa 488 channels.

The list of intensity features is: Integrated Intensity, Mean Intensity, Std Intensity, Max Intensity, Min Intensity, Integrated Intensity Edge, Mean Intensity Edge, Std Intensity Edge, Max Intensity Edge, Min Intensity Edge, Mass Displacement, Lower Quartile Intensity, Median Intensity, MAD Intensity and Upper Quartile Intensity. Each of these features is defined in Table 5.

**Granularity features:** Image granularity is a texture measurement that measures the quantity of grains at different sizes. This set of features was produced by a series of openings of the original image with structuring elements of increasing size. At each step, the volume of the open image was calculated as the sum of all pixels in the ROI. The difference in volume between the successive steps of opening was the granular spectrum. The distribution was normalized to the total volume (integrated intensity) of the ROI.

The module returns one measurement for each instance of the granularity spectrum set in Range of the granular spectrum.

**Texture features:** Texture features measure the degree and nature of textures within ROI to quantify their roughness and smoothness. This set of features measured information regarding the spatial distribution of the various channel intensity levels. A region of interest without much texture has a smooth appearance; a region of interest with a lot of texture will appear rough and show a wide variety of pixel intensities.

To calculate this set of features we used Haralick texture features (Haralick et al., 1973, *supra)* that are derived from the co-occurrence matrix. The matrix contains information about the correlation of intensity between one pixel and the one placed n-pixel further.

The list of texture features is: Angular Second Moment, Contrast, Correlation, Variance, Inverse Difference Moment, Sum Average, Sum Variance, Sum Entropy, Entropy, Difference Variance, Difference Entropy, InfoMeas1 and InfoMeas2. Each of these features is defined in Table 6.

**Intensity distribution features:** This feature set measured the spatial distribution of intensities within each object.

Given an image with identified ROI, this set of features measured the intensity distribution from each object's center to its boundary within a set of rings. The distribution was measured from the center of the object, where the center is defined as the point farthest from any edge.

The list of intensity distribution features is: FracAtD, MeanFrac, RadialCV and Zernike. Each of these features is defined in Table 7.

### Profile generation:

The generated datasets were standardized and balanced in order to have approximatively the same number of ROI in healthy and diseased cells categories. The datasets used for the training of the model were composed solely of the control conditions and contained the set of detected ROI with its extracted features.

The evaluation metric used to predict model's efficacy to predict the category of a ROI is the F-score. The F-score is a measure that evaluates the model's accuracy and is defined as: $F - score = \frac{TP}{TP + 0.5 \left(FP+FN\right)}$ where TP = True Positive, FN = False Negative and FP = False Positive values.

A F-score of 1 depicts a perfect classification: the two categories, Healthy and Disease, are completely distinguishable. A F-score equal to or greater than 0.9 is considered to allow a good separation between the Healthy and Disease phenotypes.

The evaluation of the model is performed 10 times over the dataset which is randomly split each time in 90% used for the training of the model and 10% that is used to test the model's accuracy. The performance of the model is shown as the distribution of these 10 values (see Figure 3F).

### Machine learning algorithms:

We chose to compare the following supervised machine learning methods: Random Forest (RF) and Support Vector Machine (SVM) algorithms to distinguish ROI coming from healthy and diseased categories. The Random Forest algorithm (Liaw & Wiener, 2002, R News, 2(3), 18-22) builds multiple decision trees on different random subsets of features and takes their majority vote for classifying in the two different categories. As for the SVM (Cortes et al., 1995, Machine Learning 1995 20:3, 20(3), 273-297), in the n-dimensional space of the features (n being the number of features extracted for each segmented ROI), the algorithm aims at obtaining a hyperplane that will optimally separate the items from the two healthy and diseased categories. The algorithm that showed the highest efficiency was the SVM and was kept for further comparison with Deep Learning methods (Lecun et al., 2015, Nature, 521(7553), 436-444; Schmidhuber, 2015, Neural Networks, 61, 85-117). Among deep learning methods, the convolutional neural network (CNN) (Kraus et al., 2017, Molecular Systems Biology, 13(4), 924) is a class of methods, most commonly applied to analyze images, that avoids the feature extraction step of traditional machine learning algorithms. Convolutional neural networks are composed of multiple layers, one of which is a convolution layer that performs a convolution. Each of the convolutional layers acts as a filter to filter the input data. At a high level, CNN takes the image of our region of interest, and passes it through a series of convolutional, nonlinear, pooling (down-sampling), and fully connected layers to get an output. The output can be a single class or a probability of classes that best describes the image. From the ROI, the first layer extracts basic features such as horizontal or diagonal edges. This output is passed on to the next layer which detects more complex features by combining the basic patterns of first layer. As we move deeper into the network, the features become more and more complex with each layer. The last layer will perform classification of the ROI based on the features extracted from the penultimate layer. The links between layers are optimized to get features that will improve the last layer classification.

The convolution includes generation of an inner product based on the filter and the input data. After each convolution layer, follows a nonlinear layer (or activation layer) such as ReLU (Rectified Linear Units) layer. The purpose of the nonlinear layer is to bring complexity in the features production. Without non-linear layers, a series of convolution layer is equivalent to one convolution layer. After some convolution and ReLU layers series, CNNs may have one or more pooling layers. The pooling layers are also referred to as down-sampling layers. In this category, there are also several layer options, one of them is maxpooling. The maxpooling layer applies a filter spatially every n steps. This filter retains only the maximum value of each area it applies to. As the spatial steps (or stride) is bigger than one, the output volume is lower than the input volume.

The network aims at classifying each ROI into one class out of N. At the end of the network, are stacked one or more fully connected layer and a softmax layer. The fully connected layers act as multiple classifiers. Each classifier receives the features and outputs a weight that will be transform by the softmax layer into a probability: the probability of the ROI to come from one of the N classes. The fully connected layers process the output of the previous layer (which represents the activation maps of high-level features) and determine which features most correlate to a particular class. Instead of the final fully connected (dense) and softmax layer, we also tried using a SVM classifier.

More specifically, the architecture the CNN network shown in Figure 3D includes 3 convolution and ReLU layers, 3 max pooling layers, 2 fully connected and ReLU layers and the softmax layer. In this example, the CNN network includes 15 layers and 5.3 million parameters.

Figure 3E shows the two different processing pipelines between the chosen supervised machine learning methods (RF, SVM) and the chosen deep learning method. Figure 3F shows the DMD vs healthy classification performance of the SVM, classic CNN and CNN with SVM classifier as last layer. The methods are equivalent in terms of performance; we will thus produce all the examples with the SVM model that is the most efficient and has the lowest computational load.

### Health-score definition:

The health-score is defined as the percentage of myotubes out of the total that are predicted to have a healthy phenotype by the model. For each input ROI, the model's output is a probability between 0 and 1 of belonging to one class or the other. If the probability for one ROI is below 0.5, we consider that the phenotype of the myotube comprising said ROI is a diseased phenotype while if it is higher or equal than 0.5, we consider that the phenotype of the myotube comprising said ROI is a healthy phenotype.

### Data visualization:

The representation of a ROI features dataset was done using a dimensionality reduction method (Mcinnes, L., Healy, J., & Melville, J. (2020). UMAP: Uniform Manifold Approximation and Projection for Dimension Reduction *;* Mclachlan, G. J. (2004). Discriminant Analysis and Statistical Pattern Recognition (W. Interscience (ed.)); van der Maaten & Hinton, 2008, Journal of Machine Learning Research, 9(86), 2579-2605). The method used to represent the data in Figure 4 is the SVM-classifier ad-hoc reduction. The reduction associated with an SVM classifier is a projection of the data on the axis normal to the classification hyperplane. After this projection, the data are in a one-dimensional space and it can be plotted as in graphs shown in Figure 4 as a function of density (Cortes et al., 1995, Machine Learning 1995 20:3, 20(3), 273-297) while in Figures 6, 7 and 8 we used the t-distributed stochastic neighbor embedding (t-SNE) method (van der Maaten & Hinton, 2008, Journal of Machine Learning Research, 9(86), 2579-2605) for visualization of high-dimensional features in a 2D space and the SVM-classifier ad-hoc reduction for the visualization of the data separability.

### RESULTS

### Selection and validation of cells from healthy and patients for cell profiling analysis

The procedures used to generate myotubes from patient-derived myoblasts are detailed in the Material and Method section. The examples shown in Figures 1A and 1B characterize and validate myotubes from healthy donors and donors with Duchenne Muscular Dystrophy (DMD). Those examples demonstrate the typical analyses performed on myotubes generated *in vitro.* The degree of myotube formation is monitored by staining cells for nuclei and troponin (labels myotubes) and by quantifying nuclear counts, fusion index, and main area (Figure 1 B). Since DMD patients do not express dystrophin, dystrophin expression is used to validate a disease-relevant phenotype in these cultured cells.

### Characterization of potential muscle markers for cell profiling

In DMD patients, mutations in the DMD gene prevent the expression of a functional dystrophin and the assembly of the Dystrophin-associated protein complex (DAPC). This complex is involved in mediating interactions between the basal lamina, the plasmalemma and F-actin and acts as a shock absorber during contractions. The examples shown in Figure 2A monitor the expression of proteins involved in DAPC and DAPC- basal lamina interactions (syntrophin, dystrobrevin, sarcoglycans dystroglycans, alpha-Tubulin, utrophin, integrin beta 1) in myotubes from healthy and DMD donors. To also probe other cellular structures critical for muscle function, additional proteins were evaluated including actin (sarcomere), desmin (sarcolemma-nuclear envelope connection), caveolin 3 (T-tubule biogenesis), and dysferlin (muscle membrane repair). As shown in figure 2A, monitored individually none of these proteins distinguished healthy and DMD donors.

DM1 is caused by the expansion of a (CUG) repeat in the gene DMPK. Upon expression, those expanded CUG repeats form a stemloop that is recognized by an RNA splicing factor MBNL1. The MBLN1 bound mutated DMPK transcripts form RNA foci in the nucleus of DM1 cells, resulting in a depletion of MBLN1 and consequent miss-splicing of other MBLN1 target RNAs. The examples shown in Figure 2B demonstrate that compared to a healthy donor, myotubes from DM1 donors show a significant increase in the number of nuclear RNA foci and decreased in MBLN1 expression.

### Image processing and data analysis procedure for cell profiling assays monitoring labeled muscle markers

Figure 3A outlines the general procedure used in these examples to obtain and process images of labeled disease-dependent muscle markers. The procedure is described in detail in the Material & Method section. The adaptation of this procedure for the analysis of muscle markers for DMD and DM1 are detailed in Fig. 3B and Fig. 3C, respectively. Figures 3D shows the chosen CNN architecture. Figures 3E outlines the two different processing pipelines between the chosen supervised machine learning methods (RF, SVM) and the chosen deep learning method. The classification performance of supervised machine learning and deep learning methods are shown in Figure 3F. The SVM algorithm shows equivalent accuracy to the chosen CNN models.

### Selection of imaging muscle markers for cell profiling

While the expression of Utrophin, alpha-Sarcoglygan and delta-Sarcoglycan did not significantly differ in myotubes from healthy and DMD donors (Fig. 2A), cell profiling analysis performed on myotubes labeled for these proteins distinguished between healthy and DMD donors (F-score > 0.9; Fig. 4A). In contrast, performing cell profiling analysis on myotubes labeled for either Dysferlin, Syntropin, alpha-Dystroglycan, beta-Dystroglycan, or Dystrobrevin failed to discriminate between healthy and DMD donors (Fig. 4A). However, using these proteins in combination generally improved the ability of cell profiling to distinguish healthy and DMD donors (Fig. 4B). Systematic comparison of pairwise combinations of these proteins identified that monitoring Utrophin in combination with alpha-Sarcoglycan, delta-Sarcoglycan, alpha-Dystroglycan, beta-Dystroglycan, performs optimally in these assays (Fig. 4B). We also evaluated how the cell culture conditions affect the performance of cell profiling. As shown in Fig. 4C, myotubes generated under MyoScreen conditions allowed better discrimination between healthy and DMD donors than myotubes generated on regular culture plates.

### Calibrating the phenotypic response of healthy myotubes to dystrophin downregulation

The disease phenotype of DMD patients is determined by the level and activity of dystrophin. In mdx mice (Patridge, FEBS J. 2013 Sep;280(17):4177-86), a commonly used DMD animal model, recovery of dystrophin expression using an exon-skipping morpholino restored muscle function (Wu, PNAS 2008 Sep;105(39):14814-14819).

To determine the level of dystrophin needed for cultured myotubes to display a "healthy" phenotype, dystrophin mRNA levels in myotubes from healthy donors were titrated using RNAi. As shown in Fig. 5A and B, treatment of myotubes with a DMD-specific siRNA resulted in the downregulation of dystrophin by >80%. The controls used in this experiment demonstrated that neither the transfection agent (mock) nor a non-specific control siRNA had a significant effect on dystrophin levels. The dystrophin levels detected in myotubes from DMD donors were <10%. After staining these myotubes for utrophin and alpha-sarcoglycan, cell profiling analysis was first performed on untreated and mock-treated myotubes from healthy and DMD donors. As shown in Fig. 5C and D, cell profiling phenotypically distinguished healthy and DMD myotubes with high confidence. The descriptor established in these analyses were then used to quantify the response of healthy myotubes to dystrophin downregulation. For this purpose, we defined a "health-score" (% cells with a "healthy" phenotype out of total). Myotubes expressing dystrophin to >60% of the healthy controls revealed a health-score of 100%, whereas myotubes that displayed a reduction of dystrophin levels by 40-70% or >80% displayed health-scores of >40% and <20%, respectively (Fig. 5 E, F). These results demonstrate that in cultured myotubes downregulation of dystrophin expression by >40% induces a DMD-like phenotype.

### Phenotypic response of DMD myotubes to a DMD exon skipping therapy

Exon-skipping is a clinically approved strategy to restore dystrophin expression in DMD patients (Aartsma-Rus et al., 2017, Nucleic Acid Therapeutics 27(5)). For the DMD donors shown in this example, dystrophin expression can be restored by skipping exon 45 using an oligonucleotide that masks the exon 45 splice acceptor site in the DMD pre-mRNA. To improve endosomal uptake, the experiments shown in Fig. 6 were conducted using a 26-mer vivoPMO version (Gene Tools) to induce DMD exon 45 skipping.

Treatment of DMD myotubes with the vivoPMO restored dystrophin expression in a dose- and donor-dependent manner to up to 30-40% of dystrophin levels observed in untreated healthy donors (Fig. 6A, B). Cell profiling analysis of myotubes stained for Utrophin and alpha-Sarcoglycan clearly distinguished myotubes from healthy and either untreated or treated DMD donors (Fig. 6 C, D). For both tested DMD donors the percentage of phenotypically healthy myotubes increased upon treatment with the vivoPMO in a dose-dependent manner (Fig. 6E, F). The observed correlation between dystrophin levels and health-score upon exon 45 skipping in myotubes from DMD patients is consistent with the correlation observed for RNAi-mediated down regulation of dystrophin in healthy myotubes (Fig. 6F, Fig. 5F). This result indicates that in these DMD donors, exon 45 skipping restores a truncated dystrophin that is fully functional.

### Cell profiling can distinguish the response of individual patient donors to therapeutic agents, enabling patient stratification

In DM1 patients, oligonucleotides (ASOs) that selectively hybridize with CUG repeats can induce cleavage of mutant DMPK mRNAs and release MBLN1 from the nuclear RNA foci. In the example shown in Fig. 7 we compare the response of three DM1 patients to treatment with an ASO targeting CUG repeats in DMPK. The characterization of these patients is shown in Table 11.

As expected, ASO treatment of DM1 myotubes reduced nuclear RNA foci and increased MBLN1 levels (Fig. 7 A,B). We next performed cell profiling analysis of these images and determined the F-score from cross validation analysis using SVM linear kernel by taking the 2 different categories at a time using DMPK foci and MBNL1 based features (Fig. 7 C,D). The high F-scores obtained demonstrate the ability of cell profiling to distinguish DM1 and healthy conditions. Using an SVM classifier trained on the untreated conditions between the DM1 and the healthy donors, we then monitored the effect of the ASO treatments on the 3 DM1 donors. As shown in Fig. 7E, treating DM1 myotubes with the ASO we can generate a phenotype that is similar to the healthy phenotype. Importantly, quantification of the effect of ASO treatment on generating DM1 myotubes that are phenotypically healthy (health-score), revealed significant differences in the responsiveness of the three tested DM1 donors to the ASO treatment. This example demonstrates the potential use of cell profiling as a method to stratify patients with respect to their predicted response to a treatment.

### Selection and validation of cells from healthy and DM1 donors for cell profiling analysis

To characterize the growth behavior and differentiation of healthy and DM1 donors under MyoScreen conditions, an evaluation of the myotube morphology in untreated condition was performed. Two healthy and 5 DM1 donors (3 non congenital, and 2 congenital donors) were labelled using Hoechst as a nuclei dye and myosin heavy chain (MHC) as a marker of differentiation to separate myoblasts and myotubes. Figure 8A shows that all donors form myotubes that display the standard MyoScreen morphology. DM1 #2 donor has a lower fusion index than the other HV and DM1 donors which might indicate changes in differentiation compared to the other donors (Figure 8A, B). The presence of DMPK mRNA foci was assessed using a (CAG)5-Cy3 FISH probe and the immunofluorescent staining of MBNL1 was used to determine the presence of MBNL1 protein trapped in the DMPK mRNA foci. DMPK ASO is used to remove the aberrant DMPK mRNA in the DM1 patients. All DM1 donors show the presence of co-labelled DMPK mRNA foci and MBNL1 protein, representative of the DM1 phenotype. For the congenital donors (DM1 #4 and #5) the results are also presented at a different scale (lower graph) for visibility of the dose-response effect. Increasing DMPK ASO dose leads to a decrease of MBNL1/DMPK foci co-labelled spots indicating the disappearance of RNA foci and the liberation of MBNL1 protein (Figure 8C). The same effect is observed in all donors, although statistical significance is reached only for DM1 #3 and #5 due to the important variability in the other donors. (Figure 8C).

We next performed cell profiling analysis of these images and determined the F-score from cross validation analysis using a SVM model by taking the 2 different categories at a time using DMPK foci and MBNL1 based features (Fig. 8D). The high F-scores obtained demonstrate the ability of cell profiling to distinguish DM1 and healthy phenotypes. Using the SVM classifier trained on the untreated conditions between the DM1 and the healthy donors, we then monitored the effect of the DMPK ASO treatments on the DM1 donors. As shown in Fig. 8E, treating DM1 myotubes with the DMPK ASO we can generate a phenotype that is similar to the healthy phenotype. Importantly, quantification of the effect of ASO treatment on generating DM1 myotubes that are phenotypically healthy (health-score), revealed significant differences in the responsiveness of the tested DM1 donors to the ASO treatment. This example demonstrates the potential use of cell profiling as a method to stratify patients with respect to their predicted response to a treatment.

### Phenotypic response of the DM1 donors to ASO treatment by dystrophin-glycoprotein complex monitoring

In DM1 patients, MBNL1 loss of function results in the exclusion of exons 71 and 78 from the DMD mRNA, leading to a different C-terminus for the dystrophin protein. Treatment of DM1 donors with DMPK ASO partially restores dystrophin C-terminal domain. The antibody targeting the C-terminal domain of dystrophin does not show any specific sarcolemmal staining in DM1 donors. Increasing DMPK ASO doses results in the restoration of dystrophin C-terminal signal in the DM1 donors (Figure 9A white arrows show sarcolemma positive for dystrophin signal). The response is donor-dependent with lower restoration in non-congenital donors and higher restoration in congenital donors (Figure 9A, C). Treatment of the DM1 donors with DMPK ASO led to a partial rescue of the C-terminal domain of dystrophin expression levels while the expression levels of α-sarcoglycan and β-dystroglycan are not modified by the DMPK ASO treatment (Figure 9B, C). This result demonstrates that the trap formed by CUG repeat in DMPK pre-mRNA in DM1 donors is responsible for the loss of C-terminal dystrophin expression and that is not acting on α-sarcoglycan and β-dystroglycan expression regulation.

### Cell profiling analysis of DM1 therapy using dystrophin-glycoprotein complex markers

We next performed machine learning based cell profiling analysis of the α-sarcoglycan and C-terminal dystrophin images and determined the F-score from cross validation analysis using a SVM model. Using the obtained F-scores we evaluated the separability of healthy and DM1 donors based on α-sarcoglycan, C-terminal dystrophin and the combination of the 2 markers (Figure 10A). The high F-scores obtained demonstrate the ability of cell profiling to distinguish DM1 congenital and non-congenital and healthy conditions. We can also observe that the use of a combination of the 2 markers in the construction of the model gives a better separability. Using an SVM classifier trained on the untreated conditions between the DM1 and the healthy donors, we then monitored the effect of the ASO treatments on the 3 non congenital DM1 donors and on the 2 congenital donors. As shown in Figure 10B-C, using the α-sarcoglycan and C-terminal dystrophin-based Al model, we can quantify the effect of ASO treatment on generating DM1 myotubes that are phenotypically healthy (health-score). The quantification revealed significant differences in the responsiveness of the tested DM1 donors to the ASO treatment. This example demonstrates the potential use of cell profiling as a method to stratify patients with respect to their predicted response to a treatment.

The results presented in Figure 11 demonstrate that the machine learning based cell profiling analysis can also be established on the β-dystroglycan and C-terminal dystrophin images. We next determined the F-score from cross validation analysis using a SVM model (Figure 11A). Using the obtained F-scores we evaluated the separability of healthy and DM1 donors based on β-dystroglycan, C-terminal dystrophin and the combination of the 2 markers. The high F-scores obtained demonstrate the ability of cell profiling to distinguish DM1 congenital and non-congenital and healthy conditions. We can also observe that the use of a combination of the 2 markers in the training of the model gives a better separability. Using an SVM classifier trained on the untreated conditions between the DM1 and the healthy donors, we then monitored the effect of the ASO treatments on the 3 non congenital DM1 donors and on the 2 congenital donors. As shown in Figures 11B-C, using the β-dystroglycan and C-terminal dystrophin based Al model, we can quantify the effect of ASO treatment on generating DM1 myotubes that are phenotypically healthy (health-score). The quantification revealed significant differences in the responsiveness of the tested DM1 donors to the ASO treatment. This example demonstrates the potential use of cell profiling as a method to stratify patients with respect to their predicted response to a treatment.

### Applicability of the method using a T-tubule related marker

Amphiphysin 2 protein, also known as BIN-1, is a protein involved in T-tubules biogenesis. In DM1 patients, BIN-1 is miss-spliced resulting in exclusion of Exon 11, and the absence of the phosphoinositide binding domain responsible for its membrane tubulating activity (Fugier et al. Nat Med. 2011;17(6):720-725). In healthy donors HV #1 and HV #2 BIN-1 is expressed at the sarcolemma of myotubes (white arrowheads, Figure 12A). In DM1 donors (DM1 #1, DM1 #2, DM1 #5) BIN-1 expression is lower, and it is confined to the cytoplasm (Figure 12A, B). Treatment with an DMPK ASO results in partial restoration of BIN-1 at the sarcolemma in the three DM1 donors, DM1 #5 donor being the more responsive. Total BIN-1 expression is also partially restored in DM1 donors (Figure 12B). We performed Al based cell profiling analysis on these images using BIN-1 as marker and determined the F-score from cross validation analysis using a SVM model. We evaluated the separability of healthy versus DM1 donors but also evaluated if the 2 healthy donors and the 3 DM1 donors can be separated based on the cell profiling model (Figure 12C). The high F-scores obtained demonstrate the ability of cell profiling to distinguish DM1 and healthy conditions. Healthy donors and DM1 donors show low F-scores meaning that donors belonging to the same categories (healthy and DM1) have similar profiles. For the 5 tested DM1 donors the percentage of phenotypically healthy myotubes increased upon treatment with the ASO at the tested concentration (Figure 12D). This example shows that this method can be used to monitor other complexes and proteins.

## Claims

1. An *in vitro* method of assessing potency of a compound to revert the phenotype of a myotube exhibiting features of a neuromuscular disorder of interest ("diseased myotube") into a healthy phenotype comprising
(i) providing at least one image comprising a plurality of *in vitro* cultured diseased myotubes derived from at least one patient suffering from a neuromuscular disorder of interest, or preprocessed information obtained from said at least one image, to a classifier trained to distinguish between healthy myotubes and diseased myotubes, wherein the myotubes have been contacted with the compound to be tested and have been stained for an imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one region of interest (ROI) selected from the group consisting of individual myotubes, structures of myotubes, and any combination thereof; and
(ii) using the classifier to identify each myotube corresponding to an input ROI as a healthy myotube or as a diseased myotube as an output of the classifier,
a number of myotubes classified as healthy myotubes which is above a statistically significant threshold being indicative that the compound to be tested is able to revert the phenotype of a myotube exhibiting features of the neuromuscular disorder of interest, and wherein the method further comprises before step (i)
- culturing myoblasts derived from said at least one patient on a substrate allowing the production of homogeneous population of myotubes and contacting said myoblasts and/or myotubes with the compound to be tested;
- staining these myotubes for said imaging marker and for said at least one labelling agent; and
- capturing said at least one image of these stained myotubes.

2. The method of claim 1, wherein the method further comprises calculating a health score which is defined as the percentage of myotubes out of the total that have been classified as healthy myotubes.

3. An *in vitro* method of predicting the ability of a compound to treat a neuromuscular disorder of interest comprising
assessing potency of a compound to be tested to revert the phenotype of a myotube exhibiting features of a neuromuscular disorder of interest into a healthy phenotype according to claim 1 or 2,
and optionally calculating a health score which is defined as the percentage of myotubes out of the total that have been classified as healthy myotubes,
wherein a number of myotubes classified as healthy myotubes, or a calculated health score, which is above a statistically significant threshold is indicative that said compound is useful in the treatment of said neuromuscular disorder.

4. An *in vitro* method for monitoring the response to a therapeutic compound of a patient affected with a neuromuscular disorder, wherein the method comprises
(i) providing at least one first image comprising a plurality of *in vitro* cultured myotubes derived from a patient suffering from a neuromuscular disorder of interest before the administration of the therapeutic compound to the patient, or preprocessed information obtained from said at least one first image, and at least one second image comprising a plurality of myotubes derived from said patient after the administration of the therapeutic compound, or preprocessed information obtained from said at least one second image, to a classifier trained to distinguish between healthy myotubes and diseased myotubes, wherein the myotubes have been stained for an imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one regions of interest (ROI) selected from the group consisting of individual myotubes, structures of myotubes, and any combination thereof; and
(ii) using the classifier to identify each myotube of said at least one first image corresponding to an input ROI as a healthy myotube or as a diseased myotube as a first output of the classifier, and to identify each myotube of said at least second image corresponding to an input ROI as a healthy myotube or as a diseased myotube as a second output of the classifier, and
optionally calculating health scores for the first and second outputs of the classifier which are each defined as the percentage of myotubes out of the total that have been classified as healthy myotubes,
wherein a number of myotubes classified as healthy myotubes, or a calculated health score, in the second output of the classifier which is above a number of myotubes classified as healthy myotubes or above a calculated health score in the first output of the classifier is indicative that the patient is responsive to said therapeutic compound, and wherein a number of myotubes classified as healthy myotubes or a calculated health score in the second output of the classifier which is equal to or below a number of myotubes classified as healthy myotubes or equal to or below a calculated health score in the first output of the classifier is indicative that the patient does not respond to said therapeutic compound, and
wherein the method further comprises before step (i)
culturing myoblasts derived from said patient before and after the administration of the therapeutic compound on a substrate allowing the production of homogeneous population of myotubes;
staining these myotubes for said imaging marker and with said at least one labelling agent; and
capturing said at least one image of these stained myotubes.

5. The methods of any of claims 1 to 4, wherein said imaging marker and said at least one labelling agent have been used during the training of the classifier.

6. The methods of any of claims 1 to 4, wherein the classifier has been trained using a method of training a classifier for accurately distinguishing between healthy myotubes and myotubes exhibiting features of a neuromuscular disorder of interest ("diseased myotube"), said method comprising
a) providing a training set of images of stained *in vitro* cultured myotubes, or preprocessed information obtained from said training set of images, to a classifier, the training set of images comprising images of healthy myotubes and of myotubes exhibiting features of a neuromuscular disorder of interest ("diseased myotube"), said myotubes being stained for a candidate imaging marker selected from the group consisting of identified disease driver(s) of the neuromuscular disorder of interest, proteins associated with specific functional or structural properties of muscle cells, cell morphological features affected in neuromuscular disorders, and any combination thereof, and with at least one labelling agent revealing at least one region of interest (ROI) selected from the group consisting of individual myotubes, structures of myotubes, and any combination thereof;
b) generating an output of the classifier for each input ROI, said output classifying the input ROI as associated to a healthy or diseased myotube;
c) comparing the generated output for each input ROI to a label associated with said input ROI said label comprising an indication of the healthy or diseased status of the myotube corresponding to said input ROI;
d) evaluating the classifier's accuracy for distinguishing between healthy myotubes and diseased myotubes,
wherein said candidate imaging marker is identified as an imaging marker of said neuromuscular disorder if the classifier exhibits an accuracy to distinguish between healthy myotubes and diseased myotubes corresponding to a F-score equal to or greater than 0.9.

7. The method of any of claims 1 to 6, wherein said preprocessed information are obtained by
- performing an image segmentation with an algorithm on appropriate staining channel(s) in order to identify ROI, and
- extracting from each input ROI phenotypic features associated with said imaging marker, said extracted phenotypic features being selected from the group consisting of intensity features, granularity features, intensity distribution features, texture features, size and shape features, colocalization features, run-length gray level matrix-based features, wavelet transform based features and combinations thereof, preferably selected from the group consisting of intensity features, granularity features, intensity distribution features, texture features, and any combinations thereof.

8. The method of any of claims 1 to 7, wherein the classifier is selected from Support Vector Machine (SVM) classifier, random forest (RF) classifier, decision tree classifier, K-nearest neighbor classifier (KNN), logistic regression classifier, nearest neighbor classifier, Gaussian mixture model (GMM), nearest centroid classifier, linear regression classifier, and neural networks such as artificial, deep, convolutional, fully connected neural networks.

9. The method of any of claims 1 to 8, wherein the neuromuscular disorder of interest is selected from the group consisting of muscular dystrophies, myopathies, congenital myasthenic syndromes, motor neuron diseases and metabolic muscle disorders.

10. The method of any of claims 1 to 9, wherein the neuromuscular disorder of interest is
- a muscular dystrophy selected from the group consisting of Duchenne Muscular Dystrophy (DMD), Becker Muscular Dystrophy (BMD), Myotonic Dystrophy 1 (DM1), Myotonic Dystrophy 2 (DM2), Facioscapulohumeral Muscular Dystrophy (FSHD), Emery-Dreifuss muscular dystrophy, Limb-girdle muscular dystrophies, Walker-Warburg syndrome, Muscle-eye-brain disease, Congenital muscular dystrophy, Scapuloperoneal muscular dystrophy, Tibial muscular dystrophy and Autosomal Recessive Muscular Dystrophy,- a myopathy selected from the group consisting of Bethlem & Ullrich myopathy, Myofibrillar myopathy, Distal myopathy, Rimmed vacuole myopathy, Centronuclear myopathy (CNM), X-linked myotubular myopathy (XLMTM), Tubular aggregate myopathy, Malignant hyperthermia syndrome, Inclusion body myopathy, Myofibrillar myopathy, Protein aggregate myopathy, Nemaline myopathy, Congenital myopathy (CM), Myoshi myopathy, Vici syndrome, X-linked myopathy with excessive autophagy, Danon disease, Pompe disease and Primary mitochondrial myopathies,
- a congenital myasthenic syndrome selected from the group consisting of Myasthenia gravis and other myasthenic syndromes driven by mutations in CHAT, COLQ, RAPSN, CHRNE, DOK7 and/or GFPT1 genes,
- a motor neuron disease selected from the group consisting of Spinal Muscular Atrophy (SMA), Amyotrophic Lateral Sclerosis (ALS) and Kennedy's disease, or
- a metabolic muscle disorder selected from the group consisting of cachexia, sarcopenia and muscle atrophy.

11. The method of any of claims 1 to 10, wherein the neuromuscular disorder of interest is a muscular dystrophy selected from the group consisting of Duchenne Muscular Dystrophy (DMD) and Myotonic Dystrophy 1 (DM1).

12. The method of claim 11, wherein the neuromuscular disorder of interest is Duchenne Muscular Dystrophy (DMD) and the myotubes are stained for an imaging marker which is selected from the group of proteins consisting of utrophin, alpha-sarcoglycan, delta-sarcoglycan, and the combinations of utrophin with alpha-sarcoglycan, delta-sarcoglycan, alpha-dystroglycan or beta-dystroglycan, preferably utrophin and/or alpha-sarcoglycan.

13. The method of claim 11, wherein the neuromuscular disorder of interest is Myotonic Dystrophy 1 (DM1) and the myotubes are stained for an imaging marker which is selected from the group of proteins consisting of amphiphysin 2 protein (also known as BIN-1), alpha-sarcoglycan, beta-dystroglycan, dystrophin, preferably C-terminal dystrophin, a combination of the protein MBNL1 and RNA foci, and any combinations thereof.

14. The method of claim 11, wherein the neuromuscular disorder of interest is Myotonic Dystrophy 1 (DM1) and the myotubes are stained for an imaging marker which is selected from the group of proteins consisting of (i) Amphiphysin 2 protein (also known as BIN-1), (ii) alpha-sarcoglycan, optionally in combination with dystrophin, preferably C-terminal dystrophin, (iii) beta-dystroglycan, optionally in combination with dystrophin, preferably C-terminal dystrophin, (iv) dystrophin, preferably C-terminal dystrophin, optionally in combination with alpha-sarcoglycan and/or beta-dystroglycan, and (v) a combination of the protein MBNL1 and RNA foci, and any combination thereof.

15. A computing system comprising:
- a memory storing at least one instruction of a classifier trained according to the method of training a classifier as defined in claim 6, and
- a processor accessing to the memory for reading the aforesaid instructions and executing the method according to any of claims 1 to 14.

## Patentansprüche

1. Ein *in vitro*-Verfahren zur Bewertung der Wirksamkeit einer Verbindung den Phänotyp eines Myotubus, das Merkmale einer neuromuskulären Erkrankung von Interesse ("erkrankter Myotubus") aufweist, in einen gesunden Phänotyp zurückzuverwandeln, umfassend
(i) das Bereitstellen mindestens eines Bildes umfassend eine Vielzahl von *in vitro* kultivierten erkrankten Myotuben, die von mindestens einem Patienten, der an einer neuromuskulären Erkrankung von Interesse leidet, stammen oder vorverarbeitete Informationen, die aus dem mindestens einen Bild gewonnen wurden, an einen Klassifikator, der darauf trainiert ist, zwischen gesunden Myotuben und erkrankten Myotuben zu unterscheiden, wobei die Myotuben mit der zu testenden Verbindung in Kontakt gebracht und für einen Bildgebungsmarker angefärbt wurden, der aus der Gruppe ausgewählt ist, bestehend aus identifiziertem(n) Krankheitsauslöser(n) der neuromuskulären Erkrankung von Interesse, Proteinen, die mit spezifischen funktionellen oder strukturellen Eigenschaften von Muskelzellen assoziiert sind, Zellmorphologiemerkmalen, die bei neuromuskulären Erkrankungen beeinträchtigt sind, sowie beliebigen Kombinationen davon, und mit mindestens einem Markierungsmittel, das mindestens einen Bereich von Interesse (ROI) sichtbar macht, der aus der Gruppe ausgewählt ist, bestehend aus einzelnen Myotuben, Strukturen von Myotuben und beliebigen Kombinationen davon; und
(ii) Verwendung des Klassifikators, um jedes Myotubus, das einem Eingabe-ROI entspricht, als gesundes oder als erkranktes Myotubus zu identifizieren als Ausgabe des Klassifikators, wobei eine Anzahl von Myotububen, die als gesunde Myotuben klassifiziert wurden und einen statistisch signifikanten Schwellenwert überschreiten, darauf hindeutet, dass die zu testende Verbindung in der Lage ist, den Phänotyp eines Myotubus, das Merkmale der neuromuskulären Erkrankung von Interesse aufweist, rückgängig zu machen und wobei das Verfahren ferner vor Schritt (i) umfasst:
- Kultivieren von Myoblasten, die von dem mindestens einen Patienten stammen, auf einem Substrat, das die Bildung einer homogenen Population von Myotuben ermöglicht, und Inkontaktbringen dieser Myoblasten und/oder Myotuben mit der zu testenden Verbindung;
- Färbung dieser Myotuben für den Bildgebungsmarker und für das mindestens eine Markierungsmittel; und
- Aufnahme des mindestens einen Bildes dieser gefärbten Myotuben.

2. Das Verfahren nach Anspruch 1, wobei das Verfahren ferner die Berechnung eines Gesundheitswerts umfasst, der als der prozentuale Anteil der Myotuben an der Gesamtzahl definiert ist, die als gesunde Myotuben klassifiziert wurden.

3. Ein *in vitro*-Verfahren zur Vorhersage der Eignung einer Verbindung zur Behandlung einer neuromuskulären Erkrankung von Interesse, umfassend
die Bewertung der Wirksamkeit einer zu testenden Verbindung, den Phänotyp eines Myotubs, das Merkmale einer neuromuskulären Erkrankung von Interesse aufweist, in einen gesunden Phänotyp zurückzuverwandeln, gemäß Anspruch 1 oder 2, und
optional die Berechnung eines Gesundheitswerts, der als der prozentuale Anteil der Myotuben an der Gesamtzahl definiert ist, die als gesunde Myotuben klassifiziert wurden,
wobei eine Anzahl von als gesunde Myotuben klassifizierten Myotuben oder ein berechneter Gesundheitswert, der über einem statistisch signifikanten Schwellenwert liegt, darauf hindeutet, dass die Verbindung bei der Behandlung der neuromuskulären Erkrankung nützlich ist.

4. Ein *in vitro*-Verfahren zur Überwachung des Ansprechens eines an einer neuromuskulären Erkrankung leidenden Patienten auf eine therapeutische Verbindung, wobei das Verfahren umfasst
(i) Bereitstellen mindestens eines ersten Bildes umfassend eine Vielzahl von *in vitro* kultivierten Myotuben, die von einem an einer neuromuskulären Erkrankung von Interesse leidenden Patienten stammen, vor der Verabreichung der therapeutischen Verbindung an den Patienten, oder vorverarbeiteter Informationen, die aus dem mindestens einen ersten Bild gewonnen wurden, sowie mindestens eines zweiten Bildes umfasssend eine Vielzahl von Myotuben, die von dem Patienten nach der Verabreichung der therapeutischen Verbindung stammen, oder vorverarbeitete Informationen, die aus dem mindestens einen zweiten Bild gewonnen wurden, an einen Klassifikator, der darauf trainiert ist, zwischen gesunden Myotuben und erkrankten Myotuben zu unterscheiden, wobei die Myotuben für einen Bildgebungsmarker angefärbt wurden, der aus der Gruppe ausgewählt ist, bestehend aus identifiziertem(n) Krankheitsauslöser(n) der neuromuskulären Erkrankung von Interesse, Proteinen, die mit spezifischen funktionellen oder strukturellen Eigenschaften von Muskelzellen assoziiert sind, Zellmorphologiemerkmalen, die bei neuromuskulären Erkrankungen beeinträchtigt sind, sowie beliebigen Kombinationen davon, und mit mindestens einem Markierungsmittel, das mindestens einen Bereich von Interesse (ROI) sichtbar macht, der aus der Gruppe ausgewählt ist, bestehend aus einzelnen Myotuben, Strukturen von Myotuben und beliebigen Kombinationen davon; und
(ii) Verwendung des Klassifikators, um jedes Myotubus des mindestens einen ersten Bildes, das einem Eingang-ROI entspricht, als gesundes Myotubus oder als erkranktes Myotubus zu identifizieren, als erste Ausgabe des Klassifikators, und jedes Myotubus des mindestens einen zweiten Bildes, das einem Eingabe-ROI entspricht, als gesundes Myotubus oder als erkranktes Myotubus zu identifizieren, als zweite Ausgabe des Klassifikators, und
optional Berechnen von Gesundheitswerten für die erste und zweite Ausgabe des Klassifikators, die jeweils als der prozentuale Anteil der Myotuben an der Gesamtzahl definiert sind, die als gesunde Myotuben klassifiziert wurden,
wobei eine Anzahl von Myotuben, die als gesunde Myotuben klassifiziert wurden, oder ein berechneter Gesundheitswert in der zweiten Ausgabe des Klassifikators, der über einer Anzahl von als gesunde Myotuben klassifizierten Myotuben oder über einem berechneten Gesundheitswert in der ersten Ausgabe des Klassifikators liegt, daraufhindeutet, dass der Patient auf die therapeutische Verbindung anspricht, und wobei eine Anzahl von als gesunde Myotuben klassifizierten Myotuben oder ein berechneter Gesundheitswert in der zweiten Ausgabe des Klassifikators, der gleich oder kleiner als eine Anzahl von als gesunde Myotuben klassifizierten Myotuben oder gleich oder kleiner als ein berechneter Gesundheitswert in der ersten Ausgabe des Klassifikators ist, darauf hindeutet, dass der Patient nicht auf die therapeutische Verbindung anspricht, und
wobei das Verfahren ferner vor Schritt (i) umfasst
- Kultivieren von Myoblasten, die von dem Patienten vor und nach der Verabreichung der therapeutischen Verbindung stammen, auf einem Substrat, das die Bildung einer homogenen Population von Myotuben ermöglicht,
- Färbung dieser Myotuben für den Bildgebungsmarker und für das mindestens eine Markierungsmittel; und
- Aufnahme des mindestens einen Bildes dieser gefärbten Myotuben.

5. Die Verfahren nach einem der Ansprüche 1 bis 4, wobei der Bildgebungsmarker und das mindestens eine Markierungsmittel während dem Training des Klassifikators verwendet wurde.

6. Die Verfahren nach einem der Ansprüche 1 bis 4, wobei der Klassifikator unter Verwendung eines Verfahrens zum Trainieren eines Klassifikators trainiert wurde, um genau zwischen gesunden Myotuben und Myotuben, die Merkmale einer neuromuskulären Erkrankung von Interesse ("erkrankter Myotubus") aufweisen, zu unterscheiden, wobei das Verfahren umfasst
a) Bereitstellen eines Trainingsbildsatzes mit gefärbten, *in vitro* kultivierten Myotuben oder vorverarbeiteter Informationen, die aus diesem Trainingsbildsatz gewonnen wurden, für einen Klassifikator, wobei der Trainingsbildsatz Bilder von gesunden Myotuben und von Myotuben umfasst, die Merkmale einer neuromuskulären Erkrankung von Interesse ("erkrankter Myotubus") aufweisen, wobei diese Myotuben für einen Bildgebungsmarker-Kandidaten gefärbt sind, der aus der Gruppe ausgewählt ist, bestehend aus identifiziertem(n) Krankheitsauslöser(n) der neuromuskulären Erkrankung von Interesse, Proteinen, die mit spezifischen funktionellen oder strukturellen Eigenschaften von Muskelzellen assoziiert sind, bei neuromuskulären Erkrankungen betroffenen zellmorphologischen Merkmalen und beliebigen Kombinationen davon, sowie mit mindestens einem Markierungsmittel, das mindestens einen Bereich von Interesse (ROI) sichtbar macht, der aus der Gruppe ausgewählt ist, die aus einzelnen Myotuben, Strukturen von Myotuben und beliebigen Kombinationen davon besteht;
b) Erzeugen einer Ausgabe des Klassifikators für jeden Eingabe-ROI, wobei die Ausgabe den Eingabe-ROI als mit einem gesunden oder erkrankten Myotubus assoziiert klassifiziert;
c) Vergleichen der generierten Ausgabe für jede Eingabe-ROI mit einer Markierung, die mit der Eingabe-ROI assoziiert ist, wobei die Markierung eine Angabe des gesunden oder erkrankten Zustands des Myotubus umfasst, das der Eingabe-ROI entspricht;
d) Bewertung der Genauigkeit des Klassifikators bei der Unterscheidung zwischen gesunden und erkrankten Myotuben,
wobei der Bildgebungsmarker-Kandidat als Bildgebungsmarker für die neuromuskuläre Erkrankung identifiziert wird, wenn der Klassifikator eine Genauigkeit bei der Unterscheidung zwischen gesunden und erkrankten Myotuben aufweist, die einem F-Score von gleich oder größer als 0,9 entspricht.

7. Das Verfahren gemäß einem der Ansprüche 1 bis 6, wobei die vorverarbeiteten Informationen gewonnen werden durch
- die Durchführung einer Bildsegmentierung mit einem Algorithmus auf einem oder mehreren geeigneten Färbungskanälen, um die ROI zu identifizieren, und
- die Extraktion phänotypischer Merkmale, die mit dem Bildgebungsmarker assoziiert sind, aus jeder Eingabe-ROI, wobei die extrahierten phänotypischen Merkmale ausgewählt sind aus der Gruppe bestehend aus Intensitätsmerkmalen, Granularitätsmerkmalen, Intensitätsverteilungsmerkmalen, Texturmerkmalen, Größen- und Formmerkmalen, Kolokalisierungsmerkmalen, auf Run-Length-Graustufenmatrizen basierenden Merkmalen, auf Wavelet-Transformationen basierenden Merkmalen und Kombinationen davon, vorzugsweise ausgewählt aus der Gruppe bestehend aus Intensitätsmerkmalen, Granularitätsmerkmalen, Intensitätsverteilungsmerkmalen, Texturmerkmalen und beliebigen Kombinationen davon.

8. Das Verfahren gemäß einem der Ansprüche 1 bis 7, wobei der Klassifikator ausgewählt ist aus: Support-Vektor-Maschine (SVM) Klassifikator, Random Forest (RF) Klassifikator, Entscheidungsbaum Klassifikator, K-nächster-Nachbar-Klassifikator (KNN), logistische Regression Klassifikator, Nächster-Nachbar-Klassifikator, Gauß-Mischmodell (GMM), Nächster-Zentroid-Klassifikator, lineare Regression Klassifikator sowie neuronale Netzwerke wie künstliche, tiefe, konvolutionelle und vollverbundene neuronale Netzwerke.

9. Das Verfahren gemäß einem der Ansprüche 1 bis 8, wobei die neuromuskuläre Erkrankung von Interesse ausgewählt ist aus der Gruppe bestehend aus Muskeldystrophien, Myopathien, angeborenen myasthenischen Syndromen, Motoneuronerkrankungen und metabolischen Muskelerkrankungen.

10. Das Verfahren gemäß einem der Ansprüche 1 bis 9, wobei die neuromuskuläre Erkrankung von Interesse
- eine Muskeldystrophie ausgewählt aus der Gruppe bestehend aus Duchenne-Muskeldystrophie (DMD), Becker-Muskeldystrophie (BMD), myotonischer Dystrophie Typ 1 (DM1), myotonischer Dystrophie Typ 2 (DM2), Fazioskapulohumerale Muskeldystrophie (FSHD), Emery-Dreifuss-Muskeldystrophie, Gliedergürtel-Muskeldystrophien, Walker-Warburg-Syndrom, Muskel-Auge-Gehirn-Erkrankung, angeborene Muskeldystrophie, Skapuloperoneale Muskeldystrophie, Tibiale Muskeldystrophie und autosomal-rezessive Muskeldystrophie,
- eine Myopathie ausgewählt aus der Gruppe bestehend aus Bethlem-&-Ullrich-Myopathie, myofibrillärer Myopathie, distaler Myopathie, Rimmed-Vacuole-Myopathie, zentronukleärer Myopathie (CNM), X-chromosomaler myotubulärer Myopathie (XLMTM), tubuläre Aggregat-Myopathie, malignes Hyperthermie-Syndrom, Einschlusskörper-Myopathie, myofibrilläre Myopathie, Proteinaggregat-Myopathie, Nemalin-Myopathie, kongenitale Myopathie (CM), Myoshi-Myopathie, Vici-Syndrom, X-chromosomale Myopathie mit übermäßiger Autophagie, Danon-Krankheit, Pompe-Krankheit und primäre mitochondriale Myopathien,
- ein angeborenes myasthenisches Syndrom, ausgewählt aus der Gruppe bestehend aus Myasthenia gravis und anderen myasthenischen Syndromen, die durch Mutationen in den Genen CHAT, COLQ, RAPSN, CHRNE, DOK7 und/oder GFPT1 verursacht werden,
- eine Motoneuronerkrankung ausgewählt aus der Gruppe bestehend aus spinaler Muskelatrophie (SMA), amyotropher Lateralsklerose (ALS) und der Kennedy-Krankheit oder
- eine metabolische Muskelerkrankung ausgewählt aus der Gruppe bestehend aus Kachexie, Sarkopenie und Muskelatrophie.

11. Das Verfahren gemäß einem der Ansprüche 1 bis 10, wobei die neuromuskuläre Erkrankung von Interesse eine Muskeldystrophie ist ausgewählt aus der Gruppe bestehend aus Duchenne-Muskeldystrophie (DMD) und myotonischer Dystrophie Typ 1 (DM1).

12. Das Verfahren gemäß Anspruch 11, wobei die neuromuskuläre Erkrankung von Interesse Duchenne-Muskeldystrophie (DMD) ist und wobei die Myotuben mit einem Bildgebungsmarker markiert werden, der ausgewählt ist aus der Gruppe von Proteinen bestehend aus Utrophin, Alpha-Sarkoglykan, Delta-Sarkoglykan und den Kombinationen von Utrophin mit Alpha-Sarkoglykan, Delta-Sarkoglykan, Alpha-Dystroglykan oder Beta-Dystroglykan, vorzugsweise Utrophin und/oder Alpha-Sarkoglykan.

13. Das Verfahren gemäß Anspruch 11, wobei die neuromuskuläre Erkrankung von Interesse myotonische Dystrophie Typ 1 (DM1) ist und wobei die Myotuben angefärbt wurden mit einen Bildgebungsmarker ausgewählt aus der Gruppe von Proteinen bestehend aus Amphiphysin-2-Protein (auch bekannt als BIN-1), Alpha-Sarkoglykan, Beta-Dystroglykan, Dystrophin, vorzugsweise C-terminales Dystrophin, gegebenenfalls in Kombination mit Alpha-Sarkoglykan und/oder Beta-Dystroglykan, und eine Kombination aus dem Protein MBNL1 und RNA-Foci sowie jede Kombination davon.

14. Das Verfahren gemäß Anspruch 11, wobei die neuromuskuläre Erkrankung von Interesse myotonische Dystrophie Typ 1 (DM1) ist und wobei die Myotuben mit einem Bildgebungsmarker markiert wurden, der ausgewählt ist aus der Gruppe von Proteinen bestehend aus (i) Amphiphysin-2-Protein (auch bekannt als BIN-1), (ii) Alpha-Sarkoglykan, optional in Kombination mit Dystrophin, vorzugsweise C-terminales Dystrophin, (iii) Beta-Dystroglykan, optional in Kombination mit Dystrophin, vorzugsweise C-terminales Dystrophin, (iv) Dystrophin, vorzugsweise C-terminales Dystrophin, optional in Kombination mit Alpha-Sarkoglykan und/oder Beta-Dystroglykan, und (v) eine Kombination aus dem Protein MBNL1 und RNA-Foci sowie jede Kombination davon.

15. Ein Computersystem, umfassend:
- einen Speicher, der mindestens einen Befehl eines Klassifikators speichert, der gemäß dem Verfahren zum Trainieren eines Klassifikators gemäß Anspruch 6 trainiert wurde, und
- einen Prozessor, der auf den Speicher zugreift, um die vorgenannten Befehle zu lesen und das Verfahren gemäß einem der Ansprüche 1 bis 14 auszuführen.

## Revendications

1. Méthode *in vitro* d'évaluation du pouvoir d'un composé pour restaurer le phénotype d'un myotube présentant des caractéristiques d'un trouble neuromusculaire d'intérêt ("myotube pathologique") en un phénotype sain, comprenant
(i) la fourniture d'au moins une image comprenant une pluralité de myotubes pathologiques cultivés *in vitro* dérivés d'au moins un patient souffrant d'un trouble neuromusculaire d'intérêt, ou d'informations prétraitées obtenues à partir de ladite au moins une image, à un classificateur entraîné pour distinguer les myotubes sains des myotubes pathologiques, dans laquelle les myotubes ont été mis en contact avec le composé devant être testé et ont été soumis à une coloration pour un marqueur d'imagerie choisi dans le groupe constitué par un ou plusieurs facteurs pathologiques identifiés du trouble neuromusculaire d'intérêt, les protéines associées à des propriétés fonctionnelles ou structurelles spécifiques des cellules musculaires, les caractéristiques morphologiques cellulaires affectées dans les troubles neuromusculaires, et l'une quelconque de leurs combinaisons, et avec au moins un agent de marquage révélant au moins une région d'intérêt (ROI) choisie dans le groupe constitué par les myotubes individuels, les structures de myotubes, et l'une quelconque de leurs combinaisons ; et
(ii) l'utilisation du classificateur pour identifier chaque myotube correspondant à une ROI d'entrée en tant que myotube sain ou en tant que myotube pathologique à titre de sortie du classificateur,
un nombre de myotubes classés en myotubes sains qui est supérieur à un seuil statistiquement significatif étant indicatif du fait que le composé devant être testé est capable de restaurer le phénotype d'un myotube présentant des caractéristiques du trouble neuromusculaire d'intérêt, et laquelle méthode comprend en outre, avant l'étape (i)
- la culture de myoblastes dérivés dudit au moins un patient sur un substrat permettant la production d'une population homogène de myotubes et la mise en contact desdits myoblastes et/ou myotubes avec le composé devant être testé ;
- la coloration de ces myotubes pour ledit marqueur d'imagerie et pour ledit au moins un agent de marquage ; et
- la capture de ladite au moins une image de ces myotubes colorés.

2. Méthode selon la revendication 1, laquelle méthode comprend en outre le calcul d'un score de santé qui est défini par le pourcentage de myotubes par rapport au total qui ont été classés en myotubes sains.

3. Méthode *in vitro* pour prédire l'aptitude d'un composé à traiter un trouble neuromusculaire d'intérêt, comprenant
la détermination du pouvoir d'un composé devant être testé pour restaurer le phénotype d'un myotube présentant des caractéristiques d'un trouble neuromusculaire d'intérêt en un phénotype sain selon la revendication 1 ou 2,
et éventuellement le calcul d'un score de santé qui est défini par le pourcentage de myotubes par rapport au total qui ont été classés en myotubes sains,
dans laquelle un nombre de myotubes classés en myotubes sains, ou un score de santé calculé, qui est supérieur à un seuil statistiquement significatif, est indicatif du fait que ledit composé est utile dans le traitement dudit trouble neuromusculaire.

4. Méthode *in vitro* pour surveiller la réponse à un composé thérapeutique d'un patient affecté par un trouble neuromusculaire, laquelle méthode comprend
(i) la fourniture d'au moins une première image comprenant une pluralité de myotubes cultivés *in vitro* dérivés d'un patient souffrant d'un trouble neuromusculaire d'intérêt avant l'administration du composé thérapeutique au patient, ou d'informations prétraitées obtenues à partir de ladite au moins une première image, et d'au moins une deuxième image comprenant une pluralité de myotubes dérivés dudit patient après l'administration du composé thérapeutique, ou d'informations prétraitées obtenues à partir de ladite au moins une deuxième image, à un classificateur entraîné pour distinguer les myotubes sains des myotubes pathologiques, dans laquelle les myotubes ont été soumis à une coloration pour un marqueur d'imagerie choisi dans le groupe constitué par un ou plusieurs facteurs pathologiques identifiés du trouble neuromusculaire d'intérêt, les protéines associées à des propriétés fonctionnelles ou structurelles spécifiques des cellules musculaires, les caractéristiques morphologiques cellulaires affectées dans les troubles neuromusculaires, et l'une quelconque de leurs combinaisons, et avec au moins un agent de marquage révélant au moins une région d'intérêt (ROI) choisie dans le groupe constitué par les myotubes individuels, les structures de myotubes, et l'une quelconque de leurs combinaisons ; et
(ii) l'utilisation du classificateur pour identifier chaque myotube de ladite au moins une première image correspondant à une ROI d'entrée en tant que myotube sain ou en tant que myotube pathologique à titre de première sortie du classificateur, et pour identifier chaque myotube de ladite au moins une deuxième image correspondant à une ROI d'entrée en tant que myotube sain ou en tant que myotube pathologique à titre de deuxième sortie du classificateur, et
éventuellement le calcul de scores de santé pour les première et deuxième sorties du classificateur qui sont définis chacun par le pourcentage de myotubes par rapport au total qui ont été classés en myotubes sains,
dans laquelle un nombre de myotubes classés en myotubes sains, ou un score de santé calculé, dans la deuxième sortie du classificateur, qui est supérieur à un nombre de myotubes classés en myotubes sains, ou supérieur à un score de santé calculé, dans la première sortie du classificateur, est indicatif du fait que le patient répond audit composé thérapeutique, et dans laquelle un nombre de myotubes classés en myotubes sains, ou un score de santé calculé, dans la deuxième sortie du classificateur, qui est égal ou inférieur à un nombre de myotubes classés en myotubes sains, ou égal ou inférieur à un score de santé calculé, dans la première sortie du classificateur, est indicatif du fait que le patient ne répond pas audit composé thérapeutique, et
laquelle méthode comprend en outre, avant l'étape (i)
- la culture de myoblastes dérivés dudit patient avant et après l'administration du composé thérapeutique sur un substrat permettant la production d'une population homogène de myotubes ;
- la coloration de ces myotubes pour ledit marqueur d'imagerie et pour ledit au moins un agent de marquage ; et
- la capture de ladite au moins une image de ces myotubes colorés.

5. Méthode selon l'une quelconque des revendications 1 à 4, dans laquelle ledit marqueur d'imagerie et ledit au moins un agent de marquage ont été utilisé durant l'apprentissage du classificateur.

6. Méthode selon l'une quelconque des revendications 1 à 4, dans laquelle le classificateur a été entraîné au moyen d'une méthode d'apprentissage d'un classificateur pour qu'il distingue avec précision les myotubes sains des myotubes présentant des caractéristiques d'un trouble neuromusculaire d'intérêt ("myotube pathologique"), ladite méthode comprenant
a) la fourniture d'un jeu d'apprentissage d'images de myotubes cultivés *in vitro* soumis à une coloration, ou d'informations prétraitées obtenues à partir dudit jeu d'apprentissage d'images, à un classificateur, le jeu d'apprentissage d'images comprenant des images de myotubes sains et de myotubes présentant des caractéristiques d'un trouble neuromusculaire d'intérêt ("myotube pathologique"), lesdits myotubes étant soumis à une coloration pour un marqueur d'imagerie candidat choisi dans le groupe constitué par un ou plusieurs facteurs pathologiques identifiés du trouble neuromusculaire d'intérêt, les protéines associées à des propriétés fonctionnelles ou structurelles spécifiques des cellules musculaires, les caractéristiques morphologiques cellulaires affectées lors de troubles neuromusculaires, et l'une quelconque de leurs combinaisons, et avec au moins un agent de marquage révélant au moins une région d'intérêt (ROI) choisie dans le groupe constitué par les myotubes individuels, les structures de myotubes, et l'une quelconque de leurs combinaisons ;
b) la génération d'une sortie du classificateur pour chaque ROI d'entrée, ladite sortie classant la ROI d'entrée comme étant associée à un myotube sain ou pathologique ;
c) la comparaison de la sortie générée pour chaque ROI d'entrée avec une étiquette associée à ladite ROI d'entrée, ladite étiquette comprenant une indication de l'état sain ou pathologique du myotube correspondant à ladite ROI d'entrée ;
d) l'évaluation de la précision du classificateur pour distinguer les myotubes sains des myotubes pathologiques,
dans laquelle ledit marqueur d'imagerie candidat est identifié comme étant un marqueur d'imagerie dudit trouble neuromusculaire si le classificateur présente une précision pour distinguer les myotubes sains des myotubes pathologiques correspondant à un score F égal ou supérieur à 0,9.

7. Méthode selon l'une quelconque des revendications 1 à 6, dans laquelle lesdites informations prétraitées sont obtenues par
- mise en œuvre d'une segmentation d'image avec un algorithme sur un ou plusieurs canaux de coloration appropriés afin d'identifier une ROI, et
- extraction, à partir de chaque ROI d'entrée, de caractéristiques phénotypiques associées audit marqueur d'imagerie, lesdites caractéristiques phénotypiques étant choisies dans le groupe constitué par les caractéristiques d'intensité, les caractéristiques de granularité, les caractéristiques de distribution d'intensité, les caractéristiques de texture, les caractéristiques de taille et de forme, les caractéristiques de colocalisation, les caractéristiques basées sur la matrice des longueurs de séries des niveaux de gris, les caractéristiques basées sur la transformée en ondelettes, et leurs combinaisons, de préférence choisies dans le groupe constitué par les caractéristiques d'intensité, les caractéristiques de granularité, les caractéristiques de distribution d'intensité, les caractéristiques de texture, et l'une quelconque de leurs combinaisons.

8. Méthode selon l'une quelconque des revendications 1 à 7, dans laquelle le classificateur est choisi parmi un classificateur à machines à vecteurs de support (SVM), un classificateur à forêts aléatoires (RF), un classificateur par arbres de décision, un classificateur des K plus proches voisins (KNN), un classificateur par régression logistique, un classificateur du plus proche voisin, un modèle de mélange gaussien (GMM), un classificateur par centroïde le plus proche, un classificateur par régression linéaire, et les réseaux de neurones tels que les réseaux de neurones artificiels, profonds, convolutifs, totalement connectés.

9. Méthode selon l'une quelconque des revendications 1 à 8, dans laquelle le trouble neuromusculaire d'intérêt est choisi dans le groupe constitué par les dystrophies musculaires, les myopathies, les syndromes myasthéniques congénitaux, les maladies des motoneurones et les maladies musculaires métaboliques.

10. Méthode selon l'une quelconque des revendications 1 à 9, dans laquelle le trouble neuromusculaire d'intérêt est
- une dystrophie musculaire choisie dans le groupe constitué par la dystrophie musculaire de Duchenne (DMD), une dystrophie musculaire de Becker (BMD), une dystrophie myotonique de type 1 (DM1), une dystrophie myotonique de type 2 (DM2), une dystrophie musculaire facio-scapulo-humérale (FSHD), une dystrophie musculaire d'Emery-Dreifuss, les dystrophies musculaires des ceintures, un syndrome de Walker-Warburg, une maladie muscle-œil-cerveau, une dystrophie musculaire congénitale, une dystrophie musculaire scapulo-péronière, une dystrophie musculaire tibiale et une dystrophie musculaire autosomique récessive,
- une myopathie choisie dans le groupe constitué par la myopathie de Bethlem et d'Ullrich, une myopathie myofibrillaire, une myopathie distale, une myopathie à vacuoles bordées, une myopathie centronucléaire (CNM), une myopathie myotubulaire liée à l'X (XLMTM), une myopathie à agrégats tubulaires, un syndrome d'hyperthermie maligne, une myopathie à inclusions, une myopathie myofibrillaire, une myopathie à agrégats protéiques, une myopathie némaline, une myopathie congénitale (CM), une myopathie de Myoshi, un syndrome de Vici, une myopathie liée à l'X avec autophagie excessive, une maladie de Danon, une maladie de Pompe et les myopathies mitochondriales primaires,
- un syndrome myasthénique congénital choisi dans le groupe constitué par la myasthénie grave et d'autres syndromes myasthéniques induits par des mutations dans les gènes CHAT, COLQ, RAPSN, CHRNE, DOK7 et/ou GFPT1,
- une maladie des motoneurones choisie dans le groupe constitué par une amyotrophie spinale (SMA), une sclérose latérale amyotrophique (SLA) et une maladie de Kennedy, ou
- un trouble musculaire métabolique choisi dans le groupe constitué par une cachexie, une sarcopénie et une atrophie musculaire.

11. Méthode selon l'une quelconque des revendications 1 à 10, dans laquelle le trouble neuromusculaire d'intérêt est une dystrophie musculaire choisie dans le groupe constitué par une dystrophie musculaire de Duchenne (DMD) et une dystrophie myotonique de type 1 (DM1).

12. Méthode selon la revendication 11, dans laquelle le trouble neuromusculaire d'intérêt est une dystrophie musculaire de Duchenne (DMD) et les myotubes sont soumis à une coloration pour un marqueur d'imagerie qui est choisi dans le groupe de protéines consistant en l'utrophine, l'alpha-sarcoglycane, le delta-sarcoglycane, et les combinaisons d'utrophine avec l'alpha-sarcoglycane, le delta-sarcoglycane, l'alpha-dystroglycane ou le bêta-dystroglycane, de préférence l'utrophine et/ou l'alpha-sarcoglycane.

13. Méthode selon la revendication 11, dans laquelle le trouble neuromusculaire d'intérêt est une dystrophie myotonique de type 1 (DM1) et les myotubes sont soumis à une coloration pour un marqueur d'imagerie qui est choisi dans le groupe de protéines consistant en la protéine amphiphysine 2 (également appelée BIN-1), l'alpha-sarcoglycane, le bêta-dystroglycane, la dystrophine, de préférence la dystrophine C-terminale, une combinaison de la protéine BMNL1 et de foyers d'ARN, et l'une quelconque de leurs combinaisons.

14. Méthode selon la revendication 11, dans laquelle le trouble neuromusculaire d'intérêt est une dystrophie myotonique de type 1 (DM1) et les myotubes sont soumis à une coloration pour un marqueur d'imagerie qui est choisi dans le groupe de protéines consistant en (i) la protéine amphiphysine 2 (également appelée BIN-1), (ii) l'alpha-sarcoglycane, éventuellement en combinaison avec la dystrophine, de préférence la dystrophine C-terminale, (iii) le bêta-dystroglycane, éventuellement en combinaison avec la dystrophine, de préférence la dystrophine C-terminale, (iv) la dystrophine, de préférence la dystrophine C-terminale, éventuellement en combinaison avec l'alpha-sarcoglycane et/ou le bêta-dystroglycane, et (v) une combinaison de la protéine BMNL1 et de foyers d'ARN, et l'une quelconque de leurs combinaisons.

15. Système informatique comprenant :
- une mémoire stockant au moins une instruction d'un classificateur entraîné selon la méthode d'entraînement d'un classificateur telle que définie dans la revendication 6, et
- un processeur accédant à la mémoire pour lire les instructions susdites et exécuter la méthode selon l'une quelconque des revendications 1 à 14.
